# EUROPEAN PATENT APPLICATION

(11) **EP 2 949 758 A1**
(43) Date of publication of application: **02.12.2015**
(21) Application number: 14170516.0
(22) Date of filing: 30.05.2014
(51) Int. Cl.: C12Q 1/68

(54) **DNA array for detecting canine toll-like receptor gene mutations**

(71) Applicant: Universitat Autònoma de Barcelona, 08193 Bellaterra (Cerdanyola del Valles) (ES)
(72) Inventor: Francino Marti, Olga, 08195 Sant Cugat del Vallès (ES); Cusco Marti, M Anna Maria, 08793 Cantallops d'Avinyonet del Penedes (ES); Sanchez Bonastre, Armand, 08173 Sant Cugat del Vallès (ES)

(57) **Abstract**

New mutations in canine toll-like receptor genes are provided. The invention relates further to DNA arrays comprising oligonucleotides capable of hybridizing to at least a TLR gene fragment codifying for at least one of the new mutations leading to non-functional proteins. The invention also includes methods for the analysis of mutations and for the genetic predisposition of an individual of *Canis* genera to suffer from any disease related with dysfunctions of the innate immunity system. The invention also provides a method for the individual identification of canine subjects.

## Description

Present invention relates to meaningful mutations identified in some critical genes involved in the innate immunity in dogs (canine genus), as well as to canine disease diagnosis, prediction of canine disease susceptibility, and to the individual identification of canine subjects.

### BACKGROUND ART

Increasing number of domestic animals, in particular dogs and cats, has promoted the study, particular diagnostic, and particular treatments of these animals. For the study of the diseases is interesting to know how the immune system functions in these animals, particularly when infectious or autoimmune disease are to be faced. Besides, other systems and metabolic pathways are studied in order to determine if they are specific of species or not. Additionally, and considering that dogs include the greatest number of breeds known in the animal kingdom, there can be breed specific diseases or susceptibilities which may influence the final diagnostic and treatment applied to specific breeds.

Despite it is known that some dog breeds have special susceptibilities to diseases and to particular treatments, little is known about the causes of these facts and, although it is widely accepted that genomic profiles are probably the greatest cause, poor meaningful studies have been performed. In part, this lack of knowledge is due to the lack of appropriate tools for the study of the dog genome or *Canis lupus familiaris* genome.

In the specific case of susceptibilities to some diseases, dog immune response has to be one of the objects for next years.

Besides, appropriate unequivocal identification of individuals or appropriate classification of any individual to a particular group would be also an aim.

Toll-like receptors (TLRs) are the most widely studied pattern recognition receptors (PRRs) and are considered to be the primary sensors of pathogens in innate immunity. These molecules are constituted by leucine-rich repeat (LRR) domains, a unique intramembrane domain and a Toll/Interleukin-1 receptor (TIR) domain. Pathogen-associated molecular Patterns (PAMPs) are sensed through LRR domain, and signals are transduced through TIR domain, which is always located in the cytoplasm, in order to activate innate immunity response.

10 TLRs have been identified in dogs. They are classified in two groups, depending on their location and the PAMPs detected. TLR which target nucleic acids (TLR3, 7, 8 and 9) are located in endosomal membranes and are also known as viral TLRs. On the other hand, there are membrane TLRs (TLR1, 2, 4, 5 and 6) which detect components of extracellular pathogens (bacteria, fungi, etc). TLR10 is also a membrane TLR, however its ligand is not yet known. 1

Still today little is known about canine TLR and its role or implication in diseases, basically because, as above stated, there are no tools to test for relevant mutations. Moreover, differences among breeds have been explored poorly. Kathrani et al., in "Polymorphisms in the TIr4 and TIr5 Gene Are Significantly Associated with Inflammatory Bowel Disease in German Shepherd Dogs", PLoS ONE - 2010, Vol. No. 5 (12), pp.: 1-10, associated polymorphisms in TLR4 and TLR5 with Inflammatory Bowel disease (IBD) in German shepherd dogs (GSD). They concluded that further studies were required to confirm the functional importance of the mutations. The same Kathrani et al. showed that only some protective SNPs from TLR5 were associated with IBD in other 38 dog breeds (Kathrani et al., "Breed-independent toll-like receptor 5 polymorphisms show association with canine inflammatory bowel disease", Tissue Antigens - 2011, Vol. No. 78, pp.: 94-101). All the mutations mentioned by Kathrani have been identified by sequencing TLR genes of dogs with IBD and then comparing sequence data to the canine genome.

Although no other polymorphisms have been associated to illness in dogs until date, some studies have reported differential expression of some TLRs related to inflammatory or infectious diseases, such as TLR2 in IBD; TLRs 2, 4, 5 and 9 in chronic enteropathies in German Shepherd; TLR4 in osteoarthritis and in infected canine endometrium; TLRs 1-4, 6-10 in sino-nasal aspergillosis and idiopathic lymphoplasmacytic rhinitis; and TLR2 and TLR9 in Leishmania infected dogs.

Therefore, there is still a need of methods and tools to better characterize physiology and immunity response profiles in domestic animals, in particular in dogs.

On the other hand, nowadays identification of dogs (or other animals) is a complex task, finally carried out with DNA technologies as in humans. Nonetheless, analysis of all the genome is always tedious and expensive. Therefore, there is also a need of new tools for unequivocally identifying individuals, as well as for the traceability of their waste material, or other samples from them.

### SUMMARY OF THE INVENTION

The inventors determined new mutations, some of them being single nucleotide polymorphisms (SNP) in canine TLR genes. In addition they have developed a method and an array for determining the innate immunity profile of individuals of the *Canis* genera based on these new mutations, which are mainly causing protein disruption or loss of function. These mutations, mainly non-synonymous mutations and frameshift mutations may impair, alter or affect the innate immunity response in individuals of the *Canis* genera, thus including among other dogs and wolves.

Noteworthy, most of individuals (i.e. dogs) with innate immune system defects have intact adaptive immune systems with normal immunoglobulins, antibodies and T-cells. A type of white blood cell called an eosinophil may be increased in the blood; elevated IgE immunoglobulin levels may also be present. The diagnosis of defects or dysfunctions in innate immunity system (response) is usually made by measuring cytokine production by white blood cells, activated by microbial products that stimulate the cells. Testing of TLR function is becoming available through commercial reference laboratories, such as the one followed by ARUP LABORATORIES® with TLR ligands. Abnormal tests need to be confirmed by repeat testing. Persistently abnormal tests may be followed up by specific genetic tests.

Using informatic tools for predicting the effect of the new mutations in the proteins (gene products) codified by the canine *tlr* genes, inventors identified those changes leading to a loss of protein function, damaging amino acid changes, or deleterious mutations that could be involved in the loss of innate immunity response in individuals of the *Canis* genera. These mutations may also be responsible of certain susceptibilities to diseases or of treatment resistances associated with an impaired innate immunity.

New mutations correspond mainly to the following amino acid changes in the protein sequences of canine toll-like receptors:
a change of a serine (S) by a leucine (L) at position 516 of canine TLR2 polypeptide of SEQ ID NO: 1; a change of a phenylalanine (F) by a cysteine (C) at position 888 of canine TLR5 polypeptide of SEQ ID NO: 2; a change of a leucine (L) by a phenylalanine (F) at position 457 of canine TLR6 polypeptide of SEQ ID NO: 3; and a change of a valine (V) by an alanine (A) at position 157 of canine TLR8 polypeptide of SEQ ID NO: 4.

The invention relates to a DNA array comprising canine toll-like receptor (TLR) gene fragments.

Thus, in a first aspect the invention relates to DNA arrays comprising:
(i) a canine toll-like receptor 2 (*tlr 2*) gene fragment that in the entire nucleotide sequence of the gene codifies for a change of a serine (S) by a leucine (L) at position 516 of canine TLR2 polypeptide of SEQ ID NO: 1;
(ii) a canine toll-like receptor 5 (*tlr 5*) gene fragment that in the entire nucleotide sequence of the gene codifies for a change of a phenylalanine (F) by a cysteine (C) at position 888 of canine TLR5 polypeptide of SEQ ID NO: 2;
(iii) a canine toll-like receptor (*tlr 6*) gene fragment that in the entire nucleotide sequence of the gene codifies for a change of a leucine (L) by a phenylalanine (F) at position 457 of canine TLR6 polypeptide of SEQ ID NO: 3; and
(iv) a canine toll-like receptor 8 (*tlr 8*) gene fragment that in the entire nucleotide sequence of the gene codifies for a change of a valine (V) by an alanine (A) at position 157 of canine TLR8 polypeptide of SEQ ID NO: 4.

Therefore, the DNA arrays comprise canine toll-like receptor *tlr* gene fragments codifying for peptides, said peptides in the entire corresponding TLR amino acid sequences having any of the following amino acid changes:
(i) A change of a serine (S) by a leucine (L) at position 516 of canine TLR2 polypeptide of SEQ ID NO: 1;
(ii) A change of a phenylalanine (F) by a cysteine (C) at position 888 of canine TLR5 polypeptide of SEQ ID NO: 2;
(iii) A change of a leucine (L) by a phenylalanine (F) at position 457 of canine TLR6 polypeptide of SEQ ID NO: 3; and
(iv) A change of a valine (V) by an alanine (A) at position 157 of canine TLR8 polypeptide of SEQ ID NO: 4.

These DNA arrays comprise oligonucleotide sequences that are fragments of canine *tlr* genes, which fragments in the entire gene will give raise to mutated proteins. These oligonucleotides of the array are indeed complementary to canine toll-like receptor gene fragments (being the codifying or complementary strain of the gene) present in an isolated sample from a subject of the *Canis* genera. The oligonucleotides are capable of detecting the above-identified mutations by hybridizing to these gene fragments codifying for the mutations, and thus making the mutations detectable. Hybridization will take place under stringent conditions, preferably at 60 ºC, and in any case in a range from 50 ºC to 70 ºC. Particular TaqMan® probe hybridization conditions can be seen in htt://premierbiosoft.com/tech notes/TaqMan.html (retrieved in April 23, 2014)

Thus, this first aspect could also be formulated as DNA arrays comprising oligonucleotides which are complementary and capable of hybridizing at a temperature of 60 ºC (50-70 ºC) to canine toll-like receptor gene fragments from a sample, the *tlr* gene fragments of the sample codifying, when forming part of the entire corresponding gene, for at least one of the mutations indicated above from (i) to (iv).

By means of these arrays, at least five critical mutations are detected in a simple and fast way, and allow obtaining a genomic profile with important information regarding innate immunity due to the fact that the identified mutations lead to a loss of protein function and/or to an altered protein structure. Indeed, the arrays allow compiling important information of several proteins/genes which, if mutated, will in addition probably lose its function and/or structure.

Leading then with the advantages of the arrays, a second aspect of the invention is a method for the identification of mutations in canine toll-like receptor genes, the method comprising:
- adding an isolated animal sample comprising DNA to a DNA array as defined above; and
- determining if the canine toll-like receptor gene fragments hybridize with sample gene fragments.

This method represents a simple way to detect if an isolated sample comprising genetic information (i.e polynucleotide data, namely DNA data) includes *tlr* gene fragments carrying any of the newly identified critical mutations. Genetic information may be retrieved from the DNA of the sample as well as from cDNA in case the starting material is RNA, which is firstly converted to cDNA by any routinely methods such as the Reverse Transcription Polymerase Chain Reaction (RT-PCR).

Detection of these particular non-synonymous mutations in a TLR proteins (namely in canine TLR2, TLR5, TLR6 and TLR8) have impact on protein function and/or structure and could finally alter or modify the innate immunity system (response) of the animal. This modification of the final innate immunity response will be informing about the predisposition of suffering from particular diseases. On the other hand, detection of mutated TLR with impact on the protein function could serve as basis for determining the cause of a particular disease yet manifested in a canine individual. The combined detection of these five mutations represents meaningful and precise information. It is widely accepted that the final result of a response (i.e. innate immunity response) derives from all the proteins and compounds intervening in it. Therefore, with the detection or determination of many of the critical mutations a more reliable data of this final result can be obtained.

On the other hand, the detection of a particular frameshift mutation in a *tlr* gene will also have impact on protein function and on the innate immunity system (response) of the animal.

The critical mutations in each of canine TLR2, TLR5, TLR6 and TLR8, were detected in animals (dogs and wolves) some of them suffering from leishmaniosis, and demodicosis. In particular, mutation S516L in SEQ ID NO: 1 (canine TLR2) has been detected in German Shepherd Dog and in French Bulldog in cohorts with leishmaniosis, and/or demodicosis. The mutation V157A in SEQ ID NO: 4 (TLR8) is present in Yorkshire and French Bulldog in a cohort with demodicosi. Therefore, an application of the array of the invention derives from allowing determination of some critical mutations that may be shared in a particular animal cohort. This knowledge may help in the development of specific therapies or assays in homogenized animal cohorts. Besides, the array may also be useful in the detection of particular susceptibilities to diseases.

With regard to critical mutations in TLR2 of SEQ ID NO: 1, an aspect of the invention is a peptide sequence comprising SEQ ID NO: 48 (TINAFLKEQL), as well as an oligonucleotide codifying for said SEQ ID NO: 48. This peptide sequence is a fragment of the TLR2 polypeptide of SEQ ID NO: 1, having a change of a serine (S) by a leucine (L) at position 516 of this SEQ ID NO: 1. This is a deleterious mutation (according to Provean informatics tool), and affects the function of the protein according to SIFT bioinformatics tool, and still is a probably damaging mutation according to the bioinformatic predicting tool Polyphen-2.

With regard to critical mutations in TLR6 of SEQ ID NO: 3, an aspect of the invention is a peptide sequence comprising SEQ ID NO: 49, (KVKVLDX¹HDN RIRSIPKPIM KLEDLQELNV ASNSLAHFPD CGTFNRLSVL IIDSNSISNP SADFLQSCHN IRSMSAGNNP FQCTCELREF VQSLGQVASK VVEGWPDSYK CDSPENYKGT LLKDFHVSX²L), wherein X¹ is selected from I and F, and X² is selected from P and L, with the proviso that at least one of X¹ or X² is, respectively, F or L. Another aspect is further an oligonucleotide codifying for said SEQ ID NO: 49. This peptide sequence is a fragment of canine TLR6 polypeptide of SEQ ID NO: 3 having the change of a leucine (L) by a phenylalanine (F) at position 457 and a change of a proline (P) by a leucine (L) at position 579 of canine of this SEQ ID NO: 3. Both are deleterious mutations, according to Provean informatics tool, affect the function of the protein according to SIFT bioinformatics tool, and still are probably damaging mutations according to the bioinformatic predicting tool Polyphen-2.

With regard to critical mutations in TLR8 of SEQ ID NO: 4, an aspect of the invention is a peptide sequence comprising SEQ ID NO: 50 (QNNIIWATKK), as well as an oligonucleotide codifying for said SEQ ID NO: 50. This peptide sequence is a fragment of canine TLR8 polypeptide of SEQ ID NO: 4 having the change of a valine (V) by an alanine (A) at position 157 of SEQ ID NO: 4. It is a deleterious mutations, according to Provean informatics tool, it affects the function of the protein according to SIFT bioinformatics tool, and still is a probably damaging mutation according to the bioinformatic predicting tool Polyphen-2.

With regard to critical mutations in TLR5 of SEQ ID NO: 2, an aspect of the invention is a peptide sequence comprising SEQ ID NO: 51 (GVTLFLLAVL), as well as an oligonucleotide codifying for said SEQ ID NO: 51. This peptide sequence is a peptide fragment of canine TLR5 of SEQ ID NO: 2, that includes the change of a leucine (L) by a phenylalanine (F) at position 888 of of this SEQ ID NO: 2

Bioinformatic tools, Provean, SIFT and Polyphen-2 used in the present invention are referenced in the examples.

Therefore, another aspect of the invention is an in vitro method for the analysis of the genetic predisposition of a dog to suffer from any disease related with dysfunctions of the innate immunity system, the method comprising carrying out the method as defined above, including the detection of particular *tlr* gene mutations, and correlating any detected mutation with a disease.

Information from *tlr* gene mutations represents, indeed, an imprint or image of the innate immunity profile of an animal, in this case of a dog or a dog breed, generically in an individual of the *Canis* genera, including dogs (*Canis lupus familiaris* subspecies) and wolfs (*Canis lupus* species). Thus, another aspect of the invention is an in vitro method for the analysis of the canine innate immunity profile, the method comprising determining the presence or absence of canine toll-like receptor gene mutations by means of the DNA array as defined above.

This aspect can also be formulated as an in vitro method for the analysis of the canine innate immunity profile, the method comprising determining the presence or absence of canine toll-like receptor mutations selected from
(i) A change of a serine (S) by a leucine (L) at position 516 of canine TLR2 polypeptide of SEQ ID NO: 1;
(ii) A change of a phenylalanine (F) by a cysteine (C) at position 888 of canine TLR5 polypeptide of SEQ ID NO: 2;
(iii) A change of a leucine (L) by a phenylalanine (F) at position 457 of canine TLR6 polypeptide of SEQ ID NO: 3; and
(iv) A change of a valine (V) by an alanine (A) at position 157 of canine TLR8 polypeptide of SEQ ID NO: 4.

A global knowledge of the possible functionality status of innate immunity effectors (i.e., the proteins and routes involved in the response), allows predicting the most probable response type.

At the same time, knowing the innate immunity profile or, in other words, the genomic profile of this response by means of TLR sequences of an individual, permits classifying said individual to homogeneous groups with regard to these features (sequences of TLR). Homogeneous groups of dogs or wolves may be important for the study of some metabolic routes or to perform clinical trials without the individual variability factor.

In the same way, knowing the TLR profile as such may be useful not only to homogenize a test canine population for a particular assay, but also for performing prevalence studies of infectious diseases or microbiome studies, in which TLR may be involved.

Finally, the inventors have also proved that surprisingly TLR genes or the corresponding codified proteins may be used as a way to unequivocally identify *Canis lupus* genera individuals. Indeed, by determining some TLR mutations by means of the array as defined above, sufficient information is provided from an individual to distinct it from another one. This use of TLR genes opens the possibility of identifying waste material, or other samples from an individual and to correlate the sample with said individual. Thus, another aspect of the invention is an in vitro method for the individual identification of a *Canis lupus* genera animal, comprising determining the presence or absence of canine toll-like receptor mutations in an isolated sample by means of the DNA array as defined above; and establishing a combination of detected and non-detected mutations in said sample.

### DETAILED DESCRIPTION OF THE INVENTION

In the sense of the present invention, the term "canine" relates to the family of *Canidae* including domestic dogs, wolves, foxes, jackals, coyotes, and other dog-like mammals. This family includes the sub-family *Caninae* including in turn the genus *Canis* (including dogs, wolves, coyotes and jackals). Among the *Canis* species there is the *Canis lupus* (primarily represented by wolves) specie, with the sub-species *Canis lupus familiaris* (represented by domestic dogs). When canine is used in the present invention, it encompasses in particular the species and subspecies *Canis lupus* and *Canis lupus familiaris.* Thus, by "canine toll-like receptor (TLR) is to be understood the TLR of any of the individuals of the Canis genus, and in particular of the species *Canis lupus* (wolves) and *Canis lupus familiaris* (dogs).

Oligonucleotides are short (from 10 to 60 nucleotide monomers), single-stranded DNA or RNA molecules that have a wide range of applications in genetic testing, research, and forensics. Commonly made in the laboratory by solid-phase chemical synthesis, these small bits of nucleic acids can be manufactured with any user-specified sequence, and so are vital for artificial gene synthesis, polymerase chain reaction (PCR), DNA sequencing, library construction and as molecular probes. In nature, oligonucleotides are usually found as small RNA molecules that function in the regulation of gene expression (e.g. microRNA), or are degradation intermediates derived from the breakdown of larger nucleic acid molecules. Oligonucleotides are characterized by the sequence of nucleotide residues that make up the entire molecule. The length of the oligonucleotide is usually denoted by "-mer" (from Greek meros, "part"). For example, an oligonucleotide of six nucleotides (nt) is an hexamer, while one of 25 nt would usually be called a "25-mer". Oligonucleotides readily bind, in a sequence-specific manner, to their respective complementary oligonucleotides, DNA, or RNA to form duplexes or, less often, hybrids of a higher order. This basic property serves as a foundation for the use of oligonucleotides as probes for detecting DNA or RNA. Examples of procedures that use oligonucleotides include DNA microarrays, Southern blots, ASO analysis, fluorescent in situ hybridization (FISH), and the synthesis of artificial genes. Oligonucleotides are also indispensable elements in antisense gene therapy.

In the sense of the present invention a "gene fragment that in the entire nucleotide sequence of the gene codifies for a change of amino acids", for example, codifying for a change of a serine (S) by a leucine (L) at position 516 of canine TLR2 polypeptide of SEQ ID NO: 1, refers to a nucleic acid fragment, which when forming part of the complete *tlr2* gene, said fragment gives rise to a mutated protein in the indicated amino acid position after the transcription and translation of the entire gene. Usually, the gene fragments are oligonucleotides as above defined, thus from 10 to 30 nucleotides.

For "frameshift mutation" is to be understood as a genetic mutation caused by indels (insertions or deletions) of a number of nucleotides in a DNA sequence that is not divisible by three. Due to the triplet nature of gene expression by codons, the insertion or deletion can change the reading frame (the grouping of the codons), resulting in a completely different translation from the original. The earlier in the sequence the deletion or insertion occurs, the more altered the protein. A frameshift mutation is not the same as a single-nucleotide polymorphism in which a nucleotide is replaced, rather than inserted or deleted. A frameshift mutation will in general cause the reading of the codons after the mutation to code for different amino acids. The frameshift mutation will also alter the first stop codon ("UAA", "UGA" or "UAG") encountered in the sequence. The polypeptide being created could be abnormally short or abnormally long, and will most likely not be functional.

For "single-nucleotide polymorphism" is to be understood as DNA sequence variation occurring when a Single Nucleotide - A, T, C or G - in the genome (or other shared sequence) differs between members of a biological species or paired chromosomes.

A "non-synonymous mutations" or "non-synonymous substitution" is a nucleotide mutation that alters the amino acid sequence of a protein. It is contrasted with synonymous substitutions which do not alter amino acid sequences. As non-synonymous substitutions result in a biological change in the organism, they are subject to natural selection.

When in the present invention the expression "In vitro" is used it encompasses any method modality or use, in which an study is conducted using components of an organism that have been isolated (isolated samples) from their usual biological surroundings in order to permit a more detailed or more convenient analysis than can be done with whole organisms. An "isolated sample" relates according to the invention to any sample (specimen) obtained from an individual and from which a DNA analysis can be performed. Examples of samples include plasma, urine, serum, saliva, semen, etc., as well as in-silico data contained in a computer-readable support (CD, diskettes, MP3).

In the present description the "innate immunity profile", relates to the gene polymorphisms in Toll-like receptors (TLRs) that are pattern recognition receptors (PRRs) considered to be the primary sensors of pathogens in innate immunity, being able to face pathogens in a first immunity response, much before the adaptive immunity response (that implying T and B lymphocytes) is properly activated.

A mutation may be indicated making reference to the alteration of the gene (nucleotide) sequence, or at protein level, which means that it is indicated the change in the amino acid sequence occasioned due to the nucleotide change in the gene. The nucleotide change may be a deletion of one or more nucleotides, an insertion of one or more nucleotides, or a change of a nucleotide for another with a different base (substitution). When in the present invention the mutation is indicated making reference to the gene alteration, if the alteration is an indel (insertion-deletion), it is indicated by "-". If the mutation is a change of a nucleotide, it is indicated using the alternating bases, such as G/T (a change of a guanine by a thymine), T/G, T/C, etc., and also the nucleotide position in the corresponding chromosome where the change takes place, such as G/T 3:73542337, which means that the change is in chromosome 3 of *Canis lupus familiaris,* at the nucleotide position 73542337 according to SNP data base from NCBI http://www.ncbi.nlm.nih.gov/snp/ for the canine chromosomes (equivalent data is retrievable from the database ENSEMBL CanFam3.1 - September 2012; http://www.ensembl.org). If the mutation implies a change in the amino acid sequence, the mutation can also be expressed indicating the two amino acids which have been changed in a particular position of the polypeptide sequence, such as S281 A of SEQ I D NO: 1, which means that at position 281 of SEQ I D NO: 1 (for example) a serine has been substituted by an alanine. Mutations referred in the present invention are preferably disclosed using the amino acid exchange that takes place in the final protein (i.e. in TLR 1-10) codified for a *tlr* gene (*tlr* 1 -10). A change of an amino acid by another may be caused by several base pair mutations in the corresponding nucleotide sequence. Any of the degenerate codons for an amino acid causing the amino acid change have to be considered as encompassed in the *tlr* (1-10) gene fragments of the arrays. As an example, a change of a (S) by a leucine (L) at position 516 of canine TLR2 polypeptide of SEQ ID NO: 1 can take place if the codon for a serine at this position of the open reading frame (for transcribing and translating) is changed due to the mutation of one or more bases of said codon to finally represent a leucine in the same open reading frame. Codon degeneracy and codon tables are well known by the skilled man in the art.

When referring to the gene sequence (nucleotide sequences) the invention uses cursive letters (*tlr 1-10* gene). When referring to the amino acid sequences of the proteins codified by said canine genes, capital letters are used (TLR 1-10 protein). If not indicated the contrary, all *tlr 1-10* genes and fragments thereof, and all the TLR 1-10 proteins are from *Canis* genera, and in particular from *Canis lupus* specie.

Present invention relates to DNA arrays comprising oligonucleotides (i.e. probes) capable of hybridizing with the canine t/r genes or fragments of an isolated sample, said genes including mutations leading to the following changes in the codified protein sequences: S516L in SEQ ID NO: 1, F888C in SEQ ID NO: 2, L457F in SEQ ID NO: 3; and V157A in SEQ ID NO: 4. Thus, the arrays comprise also *tlr* gene fragments including the above mentioned mutations. As above exposed the oligonucleotides or probes hybridize with *tlr* genes or fragments of an isolated sample, preferably at a temperature from 50 ºC to 70 ºC, more preferably 60 ºC as for a standard TaqMan® type probe. Obviously, other similar conditions may apply depending of the technology used to detect if hybridization took place. The skilled man will know how to adapt the hybridization conditions.

With the detection of at least one of these mutations it can be concluded that the individual will have probably an impaired innate immunity response, or will be susceptible to suffer from any disease related with dysfunctions of the innate immunity system, because at least one of the *tlr* genes is codifying for an altered protein (TLR).

SEQ ID NOs: 1, 2, 3 and 4 correspond to the protein sequences of the Ensembl database CanFam3.1, release 75 at filing date and being the version of February 2014. In particular,
SEQ ID NO: 1 corresponds with the identification number Ensembl Protein ID ENSCAFP00000012269.2, codified by gene Ensembl Gene ID ENSCAFG00000008351, Ensembl version 2;
SEQ ID NO: 2 corresponds with the identification number Ensembl Protein ENSCAFP00000016726.3, codified by gene Ensembl Gene ID ENSCAFG00000011368, Ensembl version 3;
SEQ ID NO: 3 corresponds with the identification number Ensembl Protein ID ENSCAFP00000023836.3 codified by gene Ensembl Gene ID ENSCAFG00000016172, Ensembl version 3; and
SEQ ID NO: 4 corresponds with the identification number Ensembl Protein ID ENSCAFP00000031505.3, codified by gene Ensembl Gene ID ENSCAFG00000023498, Ensembl version 3.
All genes ID are also entries of CanFam3.1 database, release 75 at filing date and being the version of February 2014.

In a particular embodiment, the canine *tlr* gene fragments of i) to iv) comprise, respectively, the nucleotide sequences SEQ ID NO: 11 to 13, wherein SEQ ID NO: 11 in the entire nucleotide sequence of *tlr 2* gene codifies for the mutation S516L in SEQ ID N0:1; SEQ ID NO: 12 in the entire nucleotide sequence of *tlr* 6 gene codifies for the mutation L457 in SEQ ID NO: 3; SEQ ID NO: 13 in the entire nucleotide sequence of *tlr* 8 gene codifies for the mutation V157A in SEQ ID NO: 4; and SEQ ID NO: 47 in the entire nucleotide sequence of *tlr 5* gene codifies for the mutation F888C in SEQ ID NO: 2.

The fact that a nucleotide sequence codifies for a mutation, means as above exposed that this *tlr* gene fragment would give raise to the mutated protein, if forming part of the whole gene. These sequences of the DNA arrays are capable of hybridizing with the complementary chains from a DNA sample isolated from a subject (either with the codifying or the complementary chain including the *tlr* gene in question)

These *tlr* gene fragments are oligonucleotides comprising any of SEQ ID NO: 11 to 13, and 47, in particular from 10 to 30 nucleotides, which can hybridize with canine toll-like receptor gene fragments derived or obtained from an isolated sample of a subject (dog or wolf). Indeed, the *tlr* gene fragments of the array may act as DNA probes (i.e. TaqMan® probes).

In a particular embodiment, optionally in combination with any embodiment above or below, the *tlr* gene fragments in the array have a length comprised from 10 to 20 nucleotides. In a preferred embodiment the *tlr* gene fragments in the array have a length selected from 10, 11, 12, 13, 14, 15, 16, 17, 18, 19, and 20 nucleotides.

These gene fragments may be disposed in the array in separated spots or areas (one area for a particular probe), wherein the gene fragments can be in lyophilized form ready to be suspended with any reaction buffer (for example a PCR buffer) in order to promote hybridization with a canine *tlr* gene fragments of a sample.

In another particular embodiment, optionally in combination with any embodiment above or below, the DNA array according the invention further comprises primers for amplifying toll-like receptor genes, or fragments thereof, codifying for any of the mutations or for the wild-type *tlr* genes. In a more particular embodiment, the primers have a length from 12 to 40 nucleotides. This particular embodiments relate to arrays further comprising in separate spots the combination of primers/probe for amplifying in an isolated sample the canine *tlr* gene fragment, in which the particular mutation is desired to be assayed, detected or verified. Examples of commercial arrays including primers and probes for the detection of other genes are those of the type TaqMan Open Array ® of Life Technologies.

In a particular embodiment the *tlr* gene fragments comprising, respectively, any of SEQ ID NO: 11 to 13 and 47, are thus conceived as DNA Taqman® probes, thus comprising a fluorophore at the 5'-end and a quencher at the 3'-end. In a particular embodiment, the fluorophore is selected from 6-carboxyfluorescein (FAM), tetrachlorofluorescein, and VIC; and the quencher is selected from tetramethylrhodamine and dihydrocyclopyrroloindole tripeptide minor groove binder. The array may also comprise primers and other reagents (nucleotides, Taq polymerase, and buffer) for the amplification of the DNA of an isolated sample. VIC is a fluorescent dye that was originally developed by Applied Biosystems, but is now proprietary to Life Technologies. VIC has an absorbance maximum of 538 nm and an emission maximum of 554 nm, thus emitting in the green-yellow part of the visible spectrum. It is used to fluorescently label oligonucleotides at the 5'-end (for use as probes in a variety of real-time PCR, hybridization, and fluorescence-based genetic analysis applications) and is currently only available from LifeTechnologies. VIC's chemical structure is currently not publicly available.

In another particular embodiment the *tlr* gene fragments of i) to iv) of the first aspect of the invention consist, respectively, in SEQ IDs NO: 11 to 13 and 47, each sequence in the entire corresponding *tlr* gene codifying for a TLR amino acid sequence with the above indicated mutations.

Besides the mutations indicated above, in a particular embodiment in combination with any of the embodiments above or below, the array further comprises one or more canine *tlr* gene fragment selected from the group consisting of:
v) A *tlr1* gene fragment that in the entire nucleotide sequence of the gene codifies for a change of a serine (S) by an alanine (A) at position 281, and/or for a change of a valine (V) by an isoleucine (I) at position 312 of TLR1 polypeptide of SEQ ID NO: 5;
(vi) A *tlr2* gene fragment that in the entire nucleotide sequence of the gene codifies for a change of a threonine (T) by a methionine (M) at position 606 of canine TLR2 polypeptide of SEQ ID NO: 1
(vii) A *trl 3* gene fragment that in the entire nucleotide sequence of the gene codifies for a change of a glutamic (E) by a glutamic (D) at position 176 of canine TLR3 polypeptide of SEQ ID NO: 6;
(viii) A *tlr 4* gene fragment that in the entire nucleotide sequence of the gene codifies for a change of a valine (V) by a methionine (M) at position 82, and/or a change of an alanine (A) by a threonine (T) at position 347, and/or a change of a threonine (T) by an alanine (A) at position 577 of canine TLR4 polypeptide of SEQ ID NO: 7;
(ix) A *tlr 5* gene fragment that in the entire nucleotide sequence of the gene codifies for a change of a change of a glutamic (E) by an aspartic (D) at position 169, and/or a change of a serine (S) by an arginine (N) at position 177, and/or a change of a valine (V) by a leucine (L) at position 296, and/or a change of a leucine (L) by a serine (S) at position 383, and/or a change of an arginine (R) by a glutamine (Q) at position 402, and/or a change of an arginine (R) by a glutamine (Q) at position 416, and/or a change of a valine (V) by an isoleucine (I) at position 296, and/or a change of a glycine (G) by a serine (S) at position 533, and/or a change of an arginine (R) by a glutamine (Q) at position 734, and/or a change of a glutamic (D) by a tyrosine (Y) 767, and/or a change of a asparagine by a lysine (K) at position 833, and/or a change of an arginie (R) by a cysteine (C) at position 844, and/or the presence of a leucine (L) instead of a serine (S) at position 850, and/or a change of an alanine by a threonine (T) at position 896, and/or a change of an hystidine (H) by a tyrosine (Y) at position 1017, and/or a change of a glycine (G) by a serine (S) at position 1020, and/or a change of an arginine (R) by a glutamine (Q) at position 1049, and/or a change of an alanine (A) by a threonine (T) at position 1078 of canine TLR5 polypeptide of SEQ ID NO: 2);
(x) A *tlr 6* gene fragment that in the entire nucleotide sequence of the gene codifies for a change of a tyrosine (Y) by a cysteine (C) at position 182 of canine TLR6 polypeptide of SEQ ID NO: 3;
(xi) A *tlr 10* gene fragment that in the entire nucleotide sequence of the gene codifies for a change of a methionine (M) by a valine (V) at position 592 of canine TLR10 polypeptide of SEQ ID NO: 8;
(xii) A *tlr* 7 gene fragment that in the entire nucleotide sequence of the gene codifies for a change of an alanine by a glycine at position 16, and/or a change of a phenylalanine (F) by a leucine (L) at position 167, and/or a change of a proline (P) by a leucine (L) at position 1066 of canine TLR7 polypeptide of SEQ ID NO: 9;
(xiii) A *tlr 8* gene fragment that in the entire nucleotide sequence of the gene codifies for a change of an arginine (R) by glutamine (Q) at position 298 of canine TLR8 polypeptide of SEQ ID NO: 4;
(xiv) A *tlr 9* gene fragment that in the entire nucleotide sequence of the gene codifies for a change of a valine (V) by an isoleucine (I) at position 87, and/or a chang eof a lysine (K) by a glutamic (E) at position 381, and/or a change of a proline (P) by a threonine (T) at position 459, and/or a proline (P) by a leucine (L) at position 787, and/or an arginine (R) by a tryptophan (W) at position 862 of canine TLR 9 polypeptide of SEQ ID NO: 10, and combinations of said gene fragments

For combination of gene fragments is to be understood a combination of two of the gene fragments in the array, three of them, four, five, and until a number including so many gene fragments in such a way that at least one gene fragment codifying in the entire gene for the each one of mutations indicated from (v) to (xiv) is present in the array. In the same way, the expression "one or more" indicates that the array may include combinations of different *tlr* gene fragments from *tlr* 1 to *tlr* 10, as well as different gene fragments of the same *tlr* gene (i.e., two *tlr 9* gene fragments, both codifying in the entire *tlr 9* gene for a TLR9 amino acid sequence including different mutations).

SEQ ID NOs: 5, 6, 7, 8, 9 and 10 correspond to the protein sequences of the Ensembl database CanFam3.1, release 75 at filing date and being the version of February 2014. In particular, SEQ ID NO: 5 corresponds with the identification number Ensembl Protein ID ENSCAFP00000032660.1, codified by gene Ensembl Gene ID ENSCAFG00000024010, version 1; SEQ ID NO: 6 corresponds with the identification number Ensembl Protein ID ENSCAFP00000011004.3, codified by gene Ensembl Gene ID ENSCAFG00000007406, version 3; SEQ ID NO: 7 corresponds with the identification number Ensembl Protein ID ENSCAFP00000031395.2, codified by gene Ensembl Gene ID ENSCAFG00000003518, version 3; SEQ ID NO: 8 corresponds with the identification number Ensembl Protein ID ENSCAFP00000023840.1, codified by gene Ensembl Gene ID ENSCAFG00000016175, version 1; SEQ ID NO: 9 corresponds with the identification number Ensembl Protein ID ENSCAFP00000017193.3, codified by gene Ensembl Gene ID ENSCAFG00000011698, version 3, and SEQ ID NO: 10 corresponds with the identification number Ensembl Protein ID ENSCAFP00000030804.2, codified by gene Ensembl Gene ID ENSCAFG00000023201, version 2. All genes ID are also entries of CanFam3.1 database, release 75 at filing date and being the version of February 2014.

In a particular embodiment, optionally in combination with any embodiments below or above, the DNA array comprises canine *tlr* gene fragments, wherein the *tlr 1* gene fragment of (v) is selected from a group consisting of a sequence comprising SEQ ID NO: 14, a sequence comprising SEQ ID NO: 15, and combinations thereof; the *tlr 2* gene fragment of (vi) is a sequence comprising SEQ ID NO: 16; the *tlr 3* gene fragment of (vii) is a sequence comprising SEQ ID NO: 17; the *tlr 4* gene fragment of (viii) is selected from a group consisting of a sequence comprising SEQ ID NO: 18, a sequence comprising SEQ ID NO: 19, a sequence comprising SEQ ID NO: 20, and combinations thereof; the *tlr 5* gene fragment of (ix) is selected from a group consisting of a sequence comprising any of sequences SEQ ID NO: 21-35 and SEQ ID NO: 52, and combinations thereof; the *tlr 6* gene fragment of (x) is a sequence comprising SEQ ID NO: 36; the *tlr* 10 gene fragment of (xi) is a sequence comprising SEQ ID NO: 46; the *tlr* 7gene fragment of (xii) is selected from a group consisting of a sequence comprising any of sequences SEQ ID NO: 37-39, and combinations thereof; the *tlr 8* gene fragment of (xiii) is a sequence comprising SEQ ID NO: 40; and the *tlr 9* gene fragment of (xiv) is selected from a group consisting of a sequence comprising any of sequences 41-45, and combinations thereof.
SEQ ID NO: 14 (CATTTGAAATTGAGAAATAC) codifies in the entire *tlr* 1 gene fragment for the mutation S281A in SEQ ID NO: 5; SEQ ID NO: 15 (ACACCAAGTCGTTAGTAAT) codifies in the entire *tlr* 1 gene fragment for the mutation V3121 in SEQ ID NO: 5.
SEQ ID NO: 16 (CCCGCCGTGAGCAG) codifies in the entire *tlr2* gene fragment for the mutation T606M in SEQ ID NO: 1.
SEQ ID NO: 17 (TCTCCAAGAGCTTCTG) codifies in the entire *tlr 3* gene fragment for the mutation E176D in SEQ ID NO: 6.
SEQ ID NO: 18 (CAAAACTGCAGGTGCTGG) codifies in the entire *tlr 4* gene fragment for the mutation V82M in SEQ ID NO: 7; SEQ ID NO: 19 (CCCATGCGGGAAAT) codifies in the entire *tlr 4* gene fragment for the mutation A347T in SEQ ID NO: 7; SEQ ID NO: 20 (AAGTTATTCCGAGTAAGATT) codifies in the entire *tlr 4* gene fragment for the mutation T577A in SEQ ID NO: 7.
SEQ ID NO: 21 (TTCCCCGAAGCCCTG) codifies in the entire *tlr* 5 gene fragment for the mutation E169D in SEQ ID NO: 2; SEQ ID NO: 22 (TGTTTGCCGTCACTCAA) codifies in the entire *tlr* 5 gene fragment for the mutation S177N in SEQ ID NO: 2; SEQ ID NO: 23 (TCCGGGCCGTCACC) codifies in the entire *tlr* 5 gene fragment for the mutation V2961 in SEQ ID NO: 2; SEQ ID NO: 24 (AGGCGCAACAAAG) codifies in the entire *tlr* 5 gene fragment for the mutation L383S in SEQ ID NO: 2; SEQ ID NO: 25 (TCCTTCCGGGAGCTG) codifies in the entire *tlr* 5 gene fragment for the mutation R402Q in SEQ ID NO: 2; SEQ ID NO: 26 (CTCAACCGGATCCC) codifies in the entire *tlr* 5 gene fragment for the mutation R416Q in SEQ ID NO: 2; SEQ ID NO: 27 (TCGCACGGCTTCG) codifies in the entire *tlr* 5 gene fragment for the mutation G533S in SEQ ID NO: 2; SEQ ID NO: 28 (AGCCCCCGGAACAC) codifies in the entire *tlr* 5 gene fragment for the mutation R734Q in SEQ ID NO: 2; SEQ ID NO: 29 (TCAGGTCGAGGCCC) codifies in the entire *tlr* 5 gene fragment for the mutation D767Y in SEQ ID NO: 2; SEQ ID NO: 30 (CGCAGCGCACGTC) codifies in the entire *tlr* 5 gene fragment for the mutation R844C in SEQ ID NO: 2; SEQ ID NO: 52 (CCCAGCTTGCTTGC) codifies in the entire *tlr* 5 gene fragment for the mutation L850S in SEQ ID NO: 2; SEQ ID NO: 31 (CTCGTGGCCGCCAAG) codifies in the entire *tlr* 5 gene fragment for the mutation A896T in SEQ ID NO: 2; SEQ ID NO: 32 (CCAGGTGGGACGCG) codifies in the entire *tlr* 5 gene fragment for the mutation H1017Y in SEQ ID NO: 2; SEQ ID NO: 33 (CCTGGACGGCGCCCT) codifies in the entire *tlr* 5 gene fragment for the mutation G1020S in SEQ ID NO: 2; SEQ ID NO: 34 (CAGCGCCGGTACTT) codifies in the entire *tlr* 5 gene fragment for the mutation R1049Q in SEQ ID NO: 2; SEQ ID NO: 35 (CGCGCGCCCCTC) codifies in the entire *tlr* 5 gene fragment for the mutation A1078T in SEQ ID NO: 2.
SEQ ID NO: 36 (TTCTTTTGCATAATAACC) codifies in the entire *tlr* 6 gene fragment for the mutation Y182C in SEQ ID NO: 3.
SEQ ID NO: 37 (AGGGAGCCTGCTTCT) codifies in the entire *tlr* 7gene fragment for the mutation A16G in SEQ ID NO: 9; SEQ ID NO: 38 (TGCATCGGAAATCG) codifies in the entire *tlr* 7gene fragment for the mutation F167L in SEQ ID NO: 9; SEQ ID NO: 39 (CTTGAGAAGCCCCTTCAG) codifies in the entire *tlr* 7gene fragment for the mutation P1066L in SEQ ID NO: 9.
SEQ ID NO: 40 (CCCTCCGGAAGATT) codifies in the entire *tlr 8* gene fragment for the mutation R298Q in SEQ ID NO: 4.
SEQ ID NO: 41 (TTGTCCACTTCGTCCACC) codifies in the entire *tlr 9* gene fragment for the mutation V871 in SEQ ID NO: 10; SEQ ID NO: 42 (CGCTCAGCAAGACCA) codifies in the entire *tlr 9* gene fragment for the mutation K381 E in SEQ ID NO: 10; SEQ ID NO: 43 (CCCAGTGGGCCCAGAG) codifies in the entire *tlr 9* gene fragment for the mutation P459T in SEQ ID NO: 10; SEQ ID NO: 44 (ACGGCTAGGCAGGCC) codifies in the entire *tlr 9* gene fragment for the mutation P787L in SEQ ID NO: 10; SEQ ID NO: 45 (CCCGCCGCCGCC) codifies in the entire *tlr 9* gene fragment for the mutation R862W in SEQ ID NO: 10.
SEQ ID NO: 46 (CCACAGCCATCCCCAG) codifies in the entire *tlr* 10 gene fragment for the mutation M592V in SEQ ID NO: 8.

In another particular embodiment, optionally in combination with any of the embodiments below or above, the canine *tlr* gene fragments of v) to xiv) of the first aspect of the invention consist, respectively, in SEQ IDs NO: 14 to 46 and SEQ ID NO: 52, each sequence in the entire corresponding *tlr* gene codifying for a TLR amino acid sequence with the above indicated mutations

As above indicated for SEQ ID NO: 11-13 and 47, any of SEQ ID NO: 14 to 46 and 52, or a *tlr* gene fragment comprising any of them, may act as DNA probes (i.e. TaqMan probes). In a particular case, the *tlr* gene fragments comprising or consisting in any of SEQ ID NO: 14 to 46 and SEQ ID NO: 52 are conceived as DNA Taqman probes, thus comprising a fluorophore at the 5'-end and a quencher at the 3'-end. The array may also comprise primers and other reagents (nucleotides, Taq polymerase, and buffer) for the amplification of the DNA of an isolated sample. They may be disposed in the array in separated spots or areas (one area for a particular probe), wherein the gene fragments can be in lyophilized form ready to be suspended with any reaction buffer (for example a PCR buffer) in order to promote hybridization with a canine TLR gene fragment of a sample.

Thus, in those particular embodiments where also mutations indicated from (v) to (xiv) are to be detected, the *tlr* gene fragments in the array have a length comprised from 10 to 30 nucleotides, more in particular from 10 to 20 nucleotides. Most particularly a length selected from 10, 11, 12, 13, 14, 15, 16, 17, 18, 19, and 20 nucleotides.

With the new mutations herewith identified, relevant mutations of canine *tlr* genes can be detected in an isolated sample. All these mutations in the different genes give raise to changes in the amino acid sequence of the proteins (TLR) codified by said genes. As will be detailed in the examples below, most of these mutations lead to deleterious proteins and, thus, they result damaging in the sense that the protein will probably lose its function. Loose of function of a protein is finally translated to an impaired whole functionality of the pathway wherein it is involved. In the present case, these non-functionalities of the proteins defining the TLR lead to innate immunity diseases, such as infectious and autoimmune diseases including, but not limited to chronic enteropathies, osteoarthritis, endometrium infections, sino-nasal aspergillosis, idiopathic lymphoplasmacytic rhinitis, Leishmania infections, atopic dermatitis, inflammatory bowel syndrome (IBS), inflammatory bowel disease (IBD), Chron's disease, viral infections and bacterial infections.

Complementation of the array may be made by adding other probes (oligonucleotides) capable of detecting other mutations in the canine tlrgenes. Examples of these probes are capable of detecting the SNPs selected from the group consisting of: rs23585044, rs23572381, rs23572380, rs22410121, rs8958543, rs22120766, rs22157966, rs22145736, rs22189454, rs22189456, rs22124023, rs22123995, rs24029590, rs9070448, rs9070450, rs9070451, rs9070452, rs9070447, rs9125247, rs24029975, rs23570247, rs24607342, rs24607358, rs9188882, rs22882109, and rs23518574, and combinations of said single nucleotide polymorphisms. All these references are retrievable from database dbSNP "Short Genetic Variations" from the National Center for Biotechnology Information (NCBI) http://www.ncbi.nlm.nih.gov/snp/.

In another particular embodiment, optionally in combination with an embodiment above or below, the DNA array comprises oligonucleotides capable of detecting wild-type canine *tlr* gene fragments. Thus, in a particular embodiment of the array, it further comprises *tlr* gene fragments codifying for the wild-type *tlr* gene in a particular possibly mutated point. These oligonucleotides (i.e. wild-type gene fragments) allow also determining if the test sample comprises wild-type forms of the genes, which are present in heterozygote individuals for said feature or character (i.e. a particular TLR). Alternatively, in case of a homozygote individual, these probes for detecting non-mutated *tlr* genes fragments represent a positive control for the array.

In a particular embodiment, the array comprises wild-type *tlr* gene fragments for each of the *tlr* gene fragments codifying for a specified mutation.

In a particular embodiment, optionally in combination with any embodiments below or above, the DNA array comprises the following pairs of primers (forward and reverse) or pairs of sequences including them usable as primers for elongating the canine *tlr* genes including the points with the possible mutations:
For canineTLR1, SEQ ID NO: 53 (AGTTGGTTTGGCATACAAGCATAGA) and SEQ ID NO: 54 (TCTGAAGTCAGGGTAACCTTGTAGT) for amplifying wild-type and mutated TLR1 gene fragment codifying for the mutation S281 A in SEQ ID NO: 5; and SEQ ID NO: 55 (GACACTTCACTGAAGGCCTTATCT) and SEQ ID NO: 56 (AGATTTTATAGATATAACTTTGTGGCAAATTGAATGC) for amplifying wild-type and mutated TLR1 gene fragment codifying for the mutation V3121 in SEQ ID NO: 5.
For canine TLR2, SEQ ID NO: 57 (CCACCTTACAAATTATGAGAATCAGCAGAA) and SEQ ID NO: 58 (AGCCTGTGAAAGGAATCCAGTTG) for amplifying wild-type and mutated TLR2 gene fragment codifying for the mutation S516L in SEQ ID NO: 1; and SEQ ID NO: 59 (GCCGTGTGCTGTGTCTTG) and SEQ ID NO: 60 (ACAGCCCGTGGAAATGGT) for amplifying wild-type and mutated TLR2 gene fragment codifying for the mutation T606M in SEQ ID NO: 1.
For canine TLR3, SEQ ID NO: 61 (AAGTTCTTCACGCCTCAGTACATT) and SEQ ID NO: 62 (ATTAGGAAGTCAGCTCCAATTGGAAAA) for amplifying wild-type and mutated TLR3 gene fragment codifying for the mutation E176D in SEQ ID NO: 6.
For canine TLR4, SEQ ID NO: 63 (CAGCCATTGCTTCTCCAACTTC) and SEQ ID NO: 64 (CTTGGGCAATGTGTAAAAGCTCAT) for amplifying wild-type and mutated TLR4 gene fragment codifying for the mutation V82M in SEQ ID NO: 7; SEQ ID NO: 65 (AGATGGCAACGGTTGGAAATAGTTA) and SEQ ID NO: 66 (GGAAGTGAGAACAAACTCCTTGAGA) for amplifying wild-type and mutated TLR4 gene fragment codifying for the mutation A347T in SEQ ID NO: 7; and SEQ ID NO: 67 (GAACAAGGACAACAGCATTTTCCAA) and SEQ ID NO: 68 (TCTGATGTTCACAGTCACAAGCA) for amplifying wild-type and mutated TLR4 gene fragment codifying for the mutation T577A in SEQ ID NO: 7.
For canine TLR5, SEQ ID NO: 69 (CACGTTCTCCCTCTTCATCTTCTC) and SEQ ID NO: 70 (CAAAGGCCCCGGAGCTT) for amplifying wild-type and mutated TLR5 gene fragment codifying for the mutation F888C in SEQ ID NO: 2; SEQ ID NO: 71 (GTATCCTCCGCCTGCCATT) and SEQ ID NO: 72 (TTACCTTGAGGAGAGTCTGTTTGC) for amplifying wild-type and mutated TLR5 gene fragment codifying for the mutation E169D in SEQ ID NO: 2; SEQ ID NO: 73 (CTCCGCCTGCCATTTTCC) and SEQ ID NO: 74 (GGTCCTTGAGAAACACTTACCTTGA) for amplifying wild-type and mutated TLR5 gene fragment codifying for the mutation S177N in SEQ ID NO: 2; SEQ ID NO: 75 (CTCCTGCTGAGCTTCAACTACA) and SEQ ID NO: 76 (GCAGCCGCTCCAGGA) for amplifying wild-type and mutated TLR5 gene fragment codifying for the mutation V2961 in SEQ ID NO: 2; SEQ ID NO: 77 (CGTGTTGACAGACGGTTATTTCAGA) and SEQ ID NO: 78 (CTCGAGGCTCCCAATCTGATTTT) for amplifying wild-type and mutated TLR5 gene fragment codifying for the mutation L383S in SEQ ID NO: 2; SEQ ID NO: 79 (GGGAGCCTCGAGCTTCAC) and SEQ ID NO: 80 (ACACGCAGCCGGGATC) for amplifying wild-type and mutated TLR5 gene fragment codifying for the mutation R402Q in SEQ ID NO: 2; SEQ ID NO: 81 (GCTGGGTTCCCTGAGGTC) and SEQ ID NO: 82 (AGGGAGAGCGCCTTGC) for amplifying wild-type and mutated TLR5 gene fragment codifying for the mutation R416Q in SEQ ID NO: 2; SEQ ID NO: 83 (TGCTGCGGCTGGATCTG) and SEQ ID NO: 84 (GGGCGTTCAGGGAGAAGA) for amplifying wild-type and mutated TLR5 gene fragment codifying for the mutation G533S in SEQ ID NO: 2; SEQ ID NO: 85 (GGGCGGTGCTGGGA) and SEQ ID NO: 86 (TGTGGTTCAGGTGCAGCA) for amplifying wild-type and mutated TLR5 gene fragment codifying for the mutation R734Q in SEQ ID NO: 2; SEQ ID NO: 87 (GACCTCACGGCGCTGA) and SEQ ID NO: 88 (GCGTGCTCAGCCTGTTG) for amplifying wild-type and mutated TLR5 gene fragment codifying for the mutation D767Y in SEQ ID NO: 2; SEQ ID NO: 89 (GTGGCTCAACCGGACCAA) and SEQ ID NO: 90 (AGCCCTCCATGGAGACAGA) for amplifying wild-type and mutated TLR5 gene fragment codifying for the mutation R844C in SEQ ID NO: 2; SEQ ID NO: 105 (TCCCGCGCAGACGTG) and SEQ ID NO: 106 (AGCCCTCCATGGAGACAGA) for amplifying wild-type and mutated TLR5 gene fragment codifying for the mutation L850S in SEQ ID NO: 2; SEQ ID NO: 91 (CCTCTTCATCTTCTCCACCGT) and SEQ ID NO: 92 (GCCTTGTAACAGAGGAAGCAAAG) for amplifying wild-type and mutated TLR5 gene fragment codifying for the mutation A896T in SEQ ID NO: 2; SEQ ID NO: 93 (TGCCTGGAGGCCTTCG) and SEQ ID NO: 94 (CACGACCACCAGGACGAG) for amplifying wild-type and mutated TLR5 gene fragment codifying for the mutation H1017Y in SEQ ID NO: 2; SEQ ID NO: 95 (TGCCTGGAGGCCTTCG) and SEQ ID NO:96 (CCCACGACCACCAGGAC) for amplifying wild-type and mutated TLR5 gene fragment codifying for the mutation G1020S in SEQ ID NO: 2; SEQ ID NO: 97 (AGGCACCCGGCCATC) and SEQ ID NO: 98 (CCACGTCCTGCAGATCCT) for amplifying wild-type and mutated TLR5 gene fragment codifying for the mutation R1049Q in SEQ ID NO: 2; and the pair SEQ ID NO: 99 (ACGCTCTCCCGACACATC) and SEQ ID NO: 100 (CGCAGCGGGATGCC) for amplifying wild-type and mutated TLR5 gene fragment codifying for the mutation A1078T.
For canine TLR6, SEQ ID NO: 101 (GTTTTCAGATGTTTACCTCCCAAGGT) and SEQ ID NO: 102 (AGCTTCATGATTGGTTTAGGAATGCT) for amplifying wild-type and mutated TLR6 gene fragment codifying for the mutation L457F in SEQ ID NO: 3; and the pair of primers SEQ ID NO: 103 (ATCTGCTACCAATTGCTCATTT) and SEQ ID NO: 104 (AGTGTTTTTGTATCTAGAATTTGAAGACTTCCT) for amplifying wild-type and mutated TLR6 gene fragment codifying for the mutation Y182C in SEQ ID NO: 3.
For canine TLR7, SEQ ID NO: 107 (AGAGAGAGGCAGGGACACA) and SEQ ID NO: 108 (GGATCGAGTCCCACATCGG) for amplifying wild-type and mutated TLR7 gene fragment codifying for the mutation A16G in SEQ ID NO: 9; SEQ ID NO: 109 (CCAGCTGGACTATCTGGTAGAGAT) and SEQ ID NO: 110 (CCCCAGTCGAACAGGTATACAG) for amplifying wild-type and mutated TLR7 gene fragment codifying for the mutation F167L in SEQ ID NO: 9; and SEQ ID NO: 111 (TCATGGATGAAAAAGTGGACGTCAT) and SEQ ID NO: 112 (GGAGCTGGAGGAACTTGGATTT) for amplifying wild-type and mutated TLR7 gene fragment codifying for the mutation P1066L in SEQ ID NO: 9.
For TLR8, SEQ ID NO: 113 (GGTCTTTGAAGGAACTTAGCCTGAT) and SEQ ID NO: 114 (CGTGAGCCCAGACGTATTCTTT) for amplifying wild-type and mutated TLR8 gene fragment codifying for the mutation V157A in SEQ ID NO: 4; and SEQ ID NO: 115 (GCTTCGCTACCTAAACCTCTCTAG) and SEQ ID NO: 116 (CATGTTGTCAAACCACGTTGCA) for amplifying wild-type and mutated TLR8 gene fragment codifying for the mutation R298Q in SEQ ID NO: 4.
For canine TLR9, SEQ ID NO: 117 (CCACCACCTCCATGACTATGACT) and SEQ ID NO: 118 (GGCAGTTCCACTTGAGATTGAGA) for amplifying wild-type and mutated TLR9 gene fragment codifying for the mutation V871 in SEQ ID NO: 10; SEQ ID NO: 119 (GCTGGACATACATGGCATCTTCTT) and SEQ ID NO: 120 (GGTGGGCCAGCGACT) for amplifying wild-type and mutated TLR9 gene fragment codifying for the mutation K381 E; SEQ ID NO: 121 (GGAGAGAGTCTGGCCACAGT) and SEQ ID NO: 122 (CGGCATGAAGGCCTCTGA) for amplifying wild-type and mutated TLR9 gene fragment codifying for the mutation P459T in SEQ ID NO: 10; SEQ ID NO: 123 (GCTGGAGGTGCAGGCT) and SEQ ID NO: 124 (GGCTGCCGCACTTGAC) for amplifying wild-type and mutated TLR9 gene fragment codifying for the mutation P787L in SEQ ID NO: 10; and SEQ ID NO: 125 (GTGCCTGGCCTGGCT) and SEQ ID NO: 126 (GCCTTGTCGAAGACCACGAA) for amplifying wild-type and mutated TLR9 gene fragment codifying for the mutation R862W in SEQ ID NO: 10.
For canine TLR9, the pair of primers SEQ ID NO: 127 (TCTGTTGATTGTCACCATTGTGGTT) and SEQ ID NO: 128 (CAGATCAAAGTAGAAGCAGCAGAAG) for amplifying wild-type and mutated TLR10 gene fragment codifying for the mutation M592V in SEQ ID NO: 8.

Each of the above listed pair of primers serve also, as indicated, for amplifying the corresponding wild-type canine *tlr* gene fragments, said wild-type gene fragments codifying for the peptide fragments of TLR protein sequences including the amino acid that could be mutated but it is not mutated according to the reference sequences. Reference sequences are those identified by the ENSEMBL database accession number above listed.

Another particular embodiment of the array of the invention, it includes wild-type canine *tlr* gene fragments.

Another aspect of present invention is the peptide sequence comprising SEQ ID NO: 48. In a particular embodiment, the peptide sequence comprising SEQ ID NO: 48, further comprises additionally other mutations, in particular a change of a threonine (T) by a methionine (M) at position 606 of canine TLR2 polypeptide of SEQ ID NO: 1.

In the same way, particular embodiments of the peptide sequences comprising any of SEQ ID NO: 49 to 51, relate to these sequences further comprising other mutations, in particular those mutations identified in embodiments above in the corresponding sequences SEQ ID NO: 1, 2, 3, or 4, such as mutation T606M of SEQ ID NO: 1; mutation Y182C of SEQ ID NO: 3; mutation R298Q of SEQ ID NO: 4; and mutations V2961 and/or L383S of SEQ ID NO: 2.

Another aspect of the invention relates to a method for identifying mutations in canine toll-like receptor genes. In a particular embodiment the method comprises:
- obtaining an isolated sample of an animal of the *Canis* genera comprising DNA;
- adding said isolated animal sample comprising DNA to a DNA array as defined in any of the aspects and embodiments disclosed above; and
- determining if the toll-like receptor gene fragments of the array hybridize with sample gene fragments.

In a particular embodiment of the method for identifying mutations, the isolated DNA sample is added to a DNA array that comprises canine *tlr* gene fragments as those defined from i) to iv) and comprising, respectively, the nucleotide sequences SEQ ID NO: 11 to 13, wherein SEQ ID NO: 11 (CTCCTTCGAGAAAGC) in the entire nucleotide sequence of *tlr 2* gene codifies for the mutation S516L in SEQ ID NO:1; SEQ ID NO: 12 (TTATCGTGAAGATCAAGC) in the entire nucleotide sequence of *tlr 6* gene codifies for the mutation L457 in SEQ ID NO: 3; SEQ ID NO: 13 (CATCATCTGGGTAACGAA) in the entire nucleotide sequence of *tlr 8* gene codifies for the mutation V157A in SEQ ID NO: 4; and SEQ ID NO: 47 (CCAGGAGGAACAGCGT) in the entire nucleotide sequence of *tlr 5* gene codifies for the mutation F888C in SEQ ID NO: 2.

In a more particular embodiment of the method for identifying mutations, the DNA sample is added to an array that further comprises oligonucleotides (i.e. *tlr* gene fragments) comprising any of the sequences SEQ ID NO: 14 to SEQ ID NO: 46, and SEQ ID NO: 52. In a more particular embodiment, the oligonucleotides in the array consist in any combination of oligonucleotides sequences SEQ ID NO: 14 to SEQ ID NO: 46 and SEQ ID NO: 52.

Another particular embodiment of the method for identifying mutations, optionally in combination with any of the embodiments below or above, the step of determining if the toll-like receptor gene fragments of the array (oligonucleotides of the array) hybridize with sample gene fragments codifying for a mutation, is carried out by a means selected from the group consisting of chemiluminescence, fluorescence, and radioisotope technologies.

Determination of hybridization allows concluding if a particular mutation is present or not in a sample.

In another particular embodiment of the method, optionally in combination with any of the embodiments below or above, the sample comprising DNA of an animal of the *Canis* genera, relates to a sample wherein DNA is present or that derives or comes from a sample comprising RNA that has been converted to the corresponding cDNA by means of the retro transcription techniques (for example, RT-PCR).

In any of the methods and arrays of the invention the sample containing DNA is selected from the group consisting of blood, saliva, faeces, urine, skin, hair or any other tissue from which DNA can be obtained.

In a particular embodiment of the method, the DNA sample may be a computer readable support containing the sequence of canine *tlr* genes of an individual, obtained for example by massive sequencing, or from a previous analysis. Then, the sequences are aligned with the sequences defining the oligonucleotides comprised in the array, also in a computer readable support.

As above exposed, the combined determination of the particular mutations gives relevant information regarding a toll-like receptor in question, as well as relevant information regarding innate immunity response, an/or diseases related with an innate immunity dysfunction, such as atopic dermatitis, Inflammaory bowel syndrome (IBS), Inflammatory Bowel disease (IBD), Chron's disease, viral infections and bacterial infections.

Thus, another aspect of the invention is an in vitro method for the analysis of the genetic predisposition of an individual of the *Canis* genera to suffer from any disease related with dysfunctions of the innate immunity system, the method comprising carrying out the method for identifying mutations in canine toll-like receptor genes as defined above, and correlating any detected mutation with a disease. In a particular embodiment, thus, the method for the analysis of the genetic predisposition of an individual of the *Canis* genera to suffer from any disease related with dysfunctions of the innate immunity system comprises determining in an isolated sample of an individual if at least the following mutations are present: a change of a serine (S) by a leucine (L) at position 516 of canine TLR2 polypeptide of SEQ ID NO: 1; a change of a leucine (L) by a phenylalanine (F) at position 457 and a change of a proline (P) by a leucine (L) at position 579 of canine TLR6 polypeptide of SEQ ID NO: 3; and a change of a valine (V) by an alanine (A) at position 157 of canine TLR8 polypeptide of SEQ ID NO: 4. In a more particular embodiment, the method comprises determining in an isolated sample of an individual if at least the following mutations are present: a change of a serine (S) by a leucine (L) at position 516 of canine TLR2 polypeptide of SEQ ID NO: 1; a change of a phenylalanine (F) by a cysteine (C) at position 888 of canine TLR5 polypeptide of SEQ ID NO: 2; a change of a leucine (L) by a phenylalanine (F) at position 457 and a change of a proline (P) by a leucine (L) at position 579 of canine TLR6 polypeptide of SEQ ID NO: 3; and a change of a valine (V) by an alanine (A) at position 157 of canine TLR8 polypeptide of SEQ ID NO: 4

Detection of the mutations may be carried out using the probes (oligonucleotides, which indeed are canine *tlr* gene fragments) as defined in SEQ ID NO: 11, 12, 13, and 47. In another particular embodiment, the mutations in canine *tlr* genes are further detected using any combination of the oligonucleotides comprising or consisting in SEQ ID NO: 14 to SEQ ID NO: 46 and SEQ ID NO: 52.

In a particular embodiment of any of the methods of the invention (for determining mutations, or for the analysis of susceptibility to a disease), as well as of the array to which a DNA sample of an individual will be added, the *Canis* genera individual is selected from the species *Canis lupus* and *Canis lupus familiaris.* In a most preferred embodiment, the individual is from the *Canis lupus familiaris* species (commonly known as domestic dogs). Among the species *Canis lupus familiaris* there are many breeds. The methods and arrays of the invention are applicable to samples of all breeds. In the same way, due to the phylogenetically proximity of wolves (*Canis lupus*), the method and arrays are also applicable to wolves.

Dog breeds are groups of closely related and visibly similar domestic dogs, which are all of the subspecies Canis lupus familiaris, having characteristic traits that are selected and maintained by humans, bred from a known foundation stock.

In another particular embodiment of any of the methods of the invention (for determining mutations, for individual identification or for the analysis of susceptibility to a disease), it further includes the step of further collecting and/or providing and/ or saving data derived from previous steps in a data carrier. A data carrier includes means (paper, CD, digitalized information, computer archives, or sound recordings) that contain meaningful information data.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

Following examples illustrate the arrays of the invention as well as a way of carrying out the methods of the invention in which the presence or absence of relevant mutations in canine TLRs are detected.

### Example 1. Identification of relevant mutations in canine TLRs genes.

For the performance of the Examples the following materials and methods were used

### DNA sources:

Samples available from the DNA bank at the SVGM (Servei Veterinari de Genetica Molecular, Molecular Genetics Veterinary Service, UAB) were used. Total DNA from blood cells had been extracted either as described elsewhere (Francino, O. et al., 2006. Advantages of real-time PCR assay for diagnosis and monitoring of canine leishmaniosis. Veterinary Parasitology 137: 214-221) or using QIAamp DNA Mini Kit (Qiagen).

DNAs from 7 different dog (Canis lupus familiaris) breeds, including Beagle, German shepherd, Yorkshire, French bulldog, Boxer, Labrador and Shar Pei were used. Also 2 different populations of wolves (*Canis lupus*) were analyzed, Iberian and Russian. Polymorphism analyses were performed using DNA pools, which were constituted of 200 ng of DNA from 25 unrelated dogs (in exception of one pool of French bulldogs, which only 10 individuals were used). Two pools of each breed were analyzed, in exception of Shar Pei (only 1 pool) and Boxer (3 pools). Pools of wolves were constituted of 50 individuals.

Some DNA samples used for a first massive sequencing analyses were also chosen to be individually genotyped in order to validate SNPs in a TaqMan Open Array® designed (see Example below). 15 Beagle, 15 Boxer, 15 French bulldog, 15 Labrador, 14 German shepherd dog, 12 Yorkshire, 11 Shar Pei and 4 wolves were used.

Exon capture and massive sequencing for SNP discovery:
Twenty exonic regions of 10 canine tlrgenes annotated in CanFam 2.0 were chosen to perform the analysis. 2372 bp for *tlr* 1 (Ensembl Gene ID ENSCAFG00000024010), 2357 bp for *tlr2* (Ensembl Gene ID ENSCAFG00000008351), 2717 bp for *tlr 3* (Ensembl Gene ID ENSCAFG00000007406), 2522 bp for *tlr4* (Ensembl Gene ID ENSCAFG00000003518),
   2576 bp for *tlr 5* (Ensembl Gene ID ENSCAFG00000011368), 2413 for *tlr 6* (Ensembl Gene ID ENSCAFG00000016172), 4241 bp for *tlr7* (Ensembl Gene ID ENSCAFG00000011698), 3119 bp for *tlr 8* (Ensembl Gene ID ENSCAFG00000023498), 3015 bp for *tlr 9* (Ensembl Gene ID ENSCAFG00000023201) and 2423 bp for *tlr 10* (Ensembl Gene ID ENSCAFG00000016175).

All gene identifications proceed from the database Ensembl project, which is a joint project between European Bioinformatics Institute (EBI), an outstation of the European Molecular Biology Laboratory (EMBL), and the Wellcome Trust Sanger Institute (WTSI). Acces of the database is available at htt://www.ensembl.org.

Oligonucleotides (120 nt) were first automatically designed for the enrichment of selected regions (https://earray.chem.agilent.com/suredesign/). Regions rejected in the automated design, because of the presence of gaps, repeats or shorter sizes than required (at least 120 nucleotides) were manually redesigned. Finally 235 capture oligonucleotides were designed, for Agilent Sure-select library, which covered 28,200 bases. High-throughput sequencing was performed using 2 lanes of Illumina HISEQ, with 8-labelled pools each, at CNAG (Centre Nacional d'Analisi Genomica).

Sequences obtained were mapped to database CanFam 3.1 of Ensembl project (released September 2012). All pools were analysed together for variant calling, for better comparison. Positions with a total coverage of less than 8,000 reads were filtered out. Alternative variant frequencies were estimated for each breed pool and wolf populations. The variants were annotated with statistical information from the Genome Analysis Tool Kit (GATK) and functional annotations were added from Ensembl using snpEff (http://snpeff.sourceforge.net/). Variants already described for dog, human, cow and pig TLRs were retrieved from ENSEMBL (CanFam 3.1).

### Prediction of functional impact of non-synonymous SNPs:

The functional impact of non-synonymous mutations detected was predicted using Polyphen-2 (http://genetics.bwh.harvard.edu/pph2/index.shtml) (Adzhubei I a, Schmidt S, Peshkin L, Ramensky VE, Gerasimova A, Bork P, Kondrashov AS, Sunyaev SR: A method and server for predicting damaging missense mutations. Nat Methods 2010, 7:248-9.), SIFT (http://sift.jcvi.org and referenced in Kumar P et al., "Predicting the effects of coding non-synonymous variants on protein function using the SIFT algorithm",. Nat Protoc.- 2009; Vol. No.4(7), pp. :1073-81) and PROVEAN (http://provean.jcvi.org/index.php and referenced in Choi Y et al., "Predicting the Functional Effect of Amino Acid Substitutions and Indels", PLoS ONE - 2012, Vol. No. 7(10): e46688.). When the mean frequency of an alternative allele on the dog population analysed was more than 0.25, both alleles of those SNPs were tested with algorithms mentioned before.

SMART program was used in order to identify protein domains of each TLR (Letunic I, Doerks T, Bork P: SMART 7: recent updates to the protein domain annotation resource. Nucleic Acids Res 2012, 40(Database issue):D302-5). Multialignments to nearest species was performed with Multialin platform (http://multalin.toulouse.inra.fr/multalin/).

With this approximation, materials and methods, there were detected known mutations as well as other new mutations, all of them identified in Table 1 of Example 2.

### Example 2. DNA array comprising canine tlr gene fragments capable of detecting critical mutations in canine TLR proteins.

An array of type TaqMan OpenArray® was designed. Selected SNPs and their surrounding sequences, 60 nucleotides upstream and 60 nucleotides downstream were introduced in Custom TaqMan® Assay Design Tool web site (www.appliedbiosystems.com/cadt) from Life Technologies® to validate if the sequences were suitable for TaqMan assay design. Other SNPs in the context sequences were indicated with an "N" before the assay designs.

The array comprised oligonucleotides with a length from 10 to 30 nucleotides, which were *tlr* gene fragments and capable of identifying the mutations indicated in next Table 1.

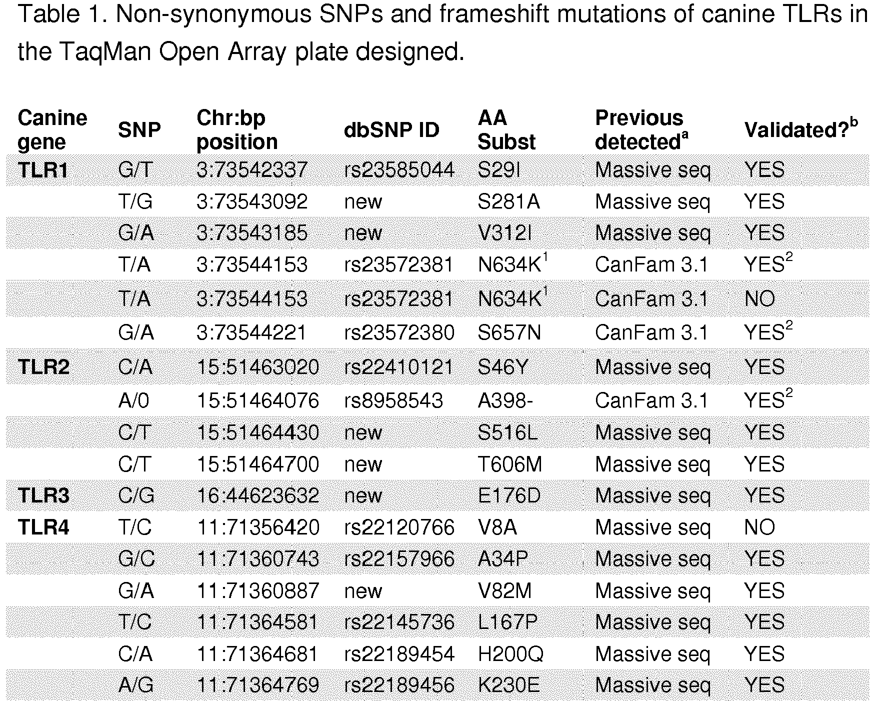

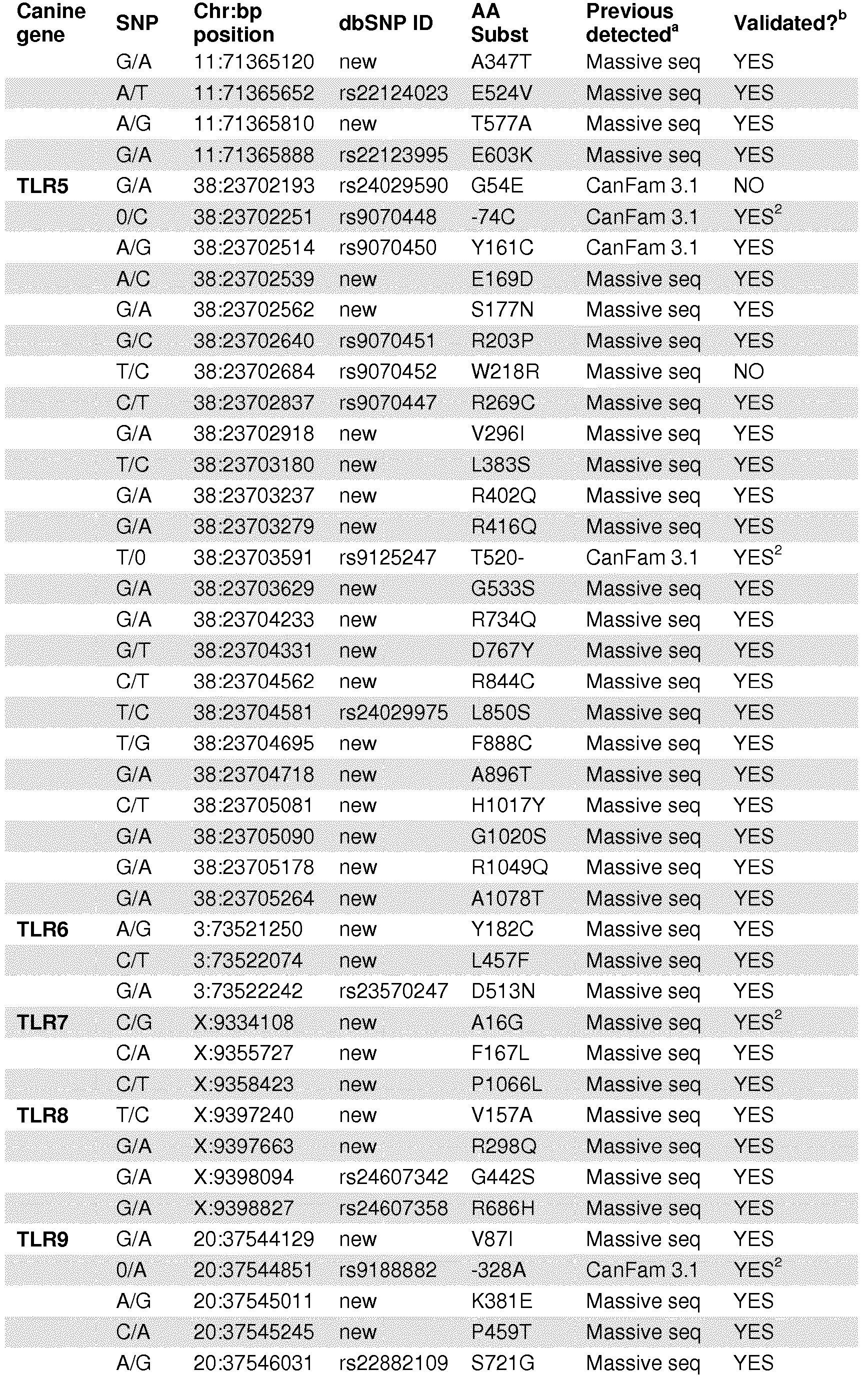

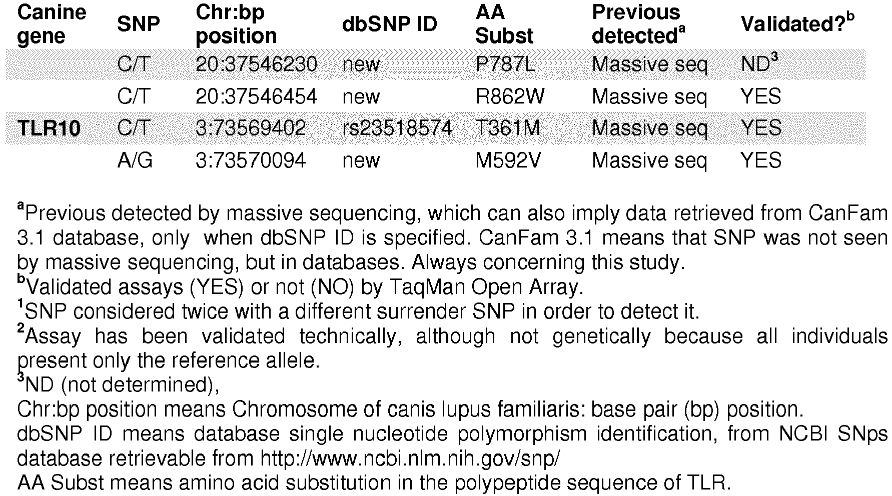

Analysis of PCR results was performed with the TaqMan Genotyper software v.1.3 (Applied Biosystems).

Probes for detecting new non-synonymous mutations included in the array were those indicated in Table 2:

**Table 2. Probes in a TaqMan type array for detecting new non-synonymous mutations in canine TLR genes and proteins.**

| Canine gene / Canine protein | AA subst | Probe sequence |
|---|---|---|
| *tlr*1 / TLR1 | S281A | CATTTGAAATTGAGAAATAC (SEQ ID NO: 14) |
| *tlr*2 /TLR2 | S516L | CTCCTTCGAGAAAGC (SEQ ID NO: 11) |
| *tlr*2 / TLR2 | T606M | CCCGCCGTGAGCAG (SEQ ID NO: 16) |
| *tlr*3 / TLR2 | E176D | TCTCCAAGAGCTTCTG (SEQ ID NO: 17) |
| *tlr*4 / TLR4 | V82M | CAAAACTGCAGGTGCTGG (SEQ ID NO: 18) |
| *tlr*4 / TLR4 | A347T | CCCATGCGGGAAAT (SEQ ID NO: 19) |
| *tlr*4 / TLR4 | T577A | AAGTTATTCCGAGTAAGATT (SEQ ID NO: 20) |
| *tlr*5 / TLR5 | F888C | CCAGGAGGAACAGCGT (SEQ ID NO: 47) |
| *tlr*5 / TLR5 | E169D | TTCCCCGAAGCCCTG (SEQ ID NO: 21) |
| *tlr*5 / TLR5 | S177N: | TGTTTGCCGTCACTCAA (SEQ ID NO: 22) |
| *tlr*5 / TLR5 | V2961 | TCCGGGCCGTCACC (SEQ ID NO: 23) |
| *tlr*5 / TLR5 | L383S | AGGCGCAACAAAG (SEQ ID NO: 24) |
| *tlr*5 / TLR5 | R402Q | TCCTTCCGGGAGCTG (SEQ ID NO: 25) |
| *tlr*5 / TLR5 | R416Q | CTCAACCGGATCCC (SEQ ID NO: 26) |
| *tlr*5 / TLR5 | G533S | TCGCACGGCTTCG (SEQ ID NO: 27) |
| *tlr5* / TLR5 | R734Q | AGCCCCCGGAACAC (SEQ ID NO: 28) |
| *tlr5* / TLR5 | D767Y | TCAGGTCGAGGCCC (SEQ ID NO: 29) |
| *tlr*5 / TLR5 | R844C | CGCAGCGCACGTC (SEQ ID NO: 30) |
| *tlr*5 / TLR5 | A896T | CTCGTGGCCGCCAAG (SEQ ID NO: 31) |
| *tlr*5 / TLR5 | H1017Y | CCAGGTGGGACGCG (SEQ ID NO: 32) |
| *tlr*5 / TLR5 | G1020S | CCTGGACGGCGCCCT (SEQ ID NO: 33) |
| *tlr*5 / TLR5 | R1049Q | CAGCGCCGGTACTT (SEQ ID NO: 34) |
| *tlr*5 / TLR5 | A1078T | CGCGCGCCCCTC (SEQ ID NO: 35) |
| *tlr6* / TLR6 | L457F | TTATCGTGAAGATCAAGC (SEQ ID NO: 12) |
| *tlr6* / TLR6 | Y182C | TTCTTTTGCATAATAACC (SEQ ID NO: 36) |
| *tlr7*/ TLR7 | A16G | AGGGAGCCTGCTTCT (SEQ ID NO: 37) |
| *tlr7*/ TLR7 | F167L | TGCATCGGAAATCG (SEQ ID NO: 38) |
| *tlr7*/ TLR7 | P1066L | CTTGAGAAGCCCCTTCAG (SEQ ID NO: 39) |
| *tlr8* / TLR8 | V157A | CATCATCTGGGTAACGAA (SEQ ID NO: 13) |
| *tlr8* / TLR8 | R298Q | CCCTCCGGAAGATT (SEQ ID NO: 40) |
| *tlr9* / TLR9 | V871 | TTGTCCACTTCGTCCACC (SEQ ID NO: 41) |
| *tlr9* / TLR9 | K381E | CGCTCAGCAAGACCA (SEQ ID NO: 42) |
| *tlr9* / TLR9 | P459T | CCCAGTGGGCCCAGAG (SEQ ID NO: 43) |
| *tlr9* / TLR9 | P787L | ACGGCTAGGCAGGCC (SEQ ID NO: 44) |
| *tlr9* / TLR9 | R862W | CCCGCCGCCGCC (SEQ ID NO: 45) |
| *tlr10* / TLR10 | M592V | CCACAGCCATCCCCAG (SEQ ID NO: 46) |

In addition, the array included the pair of primers for amplifying any *tlr* gene fragment to which the probes for identifying the above cited new mutations could hybridize. In particular, it included primers of SEQ ID NO: 53 to SEQ ID NO: 128.

The array also comprised specially designed TaqMan probes for the rest of mutations identified in Table 1 (i.e. frameshifts mutations and other known non-synonymous mutations). In addition, in order to detect also wild-type alleles of the *tlr* genes, the array comprised TaqMan probes designed for detecting wild-type canine *tlr* gene fragments. These probes were used as positive controls and allowed the detection of heterozygote individuals for said feature or character (i.e. a particular mutation in a TLR protein), as well as the detection of homozygote individuals.

Canine *tlr* gene fragments in the array that were acting as probes incorporated a 5' reporter dye (VIC for probes for detecting mutations, and FAM for probes viewing wild-type or non-mutated canine TLR from a sample), and a 3' nonfluorescent quencher.

In summary, the array according to the invention (from the type TaqMan OpenArray® plate) was developed for the detection and for the validation of the nsSNPs (non-synonymous mutations) by individual genotyping (Table 1). The panel contained (i) 27 out of 31 nsSNPs that were predicted to have an impact on the protein structure (4 wolf-specific SNPs were not considered for the array: TLR1 A525V, TLR5 N833K, TLR6 P579L and TLR10 F787L; see Table 3); (ii) 28 out of the 33 remaining nsSNPs segregating in dogs (5 SNPs that were not suitable for a correct primer design were rejected for posterior analysis: TLR4 T36A, TLR4 T361, TLR5 T243A, TLR5 Q213R and TLR9 A442V); and (iii) 4 frameshift and 4 non-synonymous TLR polymorphisms described on CanFam 3.1 but not detected in the analysed cohorts. One of the non-synonymous variants added (rs23572381, TLR1 N634K) was designed with two different TaqMan assays due to the presence of other variants close to the interrogated SNP.

Table 1 allows concluding that all newly identified mutations can be detected simultaneously in a DNA array specially designed for the study of relevant mutations in canine *tlr* genes and the corresponding TLR expressed proteins. Detection of these mutations may be useful in defining the innate immunity profile; for determining the genetic predisposition of an individual of *Canis* genera to suffer from any disease related with dysfunctions of the innate immunity, for determining variability among races and among individuals into a specific race for example with the aim of homogenising animal cohorts for experimental purposes; and for the unequivocally individual identification of samples.

This particular DNA array represents an interesting tool for genotyping an individual subject of the *Canis* genera (especially dogs), with the aim of finally determining if this individual is susceptible of suffering from any disease related with TLR dysfunctions.

Association of the detected mutations with the susceptibility of suffering from any disease related with TLR dysfunctions is derivable from next Table 3, wherein the impact of newly identified and known canine TLRs Amino Acid substitutions was predicted using bioinformatics tools.

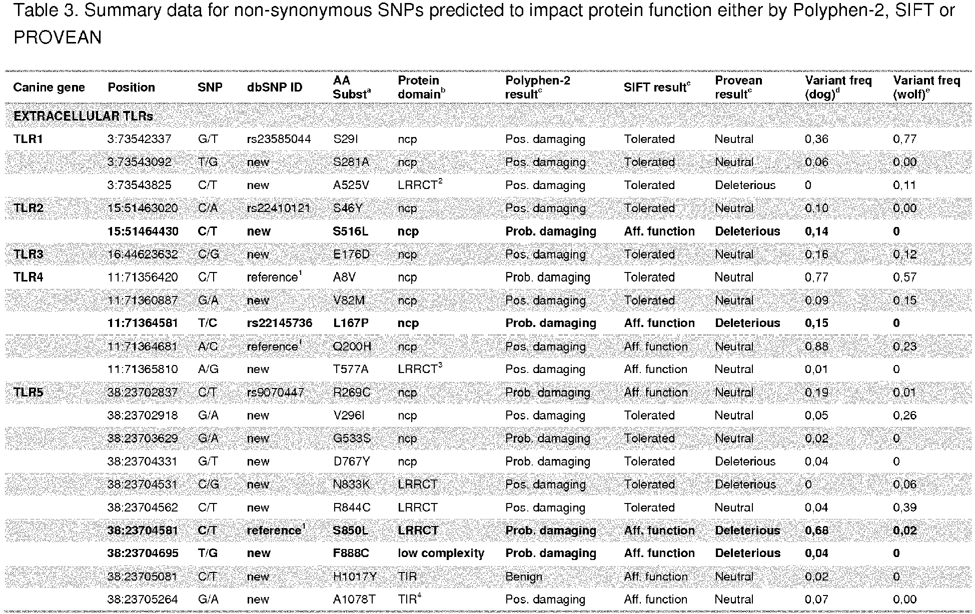

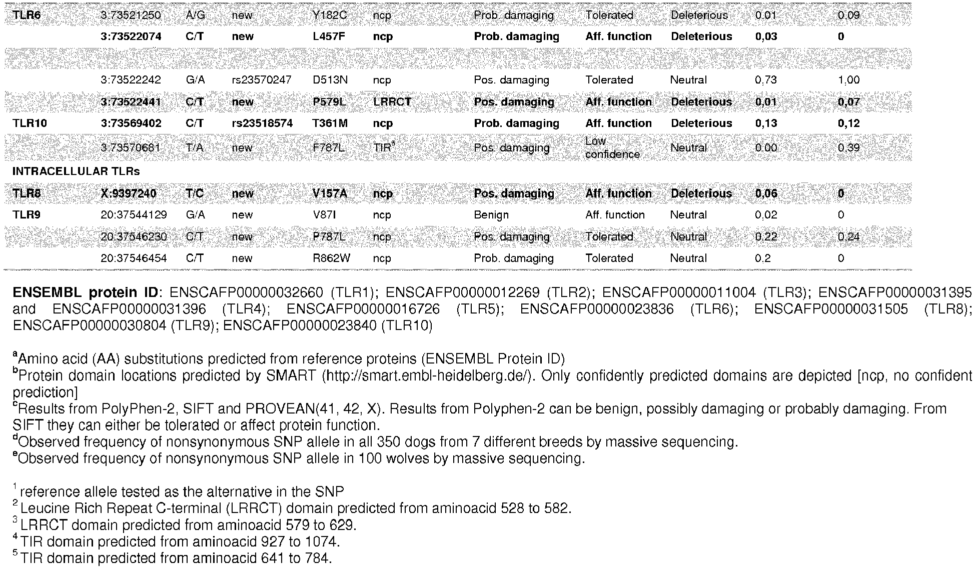

Polyphen-2, SIFT and PROVEAN tools were used in order to predict the effect of each new non-synonymous SNP (nsSNPs) in the protein structure (Table 3). 33 out of 64 nsSNPs were predicted as benign, tolerated and neutral using the three algorithms mentioned. 31 out of 64 nsSNPs were predicted to have an effect on protein structure by at least one of the tools used.

Results from Polyphen-2, SIFT and PROVEAN were convergent in predicting damaging effects of 8 out of 31 nsSNPs (27%). 6 out of 64 were not correctly predicted, giving unknown or low confidence results, because they were not aligning to enough similar sequences to give a reliable result. Curiously those 6 nsSNPs were all located on N-terminal of TLR5. Suggesting that those first aminoacids were not part of TLR5 of other species, perhaps due to a bad annotation of this protein (discussion)

Frequencies of nsSNPs on table 3 are an average of all dog breeds tested and both wolf populations respectively, so all variants are polymorphic at least in one breed (minimum allele frequency MAF>0.05). Looking at frequencies of nsSNPs predicted to be damaging in canine TLRs, it can be seen that 17 out of 31 show a MAF > 0.05 (15 out of 31 with MAF> 0.05 in wolf populations).

Protein structure of TLRs was assessed using SMART (Letunic I, Doerks T, Bork P (2012) SMART 7: recent updates to the protein domain annotation resource. Nucleic Acids Res 40:D302-305), which predicts domains taking into account aminoacid sequences. 6 out of 31 nsSNPs predicted to be damaging in canine TLRs were found to be in a Leucine Rich Repeat C-terminal (LRRCT) or really close to it. Only 1 out of 31 was found to affect TIR domain in TLR 5, another 2 were found to be really close to this domain in TLR5 and 10. With the exception of these last ones, damaging SNPs were in most cases located in the sensor domain of TLRs.

### Example 3. Individual identification of dogs from a beagle cohort and other breeds using the array of the invention.

Finally, in order to show the application of the array of the invention for unequivocally identifying an individual among a group (even of the same breed), the inventors compared the data derived from DNA samples of the 15 Beagles. The array comprised, as above indicated, the probes of Table 2, these probes are indeed *tlr* gene fragments, which in the corresponding *tlr* gene, are codifying for some mutations. The array also comprised other probes and means for the detection of the other mutations listed in Table 1.

Although data not shown, the read of the nucleotides in all these possibly mutated points of TLR genes and corresponding proteins using the array of the invention, revealed enough information from each individual for distinguishing between two animals.

Results were also confirmed in other breeds such as German Shepherd, Labrador, Boxer, Yorkshire terrier, French bulldog and Shar-pei.

The data derivable from the array may be obtained from any sample of a canine subject meanwhile the sample contains DNA or a nucleic acid transformable to DNA (i.e. RNA). Examples of samples are waste material of the subject (defecation and urine), as well as blood, semen, saliva, a biopsy, etc.

A direct use of the information is the identification of samples, in particular of waste material that can be found in the boundaries of streets or in particular gardens. Correct identification (in some cities compulsory) of dogs and their tutors allows further determining the origin of a particular waste material. Identification of the waste material will serve for sanctioning non civic behaviours.

Concluding, with the array and methods of the invention, profile of innate immunity is obtained for each of the analyzed samples. This profile may serve to decide a particular medical regimen for an individual, as well as to conclude a diagnosis. In particular, the suffering from diseases related with dysfunction of innate immunity, due to critical mutations in canine TLR2, TLR5, TLR6 and TLR8.

In addition, an innate immunity profile is a usable tool for the selection of a homogenized population among a canine breed.

The fast and economical detection of the sequences of TLRs by means of the proposed array is advantageous and aims saving cost in research studies or in clinical trials. Indeed, A the TaqMan OpenArray designed for high-throughput genotyping of TLRs in dogs, serves either to study TLR evolution in the canine genome, the association to infection susceptibility or their relationship with either the commensal or the disease associated microbiota.

Further, when the array comprises at least the probes (oligonucleotides) for detecting all mutations listed in Table 1, an individual imprinting of the individual is obtained with enough information to distinct among two individuals. Costs are avoided in relation with a complete genome analysis, and easy manipulation of data is provided with the array and methods of the invention.

### REFERENCES CITED IN THE APPLICATION

- Kathrani et al., in "Polymorphisms in the TLr4 and Tlr5 Gene Are Significantly Associated with Inflammatory Bowel Disease in German Shepherd Dogs", PLoS ONE - 2010, Vol. No. 5 (12), pp.: 1-10.
- Kathrani et al., "Breed-independent toll-like receptor 5 polymorphisms show association with canine inflammatory bowel disease", Tissue Antigens - 2011, Vol. No. 78, pp.: 94-101
- Kumar P et al., "Predicting the effects of coding non-synonymous variants on protein function using the SIFT algorithm", Nat Protoc.-2009; Vol. No.4(7), pp.:1073-81
- Choi Y et al., "Predicting the Functional Effect of Amino Acid Substitutions and Indels", PLoS ONE -2012, Vol. No. 7(10): e46688.
- Adzhubei I a, Schmidt S, Peshkin L, Ramensky VE, Gerasimova A, Bork P, Kondrashov AS, Sunyaev SR: A method and server for predicting damaging missense mutations. Nat Methods 2010, 7:248-9.
- Letunic I, Doerks T, Bork P: SMART 7: recent updates to the protein domain annotation resource. Nucleic Acids Res 2012, 40(Database issue):D302-5

## Claims

1. A DNA array comprising:
(i) a canine toll-like receptor 2 (*tlr 2*) gene fragment that in the entire nucleotide sequence of the gene codifies for a change of a serine (S) by a leucine (L) at position 516 of canine TLR2 polypeptide of SEQ ID NO: 1;
(ii) a canine toll-like receptor 5 (*tlr* 5) gene fragment that in the entire nucleotide sequence of the gene codifies for a change of a phenylalanine (F) by a cysteine (C) at position 888 of canine TLR5 polypeptide of SEQ ID NO: 2;
(iii) a canine toll-like receptor (*tlr 6*) gene fragment that in the entire nucleotide sequence of the gene codifies for a change of a leucine (L) by a phenylalanine (F) at position 457 of canine TLR6 polypeptide of SEQ ID NO: 3; and
(iv) a canine toll-like receptor 8 (*tlr 8*) gene fragment that in the entire nucleotide sequence of the gene codifes for a change of a valine (V) by an alanine (A) at position 157 of canine TLR8 polypeptide of SEQ ID NO: 4.

2. The DNA array according to claim 1, wherein the canine *tlr* gene fragments of i) to iv) comprise, respectively, the nucleotide sequences SEQ ID NO: 11 to 13, wherein SEQ ID NO: 11 in the entire nucleotide sequence of *tlr 2* gene codifies for the mutation S516L in SEQ ID NO:1; SEQ ID NO: 12 in the entire nucleotide sequence of *tlr* 6 gene codifies for the mutation L457 in SEQ ID NO: 3; SEQ I D NO: 13 in the entire nucleotide sequence of *tlr 8* gene codifies for the mutation V157A in SEQ ID NO: 4; and SEQ ID NO: 47 in the entire nucleotide sequence of *tlr 5* gene codifies for the mutation F888C in SEQ ID NO: 2.

3. The DNA array according to any of claims 1-2, comprising one or more canine *tlr* gene fragment selected from the group consisting of:
v) A *tlrl* gene fragment that in the entire nucleotide sequence of the gene codifies for a change of a serine (S) by an alanine (A) at position 281, and/or for a change of a valine (V) by an isoleucine (I) at position 312 of TLR1 polypeptide of SEQ ID NO: 5;
(vi) A *tlr2* gene fragment that in the entire nucleotide sequence of the gene codifies for a change of a threonine (T) by a methionine (M) at position 606 of canine TLR2 polypeptide of SEQ ID NO: 1
(vii) A *trl* 3 gene fragment that in the entire nucleotide sequence of the gene codifies for a change of a glutamic (E) by a glutamic (D) at position 176 of canine TLR3 polypeptide of SEQ ID NO: 6;
(viii) A *tlr 4* gene fragment that in the entire nucleotide sequence of the gene codifies for a change of a valine (V) by a methionine (M) at position 82, and/or a change of an alanine (A) by a threonine (T) at position 347, and/or a change of a threonine (T) by an alanine (A) at position 577 of canine TLR4 polypeptide of SEQ ID NO: 7;
(ix) A *tlr 5* gene fragment that in the entire nucleotide sequence of the gene codifies for a change of a change of a glutamic (E) by an aspartic (D) at position 169, and/or a change of a serine (S) by an arginine (N) at position 177, and/or a change of a valine (V) by a leucine (L) at position 296, and/or a change of a leucine (L) by a serine (S) at position 383, and/or a change of an arginine (R) by a glutamine (Q) at position 402, and/or a change of an arginine (R) by a glutamine (Q) at position 416, and/or a change of a valine (V) by an isoleucine (I) at position 296, and/or a change of a glycine (G) by a serine (S) at position 533, and/or a change of an arginine (R) by a glutamine (Q) at position 734, and/or a change of a glutamic (D) by a tyrosine (Y) 767, and/or a change of a asparagine by a lysine (K) at position 833, and/or a change of an arginine (R) by a cysteine (C) at position 844, and/or the presence of a leucine (L) instead of a serine (S) at position 850, and/or a change of an alanine by a threonine (T) at position 896, and/or a change of an hystidine (H) by a tyrosine (Y) at position 1017, and/or a change of a glycine (G) by a serine (S) at position 1020, and/or a change of an arginine (R) by a glutamine (Q) at position 1049, and/or a change of an alanine (A) by a threonine (T) at position 1078 of canine TLR5 polypeptide of SEQ ID NO: 2);
(x) A *tlr 6* gene fragment that in the entire nucleotide sequence of the gene codifies for a change of a tyrosine (Y) by a cysteine (C) at position 182 of canine TLR6 polypeptide of SEQ ID NO: 3;
(xi) A *tlr* 10 gene fragment that in the entire nucleotide sequence of the gene codifies for a change of a methionine (M) by a valine (V) at position 592 of canine TLR10 polypeptide of SEQ ID NO: 8;
(xii) A *tlr* 7 gene fragment that in the entire nucleotide sequence of the gene codifies for a change of an alanine by a glycine at position 16, and/or a change of a phenylalanine (F) by a leucine (L) at position 167, and/or a change of a proline (P) by a leucine (L) at position 1066 of canine TLR7 polypeptide of SEQ ID NO: 9;
(xiii) A *tlr 8* gene fragment that in the entire nucleotide sequence of the gene codifies for a change of an arginine (R) by glutamine (Q) at position 298 of canine TLR8 polypeptide of SEQ ID NO: 4;
(xiv) A *tlr 9* gene fragment that in the entire nucleotide sequence of the gene codifies for a change of a valine (V) by an isoleucine (I) at position 87, and/or a chang eof a lysine (K) by a glutamic (E) at position 381, and/or a change of a proline (P) by a threonine (T) at position 459, and/or a proline (P) by a leucine (L) at position 787, and/or an arginine (R) by a tryptophan (W) at position 862 of canine TLR 9 polypeptide of SEQ ID NO: 10, and combinations of said gene fragments.

4. The DNA array according to claim 3, wherein the *tlr* 1 gene fragment of (v) is selected from a group consisting of a sequence comprising SEQ ID NO: 14, a sequence comprising SEQ ID NO: 15, and combinations thereof; the *tlr2* gene fragment of (vi) is a sequence comprising SEQ ID NO: 16; the *tlr 3* gene fragment of (vii) is a sequence comprising SEQ ID NO: 17; the *tlr4* gene fragment of (viii) is selected from a group consisting of a sequence comprising SEQ ID NO: 18, a sequence comprising SEQ ID NO: 19, a sequence comprising SEQ ID NO: 20, and combinations thereof; the *tlr 5* gene fragment of (ix) is selected from a group consisting of a sequence comprising any of sequences SEQ ID NO: 21-35 and SEQ ID NO: 52, and combinations thereof; the *tlr 6* gene fragment of (x) is a sequence comprising SEQ ID NO: 36; the *tlr 10* gene fragment of (xi) is a sequence comprising SEQ ID NO: 46; the *tlr 7* gene fragment of (xii) is selected from a group consisting of a a sequence comprising any of sequences SEQ ID NO: 37-39, and combinations thereof; the *tlr 8* gene fragment of (xiii) is a sequence comprising SEQ ID NO: 40; and the *tlr 9* gene fragment of (xiv) is selected from a group consisting of a sequence comprising any of sequences 41-45, and combinations thereof.

5. The DNA array according to any of claims 1-4, comprising one or more canine *tlr* gene fragments, each gene fragment comprising one or more single nucleotide polymorphisms selected from the group consisting of :
rs23585044, rs23572381, rs23572380, rs22410121, rs8958543, rs22120766, rs22157966, rs22145736, rs22189454, rs22189456, rs22124023,
rs22123995, rs24029590, rs9070448, rs9070450, rs9070451, rs9070452, rs9070447, rs9125247, rs24029975, rs23570247, rs24607342, rs24607358, rs9188882, rs22882109, and rs23518574.

6. The DNA array according to any of claims 1-5, wherein the canine *tlr* gene fragments have a length comprised from 10 to 30 nucleotides.

7. The DNA array according to any of claims 1-6, further comprising primers for amplifying *tlr* gene fragments selected and included in the array.

8. A method for the identification of mutations in canine toll-like receptor genes, the method comprising:
- adding an isolated animal sample comprising DNA or cDNA to a DNA array as defined in any of claims 1-7; and
- determining if the canine toll-like receptor gene fragments hybridize with sample gene fragments.

9. The method according to claim 8, wherein the isolated animal sample is selected from the group consisting of blood, saliva, faeces, and urine.

10. An in vitro method for the analysis of the genetic predisposition of an individual of *Canis* genera to suffer from any disease related with dysfunctions of the innate immunity system, the method comprising carrying out the method as defined in any of claims 8-9, and correlating any detected mutation with the disease.

11. The in vitro method according to claim 10, wherein the disease related with dysfunctions of innate immunity system is selected from the group consisting of chronic enteropathies, osteoarthritis, endometrium infections, sino-nasal aspergillosis, idiopathic lymphoplasmacytic rhinitis, Leishmania infections atopic dermatitis, Inflammatory bowel syndrome, Inflammatory bowel disease, Chron's disease, viral infections and bacterial infections.

12. An in vitro method for the analysis of the canine innate immunity profile, the method comprising determining the presence or absence of canine toll-like receptor mutations by means of the DNA array as defined in any of claims 1-7.

13. An in vitro method for the individual identification of a *Canis* genera animal, comprising determining the presence or absence of canine toll-like receptor mutations in an isolated sample by means of the DNA array as defined in any of claims 1-7; and establishing a combination of detected and non-detected mutations in said sample.

14. The in vitro method according to claim 13, wherein the isolated animal sample is selected from the group consisting of blood, saliva, faeces, urine, skin, and hair.

15. An in vitro method for the analysis of variability in canine *tlr* genes, the method comprising determining the presence or absence of canine toll-like receptor mutations by means of the DNA array as defined in any of claims 1-7.


SEQUENCE LISTING

<110>  UNIVERSITAT AUTÒNOMA DE BARCELONA

<120>  DNA array for detecting canine toll-like receptor gene mutations

<130>  T-2013/039

<160>  128

<170>  PatentIn version 3.5

<210>  1
<211>  785
<212>  PRT
<213>  Canis lupus

<400>  1

Met Ser Arg Val Leu Trp Thr Leu Trp Val Leu Gly Ala Val Thr Asn
1               5                   10                  15

Leu Ser Lys Glu Glu Ala Pro Asp Gln Ser Ser Ser Leu Ser Cys Asp
            20                  25                  30

Pro Thr Gly Val Cys Asp Gly Arg Ser Arg Ser Leu Asn Ser Met Pro
        35                  40                  45

Ser Gly Leu Thr Ala Ala Val Arg Ser Leu Asp Leu Ser Asn Asn Glu
    50                  55                  60

Ile Thr Tyr Ile Gly Asn Ser Asp Leu Arg Asp Cys Val Asn Leu Lys
65                  70                  75                  80

Ala Leu Arg Leu Glu Ser Asn Gly Ile Asn Thr Ile Glu Glu Glu Ser
                85                  90                  95

Phe Phe Ser Leu Trp Ser Leu Glu His Leu Asp Leu Ser Tyr Asn Leu
            100                 105                 110

Leu Ser Asn Leu Ser Ser Ser Trp Phe Arg Pro Leu Ser Ser Leu Lys
        115                 120                 125

Phe Leu Asn Leu Leu Gly Asn Pro Tyr Lys Ser Leu Gly Glu Thr Pro
    130                 135                 140

Leu Phe Ser Gln Leu Thr Asn Leu Arg Ile Leu Lys Val Gly Asn Ile
145                 150                 155                 160

Tyr Ser Phe Thr Glu Ile Gln Asp Lys Asp Phe Ala Gly Leu Thr Phe
                165                 170                 175

Leu Glu Glu Leu Glu Ile Asp Ala Ser Asn Leu Gln Arg Tyr Glu Pro
            180                 185                 190

Lys Ser Leu Lys Ser Ile Gln Ser Ile Ser Tyr Leu Ala Leu Arg Met
        195                 200                 205

Lys Gln Pro Val Leu Leu Val Glu Ile Phe Val Asp Leu Ser Ser Ser
    210                 215                 220
Leu Lys His Leu Glu Leu Arg Asp Thr His Leu Asp Thr Phe His Phe
225                 230                 235                 240
Ser Glu Ala Ser Ile Asn Glu Thr His Thr Leu Val Lys Lys Trp Thr
                245                 250                 255
Phe Arg Asn Val Lys Val Thr Asp Arg Ser Phe Thr Glu Val Val Arg
            260                 265                 270
Leu Leu Asn Tyr Val Ser Gly Val Leu Glu Val Glu Phe Glu Asp Cys
        275                 280                 285
Thr Leu Tyr Gly Leu Gly Asp Phe Asp Ile Pro Asp Val Asp Lys Ile
    290                 295                 300
Lys Asn Ile Gly Gln Ile Glu Thr Leu Thr Val Arg Arg Leu His Ile
305                 310                 315                 320
Pro His Phe Tyr Ser Phe Tyr Asp Met Ser Ser Ile Tyr Ser Leu Thr
                325                 330                 335
Glu Asp Val Lys Arg Ile Thr Val Glu Ser Ser Lys Val Phe Leu Val
            340                 345                 350
Pro Cys Ser Leu Ser Gln His Leu Lys Ser Leu Glu Tyr Leu Asp Leu
        355                 360                 365
Ser Asp Asn Leu Val Val Glu Glu Tyr Leu Arg Asn Ser Ala Cys Gln
    370                 375                 380
His Ala Trp Pro Leu Leu Gln Thr Leu Ile Leu Arg Gln Asn Arg Leu
385                 390                 395                 400
Lys Ser Leu Glu Lys Thr Gly Glu Thr Leu Leu Thr Leu Lys Asn Leu
                405                 410                 415
Val Asn Leu Asp Ile Ser Lys Asn Asn Tyr Leu Ser Met Pro Glu Thr
            420                 425                 430
Cys Gln Trp Pro Glu Lys Leu Lys Cys Leu Asn Leu Ser Asp Thr Arg
        435                 440                 445
Met Gln Ser Ile Thr Arg Cys Ile Pro Gln Thr Leu Glu Ile Leu Asp
    450                 455                 460
Val Ser Asn Asn Asn Leu Glu Ser Phe Ser Leu Ile Leu Pro Gln Leu
465                 470                 475                 480

Lys Glu Leu Ser Ile Ser Arg Asn Lys Leu Lys Thr Leu Pro Asp Ala
                485                 490                 495

Ser Phe Leu Pro Thr Leu Gln Ile Met Arg Ile Ser Arg Asn Thr Ile
            500                 505                 510

Asn Ala Phe Ser Lys Glu Gln Leu Asp Ser Phe His Arg Leu Gln Thr
        515                 520                 525

Leu Glu Ala Gly Gly Asn Asn Phe Leu Cys Ser Cys Glu Phe Leu Ser
    530                 535                 540

Phe Thr Gln Glu Gln Gln Ala Leu Ala Gly Leu Leu Val Gly Trp Pro
545                 550                 555                 560

Glu Asp Tyr Leu Cys His Ser Pro Ser Tyr Val Arg Gly Gln Arg Val
                565                 570                 575

Gly Thr Ala Arg Leu Pro Ala Ser Glu Cys His Arg Thr Ala Leu Val
            580                 585                 590

Ala Ala Val Cys Cys Val Leu Leu Leu Leu Val Leu Leu Thr Ala Gly
        595                 600                 605

Ala Cys His His Phe His Gly Leu Trp Tyr Leu Arg Met Leu Trp Ala
    610                 615                 620

Trp Leu Gln Ala Lys Arg Lys Pro Arg Lys Ala Pro Ser Arg Asp Val
625                 630                 635                 640

Cys Tyr Asp Ala Phe Val Ser Tyr Ser Glu His Asp Ser Tyr Trp Val
                645                 650                 655

Glu Asn Leu Leu Val Gln Lys Leu Glu His Phe Asn Pro Pro Phe Lys
            660                 665                 670

Leu Cys Leu His Lys Arg Asp Phe Ile Pro Gly Lys Trp Ile Ile Asp
        675                 680                 685

Asn Ile Ile Asp Ser Ile Glu Lys Ser His Lys Thr Ile Phe Val Leu
    690                 695                 700

Ser Glu Asn Phe Val Lys Ser Glu Trp Cys Lys Tyr Glu Leu Asp Phe
705                 710                 715                 720

Ser His Phe Arg Leu Phe Asp Glu Asn Asn Asp Ala Ala Ile Leu Ile
                725                 730                 735

Leu Leu Glu Pro Ile Glu Lys Lys Ala Ile Pro Gln Arg Phe Cys Lys
            740                 745                 750

Leu Arg Lys Ile Met Asn Thr Lys Thr Tyr Leu Glu Trp Pro Thr Asp
        755                 760                 765

Asp Ala Gln Gln Glu Gly Phe Trp Leu Asn Leu Arg Thr Ala Ile Lys
    770                 775                 780

Ser
785

<210>  2
<211>  1422
<212>  PRT
<213>  Canis lupus

<400>  2

Met Gly Gly Glu Arg Ala Cys Arg Gly Ser Ser Thr Cys Gln Val Pro
1               5                   10                  15

Ala Leu Glu Val Arg Gly Glu Ala Ala Gly Ala Pro Val Leu His Pro
            20                  25                  30

Pro Pro Pro Arg Trp Ala Ser Phe Leu Cys Ile Pro Gly Ala Pro Gly
        35                  40                  45

Cys His Arg Val Ile Gly Gly Leu Pro Gly His Ser Gly Arg Cys Arg
    50                  55                  60

Leu Pro Gln Asp Val Arg Pro Ser Trp Pro Val Cys Pro Ala Pro Ala
65                  70                  75                  80

Ala Pro Cys Ala Gln Ser Gly Arg Val Ala Leu Val Gln Met Thr Gly
                85                  90                  95

Ala Leu Ser Arg Ala Ala Leu Ala Pro Thr Gly Leu Gly Arg Gly Trp
            100                 105                 110

Glu Gly Asp Ala Leu Thr Arg Gln Ala Gly Arg Phe Ala Asp Gly Gly
        115                 120                 125

Pro Lys Asp Gln Arg Val Arg Ala Asp Pro Gly Arg Val Trp Ser Ala
    130                 135                 140

Gln Gly Ala Glu Gly Arg Ala Pro Gly Thr Gly Arg Gly Arg His Gln
145                 150                 155                 160

Tyr Pro Pro Pro Ala Ile Phe Pro Glu Ala Leu His Ala Ser Arg Leu
                165                 170                 175

Ser Asp Gly Lys Gln Thr Leu Leu Lys Val Ser Val Ser Gln Gly Pro
            180                 185                 190

Ser Thr Ala Leu Ser Ala Arg Pro Ala Gly Arg Thr Cys Gly Gly Arg
        195                 200                 205

Gln Val Pro Val Gln Gly Pro Pro Ser Trp Ala Pro Gly Trp Arg Trp
    210                 215                 220

Ala Cys Thr Ala Val Phe Pro Ser Arg Arg Ile Met Gly Arg Gln Leu
225                 230                 235                 240

Gly Arg Thr Leu Gly Leu Leu Leu Val Ala Gly Ala Val Ala Ala Ala
                245                 250                 255

Ser Cys Cys Val Ala Asp Gly Arg Arg Ala Leu Tyr Arg Ser Cys Asn
            260                 265                 270

Leu Ser Gln Val Pro Pro Val Pro Ser Thr Thr Glu Ile Leu Leu Leu
        275                 280                 285

Ser Phe Asn Tyr Ile Arg Ala Val Thr Arg Ala Ser Phe Pro Leu Leu
    290                 295                 300

Glu Arg Leu Gln Leu Leu Glu Leu Gly Thr Gln Gln Thr Pro Phe Ser
305                 310                 315                 320

Val Asp Arg Glu Ala Phe Arg Asn Leu Pro Asn Leu Arg Thr Leu Asp
                325                 330                 335

Leu Gly Asn Ser Arg Val Asp Phe Leu His Pro Asp Ala Phe Gln Gly
            340                 345                 350

Leu Pro His Leu Gln Glu Leu Arg Leu Phe Ala Cys Gly Leu Ser Asp
        355                 360                 365

Val Val Leu Thr Asp Gly Tyr Phe Arg Asn Leu Gly Ala Leu Leu Arg
    370                 375                 380

Leu Asp Leu Ser Lys Asn Gln Ile Gly Ser Leu Glu Leu His Ala Ser
385                 390                 395                 400

Phe Arg Glu Leu Gly Ser Leu Arg Ser Val Asp Phe Ser Leu Asn Arg
                405                 410                 415

Ile Pro Ala Ala Cys Glu Gln Gly Leu Arg Pro Leu Gln Gly Lys Ala
            420                 425                 430

Leu Ser Leu Leu Asn Leu Ala Ala Asn Gly Leu Tyr Ser Arg Ala Pro
        435                 440                 445

Val Asp Trp Gly Arg Cys Gly Asn Pro Phe Arg Asn Val Val Leu Glu
    450                 455                 460

Thr Leu Asp Val Ser Asn Asn Gly Trp Thr Ala Asp Val Thr Gly Asn
465                 470                 475                 480

Val Thr Arg Ala Ile Gly Gly Ser Gln Ile Ser Ser Leu Val Leu Ala
                485                 490                 495

His His Ile Met Gly Gln Gly Phe Gly Phe Arg Asn Ile Arg Asp Pro
            500                 505                 510

Asp Arg Ser Thr Phe Ala Gly Leu Ala Gly Ser Ser Val Leu Arg Leu
        515                 520                 525

Asp Leu Ser His Gly Phe Val Phe Ser Leu Asn Ala Arg Leu Phe Glu
    530                 535                 540

Val Leu Gly Asp Leu Lys Leu Leu Asp Leu Ala His Asn Lys Ile Asn
545                 550                 555                 560

Arg Ile Ala Gly Glu Ala Phe His Gly Leu Gly Ser Val Gln Val Leu
                565                 570                 575

Asn Leu Ser His Asn Leu Leu Gly Glu Leu Tyr Asp Ser Asp Phe Ser
            580                 585                 590

Gly Leu Ala Glu Val Ala Tyr Ile Asp Leu Gln His Asn His Ile Gly
        595                 600                 605

Ile Ile Gln Asp Gln Thr Phe Arg Phe Leu Gly Ala Leu Arg Thr Leu
    610                 615                 620

Asp Leu Arg Asp Asn Ala Leu Lys Thr Val Ser Phe Val Pro Ser Ile
625                 630                 635                 640

Asp Thr Ile Phe Leu Gly Asn Asn Lys Leu Glu Thr Val Ser His Met
                645                 650                 655

Asp Leu Thr Ala Ser Phe Leu Glu Leu Ser Asp Asn Arg Leu Glu Asp
            660                 665                 670

Leu Gly Asp Leu Tyr Ser Leu Leu Arg Val Pro Ala Leu Gln Val Leu
        675                 680                 685

Ile Leu Asn Arg Asn Arg Leu Ser Ala Cys Arg Gly Gly His Gly Pro
    690                 695                 700

Thr Gly Ser Val Gly Pro Glu Arg Leu Phe Leu Gly Ser Asn Met Leu
705                 710                 715                 720

Gln Leu Ala Trp Glu Thr Gly Arg Cys Trp Asp Val Phe Arg Gly Leu
                725                 730                 735

Pro Arg Leu Arg Val Leu His Leu Asn His Asn Tyr Leu Ala Ala Leu
            740                 745                 750

Pro Pro Gly Leu Leu Arg Asp Leu Thr Ala Leu Arg Gly Leu Asp Leu
        755                 760                 765

Ser Ala Asn Arg Leu Ser Thr Leu Ser Arg Gly Asp Leu Pro Ala Ala
    770                 775                 780

Leu Glu Val Leu Asp Val Ser Arg Asn Gln Leu Leu Ser Leu Asp Pro
785                 790                 795                 800

Gly Leu Leu Ala Pro Leu Arg Ala Val Asp Leu Thr His Asn Lys Phe
                805                 810                 815

Ile Cys Gly Cys Glu Leu Arg Pro Leu Val Arg Trp Leu Asn Arg Thr
            820                 825                 830

Asn Val Thr Val Phe Gly Ser Arg Ala Asp Val Arg Cys Ala Tyr Pro
        835                 840                 845

Ser Leu Leu Ala Gly Thr Pro Leu Ser Ser Val Ser Met Glu Gly Cys
    850                 855                 860

Asp Asp Glu Glu Ala Leu Arg Thr Leu Thr Phe Ser Leu Phe Ile Phe
865                 870                 875                 880

Ser Thr Val Gly Val Thr Leu Phe Leu Leu Ala Val Leu Val Ala Ala
                885                 890                 895

Lys Leu Arg Gly Leu Cys Phe Leu Cys Tyr Lys Ala Ala Arg Arg Leu
            900                 905                 910

Leu Pro Ala Gly Pro Ala Glu Asp Gly Ala Pro Asp Ala Tyr Gln Tyr
        915                 920                 925

Asp Ala Tyr Leu Cys Phe Ser Gly Arg Asp Phe Glu Trp Val Gln Arg
    930                 935                 940

Ala Leu Leu Arg His Leu Asp Ala Gln Tyr Ser Ser Arg Asn Arg Leu
945                 950                 955                 960

Asn Leu Cys Phe Glu Glu Arg Asp Phe Val Pro Gly Arg Glu His Ile
                965                 970                 975

Ala Asn Ile Gln Asp Ala Val Trp Ser Ser Arg Lys Val Val Cys Leu
            980                 985                 990

Val Ser Arg His Phe Leu Arg Asp  Gly Trp Cys Leu Glu  Ala Phe Ala
        995                 1000                1005

Ala Ala  Arg Ser Arg Cys Ala  Ser His Leu Asp Gly  Ala Leu Val
    1010                1015                1020

Leu Val  Val Val Gly Ser Leu  Ser Gln Tyr Gln Leu  Arg Arg His

1025                1030                1035

Pro Ala Ile Gly Gly Phe Val Arg Gln Arg Arg Tyr Leu Arg Trp
1040                1045                1050

Pro Glu Asp Leu Gln Asp Val Gly Trp Phe Leu Asp Thr Leu Ser
1055                1060                1065

Arg His Ile Leu Gln Glu Gln Arg Gly Ala Arg Gly Asp Gly Gly
1070                1075                1080

Ile Pro Leu Arg Thr Val Ala Ala Gly Arg Arg Pro His Cys Thr
1085                1090                1095

Arg Val Gly Arg Arg Arg Pro His Cys Thr Arg Val Arg Gly Arg
1100                1105                1110

Arg Pro His Cys Thr Gly Ala Pro Gly Gly Ala Asp Leu Thr Ala
1115                1120                1125

Pro Gly Ser Gly Gly Arg Arg Pro His Cys Thr Gly Val Arg Gly
1130                1135                1140

Thr Pro Thr Ser Leu His Arg Gly Pro Gly Gly Arg Arg Pro His
1145                1150                1155

Cys Thr Gly Ala Pro Gly Gly Ala Asp Leu Thr Ala Pro Gly Ser
1160                1165                1170

Gly Gly Ala Asp Leu Ser Ala Pro Gly Thr Gly Gly Ala Asp Leu
1175                1180                1185

Thr Ala Pro Gly Pro Arg Gly Arg Cys Ser Ser Ala Ala Gly Ala
1190                1195                1200

Arg Val Arg Gln Arg Ala Arg Ala Arg Ala Val Arg Arg Arg Ser
1205                1210                1215

Ser Gly Ser Ala Arg Arg Thr Arg Glu His Thr Arg Pro Glu Glu
1220                1225                1230

Pro Pro Arg Ala Arg Pro Ala Leu Gly Arg Ala Pro Ala Ala Ala
1235                1240                1245

Pro Ala Pro Arg Ala Pro Ala Arg Pro Arg Arg Arg Pro Pro Arg
1250                1255                1260

Leu Pro Arg Pro Cys Pro Pro Ser Ala Arg Ala Gly Cys Pro Ala
1265                1270                1275

Pro Arg Pro Tyr Pro Val Ser Arg Pro Arg Gly Ala Gly Gly Gly
1280                1285                1290

Gly Gly Gly Gly Gly Gly Gly Gly Ala Gly Thr Arg Pro Arg Arg
    1295                1300                1305

Arg Gln Ala Pro Pro Thr Arg Arg Pro Arg Cys Gly Pro Gly Ala
    1310                1315                1320

Gly Phe Arg Leu Pro Pro Glu Gly Val Pro Ser Arg Thr Gly Ser
    1325                1330                1335

Pro Ala Pro Ala Ala Glu Gly Gly Gly Arg Gly Arg Ser Cys Gln
    1340                1345                1350

Gln Phe Gly Gly Ala Gly Gly His Thr Asp Val Arg Gly Leu Pro
    1355                1360                1365

Pro Ala Ser Gly Gly Pro Arg Val Ser Pro Ser Ala Arg Ser Ser
    1370                1375                1380

Pro Asp Arg Arg Val Ala Ala Gly Glu Gly Ala Gly Arg Gly Gly
    1385                1390                1395

Thr Gln Glu Gly Arg Gly Val Gly Glu Ala Gly Ala Gly Arg Glu
    1400                1405                1410

Arg Gly Gly Ala Gly Arg Gly Val Gly
    1415                1420

<210>  3
<211>  780
<212>  PRT
<213>  Canis lupus

<400>  3

Met Ile Lys Asp Lys Asp Ser Ile Thr Gly Ser Phe His Phe Val Tyr
1               5                   10                  15

Ile Val Thr Leu Ile Val Gly Thr Ile Ile Gln Phe Ser Asp Glu Ser
            20                  25                  30

Glu Phe Thr Val Asp Met Ser Asn Met Asn Leu Thr His Val Pro Glu
        35                  40                  45

Asp Leu Pro Pro Lys Thr Lys Ile Leu Asp Met Ser Gln Asn Asn Ile
    50                  55                  60

Ser Glu Leu His Leu Ser Asp Met Ser Tyr Leu Ser Gly Leu Lys Ile
65                  70                  75                  80

Leu Arg Ile Ser His Asn Arg Ile Trp Trp Leu Asp Phe Ser Ile Phe
                85                  90                  95

Lys Phe Asn Gln Asp Leu Glu Tyr Leu Asp Leu Ser Tyr Asn Gln Leu
            100                 105                 110

Arg Asn Met Ser Cys His Leu Ile Arg Ser Leu Lys His Leu Asp Leu
        115                 120                 125

Ser Phe Asn Asp Phe His Val Leu Pro Ile Cys Lys Glu Phe Gly Asn
    130                 135                 140

Leu Thr Gln Leu Gln Phe Leu Gly Leu Ser Ala Thr Lys Leu Arg Gln
145                 150                 155                 160

Leu Asp Leu Leu Pro Ile Ala His Leu His Leu Ser Tyr Ile Leu Leu
                165                 170                 175

Asp Leu Gln Gly Tyr Tyr Ala Lys Glu Ser Glu Lys Gly Ser Leu Gln
            180                 185                 190

Ile Leu Asp Thr Lys Thr Leu His Leu Val Phe His Pro Asn Gln Leu
        195                 200                 205

Phe Ser Val Gln Ala Asn Met Leu Val Asn Asn Leu Gly Cys Leu Gln
    210                 215                 220

Leu Thr Asn Ile Lys Leu Asn Asn Asp Asn Cys Gln Val Leu Ile Gln
225                 230                 235                 240

Phe Leu Ser Glu Leu Thr Arg Gly Pro Thr Leu Leu Asn Phe Thr Leu
                245                 250                 255

Gln His Val Lys Thr Thr Trp Lys Cys Leu Val Arg Ile Phe Lys Phe
            260                 265                 270

Leu Trp Pro Lys Pro Val Gln Tyr Leu Asn Ile Tyr Asn Leu Thr Ile
        275                 280                 285

Val Glu Ser Ile Asn Lys Glu Tyr Ile His Tyr Pro Lys Thr Ala Leu
    290                 295                 300

Lys Ala Leu Thr Ile Glu His Val Lys Asn Glu Val Phe Leu Phe Ser
305                 310                 315                 320

Gln Thr Ala Leu Tyr Thr Ile Phe Ser Glu Met Asn Ile Met Met Leu
                325                 330                 335

Thr Ile Ser Asp Thr Pro Phe Ile His Met Leu Cys Pro Pro Pro Ser
            340                 345                 350

Asn Thr Phe Lys Phe Leu Asn Phe Thr Gln Asn Val Phe Thr Asp Ser
        355                 360                 365

Val Phe Gln Ser Cys Ser His Leu Val Arg Leu Glu Thr Leu Ile Leu

370                 375                 380

Arg Lys Asn Lys Leu Lys Asp Leu Tyr Lys Val Gly Leu Met Thr Lys
385                 390                 395                 400

His Met Thr Ser Leu Glu Ile Leu Asp Val Ser Val Asn Ser Leu Glu
                405                 410                 415

Tyr Asp Arg Tyr Asp Gly Asn Cys Thr Trp Val Gly Ser Ile Val Val
            420                 425                 430

Leu Asn Leu Ser Ser Asn Ile Leu Thr Asp Ser Val Phe Arg Cys Leu
        435                 440                 445

Pro Pro Lys Val Lys Val Leu Asp Leu His Asp Asn Arg Ile Arg Ser
    450                 455                 460

Ile Pro Lys Pro Ile Met Lys Leu Glu Asp Leu Gln Glu Leu Asn Val
465                 470                 475                 480

Ala Ser Asn Ser Leu Ala His Phe Pro Asp Cys Gly Thr Phe Asn Arg
                485                 490                 495

Leu Ser Val Leu Ile Ile Asp Ser Asn Ser Ile Ser Asn Pro Ser Ala
            500                 505                 510

Asp Phe Leu Gln Ser Cys His Asn Ile Arg Ser Met Ser Ala Gly Asn
        515                 520                 525

Asn Pro Phe Gln Cys Thr Cys Glu Leu Arg Glu Phe Val Gln Ser Leu
    530                 535                 540

Gly Gln Val Ala Ser Lys Val Val Glu Gly Trp Pro Asp Ser Tyr Lys
545                 550                 555                 560

Cys Asp Ser Pro Glu Asn Tyr Lys Gly Thr Leu Leu Lys Asp Phe His
                565                 570                 575

Val Ser Pro Leu Ser Cys Asn Thr Thr Leu Leu Leu Val Thr Ile Gly
            580                 585                 590

Val Ala Val Leu Val Phe Thr Val Thr Val Thr Ala Leu Cys Ile Tyr
        595                 600                 605

Phe Asp Leu Pro Trp Tyr Leu Arg Met Val Phe Gln Trp Thr Gln Thr
    610                 615                 620

Arg Arg Arg Ala Arg Asn Thr Pro Leu Glu Glu Leu Gln Arg Thr Ile
625                 630                 635                 640

Gln Phe His Ala Phe Ile Ser Tyr Ser Glu His Asp Ser Ala Trp Val
                645                 650                 655

Lys Asn Glu Leu Val Pro Cys Leu Glu Lys Glu Glu Leu Arg Ile Cys
            660                 665                 670

Leu His Glu Arg Asn Phe Ile Pro Gly Lys Ser Ile Val Glu Asn Ile
        675                 680                 685

Ile Asn Cys Ile Glu Lys Ser Tyr Lys Ser Ile Phe Val Leu Ser Pro
    690                 695                 700

Asn Phe Val Gln Ser Glu Trp Cys His Tyr Glu Leu Tyr Phe Ala His
705                 710                 715                 720

His Asn Leu Phe His Glu Gly Ser Asn Asn Leu Ile Leu Ile Leu Leu
                725                 730                 735

Glu Pro Ile Pro Gln Asn Cys Ile Pro Ser Lys Tyr His Lys Leu Arg
            740                 745                 750

Ala Leu Met Thr Gln Arg Thr Tyr Leu Glu Trp Pro Lys Glu Lys Ser
        755                 760                 765

Lys His Gly Leu Phe Trp Ala Asn Ile Arg Ala Ala
    770                 775                 780

<210>  4
<211>  1038
<212>  PRT
<213>  Canis lupus

<400>  4

Met Ser Pro Arg Ser Leu Val Leu Thr Cys Leu Phe Leu Leu Ile Ser
1               5                   10                  15

Asp Ser Tyr Glu Phe Val Thr Lys Ala Asn Tyr Ser Arg Ser Tyr Pro
            20                  25                  30

Cys Asp Glu Arg Arg Gln Asn Gly Ser Val Ile Ala Glu Cys Asn Gly
        35                  40                  45

Arg Arg Leu Gln Glu Val Pro Gln Thr Val Gly Lys Tyr Val Thr Val
    50                  55                  60

Leu Asp Leu Ser Asp Asn Tyr Ile Thr His Ile Thr Asn Glu Ser Phe
65                  70                  75                  80

His Gly Leu Gln Asn Leu Thr Lys Ile Asn Leu Asn His Asn Ala Asn
                85                  90                  95

Pro Gln His Leu Ser Glu Asn Pro Asp Asn Lys Asn Gly Met Asn Ile
            100                 105                 110

Thr Asp Gly Ala Phe Leu Asn Leu Gln Asn Leu Asn Gln Leu Leu Leu
        115                 120                 125

Glu Asp Asn Gln Leu Tyr Gln Ile Pro Ala Gly Leu Pro Gly Ser Leu
    130                 135                 140

Lys Glu Leu Ser Leu Ile Gln Asn Asn Ile Ile Trp Val Thr Lys Lys
145                 150                 155                 160

Asn Thr Ser Gly Leu Thr Asn Leu Glu Arg Leu Tyr Leu Ser Trp Asn
                165                 170                 175

Cys Tyr Phe Gly Asn Asn Cys Asn Asn Lys Thr Phe Asn Ile Glu Asp
            180                 185                 190

Gly Thr Phe Glu Ser Leu Thr Asn Leu Glu Val Leu Ser Leu Ser Phe
        195                 200                 205

Asn Lys Leu Val His Val Pro Pro Lys Leu Pro Ser Ser Leu Lys Glu
    210                 215                 220

Leu Tyr Leu Ser Asn Ala Lys Ile Lys Ile Ile Ser Gln Glu Asp Phe
225                 230                 235                 240

Lys Gly Leu Arg Asn Leu Arg Val Leu Asp Leu Ser Gly Asn Cys Pro
                245                 250                 255

Arg Cys Phe Asn Ala Pro Phe Pro Cys Thr Pro Cys Glu Gly Gly Ala
            260                 265                 270

Ser Ile Gln Ile His Pro Leu Ala Phe Gln Thr Leu Thr Glu Leu Arg
        275                 280                 285

Tyr Leu Asn Leu Ser Ser Thr Ser Leu Arg Lys Ile Pro Ala Thr Trp
    290                 295                 300

Phe Asp Asn Met Arg Asn Leu Lys Val Leu His Leu Glu Phe Asn Tyr
305                 310                 315                 320

Leu Val Asp Glu Ile Ala Ser Gly Glu Phe Leu Thr Lys Leu Pro Val
                325                 330                 335

Leu Glu Ile Leu Asp Leu Ser Tyr Asn Tyr Val Lys Ala Lys Tyr Pro
            340                 345                 350

Lys Tyr Ile Asn Ile Ser His Asn Phe Ser Ser Leu Lys Leu Leu Gln
        355                 360                 365

Ala Leu His Leu Arg Gly Tyr Val Phe Gln Glu Leu Arg Ala Gly Asp
    370                 375                 380

Phe Glu Pro Leu Met Gly Leu Ser Asn Leu Lys Thr Ile Asn Leu Gly

385                 390                 395                 400

Val Asn Phe Ile Lys Gln Ile Asn Phe Thr Leu Phe Gln Asn Phe Pro
                405                 410                 415

Asn Leu Ser Ile Ile Tyr Leu Ser Glu Asn Arg Ile Ser Pro Leu Val
            420                 425                 430

Asn Asp Ile Arg Gln Asn Glu Val Asn Gly Ser Ser Ser Gln Arg His
        435                 440                 445

Val Leu Lys Pro Arg Ser Ala Asp Met Glu Phe Asp Pro His Ser Asn
    450                 455                 460

Phe Tyr His Asn Thr His Pro Leu Ile Lys Pro Gln Cys Thr Val Tyr
465                 470                 475                 480

Gly Lys Ala Leu Asp Leu Ser Leu Asn Ser Ile Phe Phe Ile Gly Arg
                485                 490                 495

Glu Gln Phe Glu Ala Phe His Asp Ile Ala Cys Leu Asn Leu Ser Ser
            500                 505                 510

Asn Gly Asn Gly Gln Val Leu His Gly Asn Glu Phe Ser Ala Val Pro
        515                 520                 525

His Ile Lys Tyr Leu Asp Leu Thr Asn Asn Arg Leu Asp Phe Asp Asp
    530                 535                 540

Asp Asn Ala Leu Ser Asp Leu Pro Glu Leu Glu Val Leu Asp Leu Ser
545                 550                 555                 560

Tyr Asn Ala His Tyr Phe Arg Ile Ala Gly Val Thr His Arg Leu Gly
                565                 570                 575

Phe Ile Gln Asn Leu Thr Gln Leu Lys Val Leu Asn Leu Ser His Asn
            580                 585                 590

Ser Ile Tyr Thr Leu Thr Glu Gln Asp Leu Arg Ser Val Ser Leu Glu
        595                 600                 605

Glu Leu Val Phe Ser Gly Asn Arg Leu Asp Ile Leu Trp Asn Ala Glu
    610                 615                 620

Gly Asp Lys Tyr Trp Lys Ile Phe Thr Arg Leu Arg Asn Leu Thr Arg
625                 630                 635                 640

Leu Asp Leu Ser Leu Asn Asn Leu Arg Arg Ile Pro Asn Glu Ala Phe
                645                 650                 655

Leu Asn Leu Pro Gln Ser Leu Thr Gln Leu Tyr Ile Lys Asn Asn Ala
            660                 665                 670

Leu Asn Phe Phe Asn Trp Thr Leu Leu Gln Glu Phe Pro Arg Leu Gln
        675                 680                 685

Val Leu Asp Leu Ser Gly Asn Arg Leu Ser Ser Ile Thr Asn Ser Leu
    690                 695                 700

Ser Lys Phe Thr Ser Ser Leu Gln Thr Leu Leu Leu His Arg Asn Arg
705                 710                 715                 720

Ile Ser His Leu Pro Ala Ser Phe Leu Ser Glu Ala Ser Ser Leu Ile
                725                 730                 735

His Leu Asp Leu Ser Ser Asn Leu Leu Lys Met Ile Asn Lys Ser Thr
            740                 745                 750

Leu Gln Thr Lys Thr Asn Thr Ser Leu Ala Ile Leu Glu Leu Gly Arg
        755                 760                 765

Asn Pro Phe Asp Cys Thr Cys Asp Ile Gly Asp Phe Arg Arg Trp Met
    770                 775                 780

Asp Glu Asn Leu Asn Val Thr Ile Pro Arg Leu Thr Asp Val Ile Cys
785                 790                 795                 800

Ser Ser Pro Gly Asp Gln Arg Gly Lys Ser Ile Val Ser Leu Glu Leu
                805                 810                 815

Thr Thr Cys Ile Ser Asp Thr Leu Ala Ala Val Leu Cys Ile Phe Thr
            820                 825                 830

Ser Phe Ile Thr Val Thr Val Met Leu Ala Ala Leu Gly His His Trp
        835                 840                 845

Phe Tyr Trp Asp Val Trp Phe Ile Tyr His Val Cys Leu Ala Lys Val
    850                 855                 860

Lys Gly Tyr Arg Ser Val Ser Thr Ser Gln Thr Phe Tyr Asp Ala Tyr
865                 870                 875                 880

Val Ser Tyr Asp Thr Lys Asp Ala Ser Val Thr Asp Trp Val Ile Asn
                885                 890                 895

Glu Leu Arg Phe His Leu Glu Glu Ser Glu Gly Lys Asn Val Leu Leu
            900                 905                 910

Cys Leu Glu Glu Arg Asp Trp Asp Pro Gly Leu Ala Ile Ile Asp Asn
        915                 920                 925

Leu Met Gln Ser Ile Asn Gln Ser Lys Lys Thr Ile Phe Val Leu Thr
    930                 935                 940

Lys Glu Tyr Ala Gln Asn Trp Asn Phe Lys Thr Ala Phe Tyr Leu Ala
945                 950                 955                 960

Leu Gln Arg Leu Met Asp Glu Asn Met Asp Val Ile Ile Phe Ile Leu
                965                 970                 975

Leu Glu Pro Val Leu Gln His Ser Gln Tyr Leu Arg Leu Arg Gln Arg
            980                 985                 990

Ile Cys Lys Ser Ser Ile Leu Gln  Trp Pro Asp Asn Pro  Lys Ala Glu
        995                 1000                 1005

Gly Leu  Phe Trp Gln Ser Leu  Lys Asn Val Val Leu  Thr Glu Asn
    1010                 1015                 1020

Asp Ser  Arg Tyr Asn Asn Leu  Tyr Val Asp Ser Ile  Lys Gln Tyr
    1025                 1030                 1035

<210>  5
<211>  790
<212>  PRT
<213>  Canis lupus

<400>  5

Met Met Lys Thr Asn Pro Ser Ile Phe Gln Phe Ala Ile Ile Phe Ile
1               5                   10                  15

Leu Ile Leu Glu Ile Arg Ile Gln Leu Ser Glu Glu Ser Asp Phe Leu
            20                  25                  30

Val Asn Arg Ser Lys Ala Gly Leu Phe His Ile Pro Lys Asp Leu Ser
        35                  40                  45

Leu Lys Thr Thr Ile Leu Asp Ile Ser Gln Asn Tyr Ile Ser Glu Leu
    50                  55                  60

Gln Thr Ser Asp Ile Leu Ser Leu Ser Lys Leu Arg Ile Leu Ile Val
65                  70                  75                  80

Ser Tyr Asn Arg Ile Gln Tyr Leu Asp Ile Ser Val Phe Lys Phe Asn
                85                  90                  95

Gln Glu Leu Glu Tyr Leu Asp Leu Ser His Asn Glu Leu Gly Arg Ile
            100                 105                 110

Ser Cys His Pro Thr Val Asn Leu Lys His Leu Asp Leu Ser Phe Asn
        115                 120                 125

Ala Phe Asp Asp Leu Pro Ile Cys Lys Glu Phe Gly Asn Met Ser Gln
    130                 135                 140

Leu Glu Phe Leu Gly Leu Ser Ala Thr Gln Leu Gln Lys Ser Ser Met

145                 150                 155                 160

Leu Pro Ile Ala Ser Leu His Ile Arg Lys Val Leu Leu Val Leu Gly
                165                 170                 175

Asp Thr Tyr Gly Lys Lys Glu Asp Pro Glu Ser Leu Gln Lys Leu Asn
            180                 185                 190

Thr Glu Ser Leu His Ile Val Phe Pro Ile Arg Lys Glu Phe Ser Phe
        195                 200                 205

Thr Leu Asp Val Ser Val Ser Thr Ala Val Ser Leu Glu Leu Ser Asn
    210                 215                 220

Ile Lys Cys Val Pro Asp Gly His Gly Trp Ser Tyr Phe Gln Asn Val
225                 230                 235                 240

Leu Ser Lys Leu Gln Lys Asn Ser Arg Leu Ser Ser Leu Thr Leu Asn
                245                 250                 255

Asn Ile Glu Thr Thr Trp Asn Phe Phe Ile Met Leu Leu Gln Leu Val
            260                 265                 270

Trp His Thr Ser Ile Glu Tyr Phe Ser Ile Ser Asn Val Lys Leu Gln
        275                 280                 285

Gly Tyr Pro Asp Phe Arg Asp Phe Asp Tyr Ser Asp Thr Ser Leu Lys
    290                 295                 300

Ala Leu Ser Ile His Gln Val Val Ser Asn Ala Phe Asn Leu Pro Gln
305                 310                 315                 320

Ser Tyr Ile Tyr Lys Ile Phe Ser Asn Met Asn Ile Gln Asn Phe Thr
                325                 330                 335

Val Ser Gly Thr His Met Val His Met Val Cys Pro Ser Gln Ile Ser
            340                 345                 350

Pro Phe Leu His Leu Asp Phe Ser Asn Asn Leu Leu Thr Asp Ile Val
        355                 360                 365

Phe Lys Asn Cys Arg Asn Leu Ile Lys Leu Glu Thr Leu Ser Leu Gln
    370                 375                 380

Met Asn Gln Leu Lys Glu Leu Ala Ser Ile Ala Gln Met Thr Asn Glu
385                 390                 395                 400

Met Lys Ser Leu Gln Gln Leu Asp Ile Ser Gln Asn Ser Leu Arg Tyr
                405                 410                 415

Asp Glu Asn Glu Gly Asn Cys Ser Trp Thr Arg Ser Leu Leu Ser Leu
            420                 425                 430

Asn Met Ser Ser Asn Ile Leu Thr Asp Ser Val Phe Arg Cys Leu Pro
        435                 440                 445

Pro Lys Val Lys Val Leu Asp Leu His Asp Asn Arg Ile Arg Ser Ile
    450                 455                 460

Pro Lys Pro Ile Met Lys Leu Glu Asp Leu Gln Glu Leu Asn Val Ala
465                 470                 475                 480

Ser Asn Ser Leu Ala His Phe Pro Asp Cys Gly Thr Phe Asn Arg Leu
                485                 490                 495

Ser Val Leu Ile Ile Asp Ser Asn Ser Ile Ser Asn Pro Ser Ala Asp
            500                 505                 510

Phe Leu Gln Ser Cys His Asn Ile Arg Ser Ile Ser Ala Gly Asn Asn
        515                 520                 525

Pro Phe Gln Cys Thr Cys Glu Leu Arg Glu Phe Val Gln Ser Leu Gly
    530                 535                 540

Gln Val Ala Ser Lys Val Val Glu Gly Trp Pro Asp Ser Tyr Lys Cys
545                 550                 555                 560

Asp Ser Pro Glu Asn Tyr Lys Gly Thr Leu Leu Lys Asp Phe His Val
                565                 570                 575

Ser Pro Leu Ser Cys Asn Thr Thr Leu Leu Leu Val Thr Ile Gly Val
            580                 585                 590

Ala Val Leu Val Phe Thr Val Thr Val Thr Ala Leu Cys Ile Tyr Phe
        595                 600                 605

Asp Leu Pro Trp Tyr Leu Arg Met Val Phe Gln Trp Thr Gln Thr Arg
    610                 615                 620

Arg Arg Ala Arg Asn Thr Pro Leu Glu Asn Leu Gln Arg Thr Ile Gln
625                 630                 635                 640

Phe His Ala Phe Ile Ser Tyr Ser Gly His Asp Ser Ala Trp Val Lys
                645                 650                 655

Ser Glu Leu Leu Pro Asn Leu Glu Lys Glu Glu Leu Arg Ile Cys Leu
            660                 665                 670

His Glu Arg Asn Phe Ile Pro Gly Lys Ser Ile Val Glu Asn Ile Ile
        675                 680                 685

Asn Cys Ile Glu Lys Ser Tyr Lys Ser Ile Phe Val Leu Ser Pro Asn
    690                 695                 700

Phe Val Gln Ser Glu Trp Cys His Tyr Glu Leu Tyr Phe Ala His His
705                 710                 715                 720

Asn Leu Phe His Glu Gly Ser Asn Asn Leu Ile Leu Ile Leu Leu Glu
                725                 730                 735

Pro Ile Pro Gln Tyr Ser Ile Pro Ser Ser Tyr His Lys Leu Lys Asn
            740                 745                 750

Leu Met Ala Gln Arg Thr Tyr Leu Glu Trp Pro Lys Glu Lys Ser Lys
        755                 760                 765

His Gly Leu Phe Trp Ala Asn Leu Arg Ala Ser Ile Asn Ile Lys Leu
    770                 775                 780

Arg Glu Gln Ala Lys Lys
785                 790

<210>  6
<211>  905
<212>  PRT
<213>  Canis lupus

<400>  6

Met Ser Gln Ser Leu Leu Tyr His Ile Tyr Ser Phe Leu Gly Leu Leu
1               5                   10                  15

Pro Phe Trp Ile Leu Cys Thr Ser Ser Thr Asn Lys Cys Val Val Arg
            20                  25                  30

His Glu Val Ala Asp Cys Ser His Leu Lys Leu Thr Gln Val Pro Asp
        35                  40                  45

Asp Leu Pro Ala Asn Ile Thr Val Leu Asn Leu Thr His Asn Gln Leu
    50                  55                  60

Arg Arg Leu Pro Pro Ala Asn Phe Thr Arg Tyr Ser Gln Leu Thr Ile
65                  70                  75                  80

Leu Asp Gly Gly Phe Asn Ser Ile Ser Lys Leu Glu Pro Glu Leu Cys
                85                  90                  95

Gln Lys Leu Pro Leu Leu Glu Ile Leu Asn Leu Gln His Asn Glu Leu
            100                 105                 110

Ser His Leu Ser Asp Gln Thr Phe Val Phe Cys Val Asn Leu Thr Glu
        115                 120                 125

Leu His Leu Met Ser Asn Ser Ile Lys Ile Ile Gln Asn Asn Pro Phe
    130                 135                 140

Arg Ser Leu Lys Asn Leu Val Lys Leu Asp Leu Ser His Asn Gly Leu

145                 150                 155                 160

Ser Ser Thr Lys Leu Gly Ser Gln Leu Gln Leu Glu Asn Leu Gln Glu
                165                 170                 175

Leu Leu Leu Ser Asn Asn Lys Ile Asn Val Leu Arg Arg Glu Glu Leu
            180                 185                 190

Asp Phe Leu Gly Asn Ser Ser Leu Glu Lys Leu Glu Leu Ser Ser Asn
        195                 200                 205

Pro Ile Lys Glu Phe Ser Pro Gly Cys Phe His Ala Ile Gly Lys Leu
    210                 215                 220

Phe Gly Leu Ser Leu Asn Asn Val Gln Leu Asn Pro Ser Leu Thr Glu
225                 230                 235                 240

Asn Leu Cys Leu Glu Leu Ser Asn Thr Ser Ile Gln Asn Leu Ser Leu
                245                 250                 255

Ser Asn Thr Gln Leu His Arg Thr Ser Asn Met Thr Phe Leu Gly Leu
            260                 265                 270

Lys His Thr Asn Leu Thr Met Leu Asp Leu Ser His Asn Asn Leu Asn
        275                 280                 285

Val Ile Glu Asn Asn Ser Phe Val Trp Leu Pro His Leu Glu Tyr Phe
    290                 295                 300

Leu Leu Glu Tyr Asn Asn Ile Glu His Leu Phe Ser His Ser Phe Tyr
305                 310                 315                 320

Gly Leu Leu Asn Val Arg Tyr Leu Asp Leu Lys Arg Ser Phe Ala Lys
                325                 330                 335

Gln Ser Thr Ser Leu Ala Ser His Pro Arg Ile Asp Asp Phe Ser Phe
            340                 345                 350

Gln Trp Leu Lys Cys Leu Gln Tyr Leu Asn Met Glu Asp Asn Tyr Phe
        355                 360                 365

Ala Gly Ile Lys Ser Asn Met Phe Thr Gly Leu Ile Lys Leu Lys His
    370                 375                 380

Leu Ser Leu Ser Asn Ser Phe Thr Ser Leu Gln Thr Leu Thr Asn Glu
385                 390                 395                 400

Thr Phe Leu Ser Leu Ala Gln Ser Pro Leu Ile Thr Leu Asn Leu Thr
                405                 410                 415

Lys Asn Lys Ile Ser Lys Ile Glu Ser Gly Ala Phe Ser Trp Leu Gly
            420                 425                 430

His Leu Gln Val Leu Asp Leu Gly Leu Asn Glu Ile Gly Gln Glu Leu
        435                 440                 445

Thr Gly Gln Glu Trp Arg Gly Leu Glu Asn Ile Val Glu Ile Tyr Leu
    450                 455                 460

Ser Tyr Asn Lys Tyr Leu Gln Leu Thr Ser Ser Ser Phe Ala Leu Ile
465                 470                 475                 480

Pro Ser Leu Arg Arg Leu Met Leu Arg Arg Thr Ala Leu Arg Asn Val
                485                 490                 495

Asp Ser Ser Pro Ser Pro Phe His Pro Leu Arg Asn Leu Asn Ile Leu
            500                 505                 510

Asp Leu Ser Asn Asn Asn Ile Ala Asn Ile Asn Asp Glu Leu Leu Glu
        515                 520                 525

Gly Leu Glu Lys Leu Glu Ile Leu Asp Met Gln His Asn Asn Leu Ala
    530                 535                 540

Arg Leu Trp Lys His Ala Asn Pro Gly Gly Pro Val His Phe Leu Lys
545                 550                 555                 560

Gly Leu Ser His Leu His Ile Leu Asn Leu Glu Ser Asn Gly Phe Asp
                565                 570                 575

Glu Ile Pro Ala Glu Val Phe Lys Gly Leu Ser Glu Leu Lys Ser Ile
            580                 585                 590

Asp Leu Gly Leu Asn Asn Leu Asn Ile Phe Pro Ser Ser Leu Phe Asn
        595                 600                 605

Asp Gln Val Ser Leu Lys Ser Leu Asn Leu Gln Lys Asn Leu Ile Thr
    610                 615                 620

Ser Val Glu Lys Asn Val Phe Gly Pro Ala Phe Arg Asn Leu Ser Asn
625                 630                 635                 640

Leu Asp Met Ser Phe Asn Pro Phe Asp Cys Thr Cys Glu Ser Ile Ala
                645                 650                 655

Trp Phe Val Asn Trp Ile Asn Ser Thr His Thr Asn Ile Ser Glu Leu
            660                 665                 670

Ser Ser His Tyr Leu Cys Asn Thr Pro Pro Gln Tyr His Gly Phe Pro
        675                 680                 685

Val Met Leu Phe Asp Ile Ser Pro Cys Lys Asp Ser Ala Pro Phe Glu
    690                 695                 700

Ile Phe Phe Ile Ile Asn Thr Ser Val Leu Leu Thr Phe Ile Phe Ile
705                 710                 715                 720

Val Leu Leu Ile His Phe Glu Gly Trp Arg Ile Ser Phe Tyr Trp Asn
                725                 730                 735

Val Ser Val His Arg Ile Leu Gly Phe Lys Glu Ile Asp Lys Gln Pro
            740                 745                 750

Glu Gln Phe Glu Tyr Ala Ala Tyr Ile Ile His Ala Tyr Lys Asp Arg
        755                 760                 765

Asp Trp Val Trp Glu His Phe Ser Pro Met Glu Glu Lys Asp Glu Thr
    770                 775                 780

Leu Lys Phe Cys Leu Glu Glu Arg Asp Phe Glu Ala Gly Val Leu Glu
785                 790                 795                 800

Leu Glu Ser Ile Ile Asn Ser Ile Lys Lys Ser Arg Lys Thr Ile Phe
                805                 810                 815

Val Ile Thr Gln His Leu Leu Lys Asp Pro Leu Cys Lys Arg Phe Lys
            820                 825                 830

Val His Gln Ala Val Gln Gln Ala Ile Glu Gln Asn Leu Glu Ser Ile
        835                 840                 845

Ile Leu Ile Phe Leu Glu Glu Ile Pro Asp Tyr Lys Leu Asn His Ala
    850                 855                 860

Leu Cys Leu Arg Arg Gly Met Phe Lys Ser His Cys Ile Leu Asn Trp
865                 870                 875                 880

Pro Val Gln Lys Glu Arg Val Asn Ala Phe His His Lys Leu Gln Val
                885                 890                 895

Ala Leu Gly Ser Arg Asn Ser Ile His
            900                 905

<210>  7
<211>  833
<212>  PRT
<213>  Canis lupus

<400>  7

Met Met Ser Pro Thr Arg Leu Val Gly Ile Leu Ile Pro Ala Met Ala
1               5                   10                  15

Phe Leu Ser Cys Leu Arg Pro Glu Ser Trp Asp Pro Cys Met Gln Val
            20                  25                  30

Val Ala Asn Thr Thr Tyr Gln Cys Met Glu Leu Asn Leu Ser Lys Ile

35                  40                  45

Pro Asn Asn Ile Pro Thr Ser Thr Glu Lys Leu Asp Leu Ser Phe Asn
    50                  55                  60

Pro Leu Arg His Leu Gly Ser His Cys Phe Ser Asn Phe Pro Lys Leu
65                  70                  75                  80

Gln Val Leu Asp Leu Ser Arg Cys Glu Ile Gln Val Ile Glu Asp Asp
                85                  90                  95

Ala Tyr Gln Gly Leu Asn His Leu Ser Ile Leu Ile Leu Thr Gly Asn
            100                 105                 110

Pro Ile Gln Arg Leu Phe Pro Arg Ala Phe Ser Gly Leu Ser Ser Leu
        115                 120                 125

Lys Thr Leu Val Ala Lys Glu Thr Lys Leu Thr Ser Leu Glu Asp Phe
    130                 135                 140

Pro Ile Gly His Leu Lys Thr Leu Lys Glu Leu Asn Val Ala His Asn
145                 150                 155                 160

Leu Ile His Ser Phe Lys Leu Pro Ala Tyr Phe Ser Asn Met Pro Asn
                165                 170                 175

Leu Glu Asn Val Asp Leu Ser Asn Asn Lys Ile Gln Asn Ile Tyr Arg
            180                 185                 190

Glu Asp Leu Gln Val Leu His His Met Pro Leu Leu Asn Leu Ser Leu
        195                 200                 205

Asp Leu Ser Leu Asn Pro Leu Tyr Phe Ile Gln Pro Gly Ser Phe Lys
    210                 215                 220

Glu Ile Lys Leu His Lys Leu Thr Leu Arg Ser Asn Phe Asn Ser Thr
225                 230                 235                 240

Asp Val Met Lys Thr Phe Ile Gln Gly Leu Ala Gly Leu Lys Ile Asn
                245                 250                 255

Gln Leu Val Leu Gly Glu Phe Lys Asn Glu Arg Lys Leu Glu Ser Phe
            260                 265                 270

Asp Asn Ser Leu Leu Glu Gly Leu Cys Asn Leu Thr Ile Glu Lys Phe
        275                 280                 285

Arg Ile Ala Tyr Phe Asp Ser Phe Ser Lys Asp Thr Thr Asn Leu Phe
    290                 295                 300

Asn Gln Leu Val Asn Ile Ser Ala Ile Ser Leu Ala His Leu Tyr Leu
305                 310                 315                 320

Asp Thr Pro Lys Tyr Leu Pro Lys Asn Leu Arg Trp Gln Arg Leu Glu
                325                 330                 335

Ile Val Asn Cys Asn Leu Glu Gln Phe Pro Ala Trp Glu Leu Asp Ser
            340                 345                 350

Leu Lys Glu Phe Val Leu Thr Ser Asn Lys Gly Met Asn Thr Phe Ala
        355                 360                 365

Asp Met Lys Met Glu Ser Leu Glu Phe Leu Asp Leu Ser Arg Asn Arg
    370                 375                 380

Leu Ser Phe Lys Thr Cys Cys Ser His Ser Asp Phe Gly Thr Thr Arg
385                 390                 395                 400

Leu Lys His Leu Asp Leu Ser Phe Asn Glu Ile Ile Thr Met Ser Ser
                405                 410                 415

Asn Phe Leu Gly Leu Glu Gln Leu Glu Tyr Leu Asp Leu Gln His Ser
            420                 425                 430

Ser Leu Lys Gln Ala Ser Asp Phe Ser Val Phe Leu Ser Leu Arg Asn
        435                 440                 445

Leu Arg Tyr Leu Asp Ile Ser Tyr Thr Arg Thr Glu Val Ala Phe Gln
    450                 455                 460

Gly Ile Phe Asp Gly Leu Val Ser Leu Glu Val Leu Lys Met Ala Asp
465                 470                 475                 480

Asn Ser Phe Pro Asp Asn Ser Leu Pro Asn Ile Phe Lys Gly Leu Thr
                485                 490                 495

Asn Leu Thr Ile Leu Asp Leu Ser Arg Cys His Leu Glu Arg Val Ser
            500                 505                 510

Gln Glu Ser Phe Val Ser Leu Pro Lys Leu Gln Glu Ile Asn Met Ser
        515                 520                 525

His Asn Ser Leu Leu Ser Leu Asp Thr Leu Ala Tyr Glu Pro Leu Leu
    530                 535                 540

Ser Leu Gln Ile Leu Asp Cys Ser Phe Asn Arg Ile Val Ala Phe Lys
545                 550                 555                 560

Glu Gln Gly Gln Gln His Phe Pro Ser Asn Leu Val Ser Leu Asn Leu
                565                 570                 575

Thr Arg Asn Asn Phe Ala Cys Asp Cys Glu His Gln Ser Phe Leu Gln
            580                 585                 590

Trp Val Lys Asp His Arg Gln Leu Leu Val Glu Val Glu Gln Met Val
        595                 600                 605

Cys Ala Lys Pro Leu Asp Met Lys Asp Met Pro Leu Leu Ser Phe Arg
    610                 615                 620

Asn Ala Thr Cys Gln Arg Ser Lys Thr Ile Ile Ser Val Ser Val Phe
625                 630                 635                 640

Thr Val Leu Met Val Ser Leu Val Ala Val Leu Ala Tyr Lys Phe Tyr
                645                 650                 655

Phe His Leu Met Leu Leu Ala Gly Cys Lys Arg Tyr Asn Arg Gly Glu
            660                 665                 670

Ser Thr Tyr Asp Ala Phe Val Ile Tyr Ser Ser Gln Asp Glu Asp Trp
        675                 680                 685

Val Arg Asn Glu Leu Val Lys Asn Leu Glu Glu Gly Val Pro Pro Phe
    690                 695                 700

Gln Leu Cys Leu His Tyr Arg Asp Phe Ile Pro Gly Val Ala Ile Ala
705                 710                 715                 720

Ala Asn Ile Ile Gln Glu Gly Phe Tyr Lys Ser Arg Lys Val Ile Val
                725                 730                 735

Val Val Ser Gln His Phe Ile Gln Ser Arg Trp Cys Ile Phe Glu Tyr
            740                 745                 750

Glu Ile Ala Gln Thr Trp Gln Phe Leu Ser Ser Arg Ala Gly Ile Ile
        755                 760                 765

Phe Ile Val Leu Gln Lys Val Glu Lys Ser Leu Leu Arg Gln Gln Val
    770                 775                 780

Glu Leu Tyr Arg Leu Leu Ser Arg Asn Thr Tyr Leu Glu Trp Glu Asp
785                 790                 795                 800

Ser Val Leu Gly Arg His Ile Phe Trp Arg Arg Leu Arg Lys Ala Leu
                805                 810                 815

Leu Asp Gly Lys Pro Trp Ser Pro Glu Gly Thr Glu Asp Ala Glu Lys
            820                 825                 830

Ser

<210>  8
<211>  807
<212>  PRT
<213>  Canis lupus

<400> 8

Met Lys Arg Ile Arg Ser Ile Tyr Ile Phe Cys Ser Ile Ala Ile Ser
1               5                   10                  15

Val Gln Gly Trp Ala Ser Lys Leu Pro Glu Glu Arg Glu Leu Thr Thr
            20                  25                  30

Asn Cys Ser Asn Met Ser Leu Arg Lys Ile Pro Ala Asp Leu Thr Pro
        35                  40                  45

Thr Thr Thr Thr Leu Asp Leu Ser Tyr Asn Leu Leu Ser Gln Leu Gln
    50                  55                  60

Ser Ser Asp Phe Arg Ser Val Ser Lys Leu Lys Val Leu Ile Leu Cys
65                  70                  75                  80

His Asn Arg Ile Gln Glu Leu Asn Ile Lys Ile Phe Glu Phe Asn Arg
                85                  90                  95

Glu Leu Arg Tyr Leu Asp Leu Ser Tyr Asn Arg Leu Lys Ile Val Thr
            100                 105                 110

Trp Tyr Ser Leu Ala Gly Leu Arg His Leu Asp Leu Ser Phe Asn Asp
        115                 120                 125

Phe Asp Thr Val Pro Ile Cys Glu Glu Thr Gly Asn Met Ser His Leu
    130                 135                 140

Glu Ile Leu Gly Leu Ser Gly Ala Lys Ile Gln Lys Ser Asn Phe Gln
145                 150                 155                 160

Lys Ile Ala His Leu His Leu Lys Thr Val Phe Leu Gly Leu Arg Ser
                165                 170                 175

Leu Ser His Tyr Glu Glu Gly Ser Leu Pro Ile Leu Asn Thr Thr Lys
            180                 185                 190

Leu His Ile Val Leu Pro Met Asn Thr Asn Phe Trp Val Leu Leu His
        195                 200                 205

Asp Gly Ile Lys Thr Ser Lys Ile Leu Glu Met Thr Asn Ile Asp Gly
    210                 215                 220

Lys Ser Gln Phe Ala Ser Tyr Gly Thr Gln Lys Asn Leu Thr Leu Glu
225                 230                 235                 240

Asn Ser Lys Thr Ser Ile Leu Leu Leu Asn Lys Val Asp Leu Leu Trp
                245                 250                 255

Asp Asp Leu Leu Leu Ile Phe Gln Phe Val Trp His Thr Ser Val Glu
            260                 265                 270

Cys Phe Gln Ile Gln His Leu Thr Phe Gly Gly Lys Val Tyr Leu Asp
        275                 280                 285

His Tyr Ser Phe Asp Tyr Ser Asn Thr Val Met Arg Thr Ile Lys Leu
    290                 295                 300

Glu His Val Gln Phe Arg Ile Phe Tyr Ile Pro Gln Glu Arg Val Tyr
305                 310                 315                 320

Leu Leu Phe Thr Lys Met Asp Ile Glu Asn Leu Thr Ile Ser Asp Ala
                325                 330                 335

Gln Met Pro Tyr Met Leu Phe Pro Ile Tyr Pro Thr Arg Phe Gln Tyr
            340                 345                 350

Leu Asn Phe Ala Asn Asn Ile Leu Thr Asp Asp Leu Phe Lys Gln Pro
        355                 360                 365

Ile Gln Leu Pro His Leu Lys Thr Leu Ile Leu Lys Gly Asn Lys Leu
    370                 375                 380

Glu Thr Phe Ser Leu Val Ser Phe Phe Ala Asn Asn Thr Ser Leu Lys
385                 390                 395                 400

His Leu Asp Leu Ser Gln Asn Leu Leu Gln His Glu Asn Gly Glu Asn
                405                 410                 415

Cys Phe Trp Pro Glu Thr Leu Ile Thr Met Asn Leu Ser Ser Asn Lys
            420                 425                 430

Phe Ala Asp Ser Val Phe Arg Cys Leu Pro Arg Asn Ile Gln Ile Leu
        435                 440                 445

Asp Leu Asn Asn Asn Lys Ile Gln Thr Val Pro Lys Asp Ile Ile His
    450                 455                 460

Leu Lys Ser Leu Gln Glu Leu Asn Leu Ala Phe Asn Phe Leu Thr Asp
465                 470                 475                 480

Leu Pro Gly Cys Ser His Phe Arg Lys Leu Ser Ile Leu Asn Ile Glu
                485                 490                 495

Met Asn Leu Ile Leu Ser Pro Ser Leu Asp Phe Phe Gln Ser Cys Gln
            500                 505                 510

Glu Val Lys Ile Leu Asn Ala Gly Arg Asn Pro Phe Arg Cys Thr Cys
        515                 520                 525

Glu Leu Arg Asp Phe Ile Gln Leu Glu Glu Tyr Ser Glu Gly Met Met
    530                 535                 540

Ile Gly Trp Leu Asp Ser Tyr Ile Cys Glu Tyr Pro Leu Thr Leu Lys
545                 550                 555                 560

Gly Thr Leu Leu Lys Asp Val His Leu Pro Glu Leu Ser Cys Asn Thr
                565                 570                 575

Thr Leu Leu Ile Val Thr Ile Val Val Ile Met Leu Val Leu Gly Met
            580                 585                 590

Ala Val Ala Phe Cys Cys Phe Tyr Phe Asp Leu Pro Trp Tyr Leu Arg
        595                 600                 605

Met Leu Gly Gln Trp Thr Leu Gln Arg Ile Arg Lys Thr Thr Gln Glu
    610                 615                 620

Gln Leu Lys Arg Asn Val Gln Phe His Val Phe Ile Ala Tyr Ser Glu
625                 630                 635                 640

His Asp Ser Thr Trp Val Lys His Glu Leu Ile Pro Asn Leu Glu Lys
                645                 650                 655

Lys Glu Lys Leu Ile Cys Leu His Glu Gly Asn Phe Asp Pro Gly Lys
            660                 665                 670

Ser Ile Ile Glu Asn Ile Met Asn Cys Ile Glu Lys Ser Tyr Lys Ser
        675                 680                 685

Ile Phe Val Leu Ser Pro Asn Phe Val Gln Ser Glu Trp Cys His Tyr
    690                 695                 700

Glu Leu Tyr Phe Ala His His Ser Leu Phe His Glu Asn Ser Asp Tyr
705                 710                 715                 720

Ile Ile Phe Ile Leu Leu Glu Pro Ile Pro Leu Tyr Cys Ile Pro Thr
                725                 730                 735

Lys Tyr Pro Lys Leu Lys Ala Leu Met Glu Lys Lys Ala Tyr Leu Glu
            740                 745                 750

Trp Pro Lys Asp Arg Arg Ile Cys Gly Leu Phe Trp Ala Asn Leu Arg
        755                 760                 765

Ala Ala Ile Asn Ala Asn Leu Leu Glu Thr Arg Glu Met Tyr Glu Leu
    770                 775                 780

Gln Thr Phe Val Gly Leu Asn Glu Glu Ser Gln Gly Ser Ala Ile Ser
785                 790                 795                 800

Leu Ile Arg Thr Asp Cys Leu
                805

<210>  9
<211>  1121
<212>  PRT
<213>  Canis lupus

<400>  9

Met Arg Asp Thr Gln Arg Glu Ala Gly Thr Gln Ala Glu Gly Glu Ala
1               5                   10                  15

Gly Ser Leu Gln Gly Ala Arg Cys Gly Thr Arg Ser Gln Asp Pro Gly
            20                  25                  30

Ile Thr Pro Trp Ala Glu Gly Arg Arg Ser Thr Pro Glu Pro Pro Arg
        35                  40                  45

Ser Pro Gln Pro Leu Cys Ser Ala Leu Phe Asn Gln Thr Ser Val Phe
    50                  55                  60

His Gly Gly Arg Arg Leu Lys Met Val Phe Pro Met Trp Thr Leu Lys
65                  70                  75                  80

Arg Gln Phe Phe Ile Leu Leu Asn Ile Ile Leu Ile Ser Lys Leu Leu
                85                  90                  95

Gly Ala Arg Trp Phe Pro Lys Thr Leu Pro Cys Asp Val Ser Leu Asp
            100                 105                 110

Ala Pro Lys Ala His Val Ile Val Asp Cys Thr Asp Lys His Leu Thr
        115                 120                 125

Glu Ile Pro Gly Gly Ile Pro Ser Asn Ala Thr Asn Leu Thr Leu Thr
    130                 135                 140

Ile Asn His Ile Pro Gly Ile Ser Pro Ala Ser Phe His Gln Leu Asp
145                 150                 155                 160

Tyr Leu Val Glu Ile Asp Phe Arg Cys Asn Cys Ile Pro Val Arg Leu
                165                 170                 175

Gly Pro Lys Asp His Leu Cys Thr Arg Arg Pro Gln Ile Lys Pro Arg
            180                 185                 190

Ser Phe Ser Ser Leu Thr Tyr Leu Lys Ser Leu Tyr Leu Asp Gly Asn
        195                 200                 205

Gln Leu Leu Glu Ile Pro Glu Gly Leu Pro Pro Ser Leu Glu Leu Leu
    210                 215                 220

Ser Leu Glu Ala Asn Ser Ile Phe Ser Ile Met Lys Asn Asn Leu Thr
225                 230                 235                 240

Glu Leu Thr Asn Ile Glu Arg Leu Tyr Leu Gly Gln Asn Cys Tyr Phe
                245                 250                 255

Arg Asn Pro Cys Asn Val Ser Phe Phe Ile Glu Lys Asp Ala Phe Leu
            260                 265                 270

Ser Leu Lys Asn Leu Lys Leu Leu Ser Leu Lys Asp Asn Asn Ile Thr
        275                 280                 285

Tyr Val Pro Thr Thr Leu Pro Ser Thr Leu Thr Glu Leu Tyr Leu Tyr
    290                 295                 300

Asn Asn Ala Ile Ala Lys Ile Gln Glu Asp Asp Phe Asn Asn Leu Asn
305                 310                 315                 320

Gln Leu Arg Ile Leu Asp Leu Ser Gly Asn Cys Pro Arg Cys Tyr Asn
                325                 330                 335

Val Pro Phe Pro Cys Thr Pro Cys Glu Asn Asn Ser Pro Leu Gln Ile
            340                 345                 350

His Glu Ser Ala Phe Asp Ala Leu Thr Glu Leu Gln Val Leu Arg Leu
        355                 360                 365

His Ser Asn Ser Leu Gln Arg Val Pro Gln Arg Trp Phe Lys Asn Ile
    370                 375                 380

Lys Lys Leu Lys Glu Leu Asp Leu Ser Gln Asn Phe Leu Ala Lys Glu
385                 390                 395                 400

Ile Gly Asp Ala Lys Phe Leu Tyr Leu Leu His Asp Leu Val Gln Leu
                405                 410                 415

Asp Leu Ser Phe Asn Tyr Glu Leu Gln Val Tyr Arg Ala Ala Leu Asn
            420                 425                 430

Leu Ser Asp Ala Phe Ser Ser Leu Lys Asn Leu Lys Val Leu Arg Ile
        435                 440                 445

Lys Gly Tyr Val Phe Lys Glu Leu Ser Ser His His Leu Ser Pro Leu
    450                 455                 460

Gln Ser Leu Thr Asn Leu Glu Val Leu Asp Leu Gly Thr Asn Phe Ile
465                 470                 475                 480

Lys Ile Ala Asp Leu Ser Ile Phe Glu Gln Phe Lys Thr Leu Lys Val
                485                 490                 495

Ile Asp Leu Ser Met Asn Lys Ile Ser Pro Ser Gly Asp Ser Gly Glu
            500                 505                 510

Val Gly Phe Cys Ser Ser Thr Arg Thr Ser Val Glu Gly His Ala Pro
        515                 520                 525

Gln Val Leu Glu Thr Leu His Tyr Phe Arg Tyr Asp Glu Tyr Ala Arg
    530                 535                 540

Ser Cys Arg Phe Lys Asn Lys Glu Thr Pro Ser Phe Leu Pro Phe Asn
545                 550                 555                 560

Lys Asp Cys Tyr Met Tyr Gly Gln Thr Leu Asp Leu Ser Arg Asn Asn
                565                 570                 575

Ile Phe Phe Ile Lys Ser Ser Asp Phe Gln His Leu Ser Phe Leu Lys
            580                 585                 590

Cys Leu Asn Leu Ser Gly Asn Thr Ile Gly Gln Thr Leu Asn Gly Ser
        595                 600                 605

Glu Phe Gln Pro Leu Val Glu Leu Lys Tyr Leu Asp Phe Ser Asn Asn
    610                 615                 620

Arg Leu Asp Leu Leu Tyr Ser Thr Ala Phe Glu Glu Leu Arg Lys Leu
625                 630                 635                 640

Glu Val Leu Asp Ile Ser Ser Asn Ser His Tyr Phe Gln Ser Glu Gly
                645                 650                 655

Ile Thr His Met Leu Asn Phe Thr Lys Asn Leu Lys Val Leu Lys Lys
            660                 665                 670

Leu Met Met Asn Asn Asn Asp Ile Ala Thr Ser Thr Ser Arg Thr Met
        675                 680                 685

Glu Ser Glu Ser Leu Lys Ile Leu Glu Phe Arg Gly Asn His Leu Asp
    690                 695                 700

Val Leu Trp Arg Asp Gly Asp Asn Arg Tyr Leu Lys Phe Phe Lys Asn
705                 710                 715                 720

Leu Leu Asn Leu Glu Glu Leu Asp Ile Ser Glu Asn Ser Leu Ser Phe
                725                 730                 735

Leu Pro Ser Gly Val Phe Asp Gly Met Pro Pro Asn Leu Lys Thr Leu
            740                 745                 750

Ser Leu Val Lys Asn Gly Leu Lys Ser Phe His Trp Glu Arg Leu Gln
        755                 760                 765

Tyr Leu Lys Asn Leu Glu Thr Leu Asp Leu Ser Tyr Asn Glu Leu Lys
    770                 775                 780

Ile Val Pro Glu Arg Leu Tyr Asn Cys Ser Arg Ser Leu Lys Lys Leu
785                 790                 795                 800

Ile Leu Lys Tyr Asn Gln Ile Arg Gln Leu Thr Lys His Phe Leu Gln
                805                 810                 815

Asp Ala Phe Gln Leu Arg Tyr Leu Asp Leu Ser Ser Asn Lys Ile Gln
            820                 825                 830

Ile Ile Gln Lys Thr Ser Phe Pro Glu Asn Val Leu Asn Asn Leu Glu
        835                 840                 845

Met Leu Leu Leu His His Asn Arg Phe Leu Cys Thr Cys Asp Ala Val
    850                 855                 860

Trp Phe Val Trp Trp Val Asn His Thr Glu Val Thr Ile Pro Tyr Leu
865                 870                 875                 880

Ala Thr Asp Val Thr Cys Val Gly Pro Gly Ala His Lys Gly Gln Ser
                885                 890                 895

Val Val Ser Leu Asp Leu Tyr Thr Cys Glu Leu Asp Leu Thr Asn Leu
            900                 905                 910

Val Leu Phe Ser Phe Ser Leu Ser Leu Ala Leu Phe Leu Met Val Ile
        915                 920                 925

Thr Thr Ala Asn His Leu Tyr Phe Trp Asp Val Trp Tyr Ser Tyr His
    930                 935                 940

Tyr Cys Lys Ala Lys Ile Lys Gly Tyr Arg Arg Leu Lys Ser Leu Asp
945                 950                 955                 960

Ser Cys Tyr Asp Ala Phe Val Val Tyr Asp Thr Lys Asp Pro Ala Val
                965                 970                 975

Thr Glu Trp Val Leu Asp Glu Leu Val Ala Lys Leu Glu Asp Pro Arg
            980                 985                 990

Glu Lys His Phe Asn Leu Cys Leu  Glu Glu Arg Asp Trp  Leu Pro Gly
        995                 1000                 1005

Gln Pro  Val Leu Glu Asn Leu  Ser Gln Ser Ile Gln  Leu Ser Lys
    1010                 1015                 1020

Lys Thr  Val Phe Val Met Thr  Asn Lys Tyr Ala Lys  Thr Glu Asn
    1025                 1030                 1035

Phe Lys  Ile Ala Phe Tyr Leu  Ser His Gln Arg Leu  Met Asp Glu
    1040                 1045                 1050

Lys Val  Asp Val Ile Ile Leu  Ile Phe Leu Glu Lys  Pro Leu Gln
    1055                 1060                 1065

Lys Ser  Lys Phe Leu Gln Leu  Arg Lys Arg Leu Cys  Lys Ser Ser

1070                    1075                    1080

Val Leu  Glu Trp Pro Arg Asn  Pro Gln Ala His Pro  Tyr Phe Trp
    1085                    1090                    1095

Gln Cys  Leu Lys Asn Ala Leu  Ala Thr Asp Asn His  Val Thr Tyr
    1100                    1105                    1110

Ser Gln  Val Phe Lys Glu Thr  Val
    1115                    1120

<210>  10
<211>  1032
<212>  PRT
<213>  Canis lupus

<400>  10

Met Gly Pro Cys Arg Gly Ala Leu His Pro Leu Ser Leu Leu Val Gln
1               5                   10                  15

Ala Ala Ala Leu Ala Leu Ala Leu Ala Gln Gly Thr Leu Pro Ala Phe
            20                  25                  30

Leu Pro Cys Glu Leu Gln Pro His Gly Leu Val Asn Cys Asn Trp Leu
        35                  40                  45

Phe Leu Lys Ser Val Pro Arg Phe Ser Ala Ala Ala Pro Arg Gly Asn
    50                  55                  60

Val Thr Ser Leu Ser Leu Tyr Ser Asn Arg Ile His His Leu His Asp
65                  70                  75                  80

Tyr Asp Phe Val His Phe Val His Leu Arg Arg Leu Asn Leu Lys Trp
                85                  90                  95

Asn Cys Pro Pro Ala Ser Leu Ser Pro Met His Phe Pro Cys His Met
            100                 105                 110

Thr Ile Glu Pro Asn Thr Phe Leu Ala Val Pro Thr Leu Glu Asp Leu
        115                 120                 125

Asn Leu Ser Tyr Asn Ser Ile Thr Thr Val Pro Ala Leu Pro Ser Ser
    130                 135                 140

Leu Val Ser Leu Ser Leu Ser Arg Thr Asn Ile Leu Val Leu Asp Pro
145                 150                 155                 160

Ala Thr Leu Ala Gly Leu Tyr Ala Leu Arg Phe Leu Phe Leu Asp Gly
                165                 170                 175

Asn Cys Tyr Tyr Lys Asn Pro Cys Gln Gln Ala Leu Gln Val Ala Pro
            180                 185                 190

Gly Ala Leu Leu Gly Leu Gly Asn Leu Thr His Leu Ser Leu Lys Tyr
        195                 200                 205
Asn Asn Leu Thr Val Val Pro Arg Gly Leu Pro Pro Ser Leu Glu Tyr
    210                 215                 220
Leu Leu Leu Ser Tyr Asn His Ile Ile Thr Leu Ala Pro Glu Asp Leu
225                 230                 235                 240
Ala Asn Leu Thr Ala Leu Arg Val Leu Asp Val Gly Gly Asn Cys Arg
                245                 250                 255
Arg Cys Asp His Ala Arg Asn Pro Cys Arg Glu Cys Pro Lys Gly Phe
            260                 265                 270
Pro Gln Leu His Pro Asn Thr Phe Gly His Leu Ser His Leu Glu Gly
        275                 280                 285
Leu Val Leu Arg Asp Ser Ser Leu Tyr Ser Leu Asp Pro Arg Trp Phe
    290                 295                 300
His Gly Leu Gly Asn Leu Met Val Leu Asp Leu Ser Glu Asn Phe Leu
305                 310                 315                 320
Tyr Asp Cys Ile Thr Lys Thr Lys Ala Phe Tyr Gly Leu Ala Arg Leu
                325                 330                 335
Arg Arg Leu Asn Leu Ser Phe Asn Tyr His Lys Lys Val Ser Phe Ala
            340                 345                 350
His Leu His Leu Ala Ser Ser Phe Gly Ser Leu Leu Ser Leu Gln Glu
        355                 360                 365
Leu Asp Ile His Gly Ile Phe Phe Arg Ser Leu Ser Lys Thr Thr Leu
    370                 375                 380
Gln Ser Leu Ala His Leu Pro Met Leu Gln Arg Leu His Leu Gln Leu
385                 390                 395                 400
Asn Phe Ile Ser Gln Ala Gln Leu Ser Ile Phe Gly Ala Phe Pro Gly
                405                 410                 415
Leu Arg Tyr Val Asp Leu Ser Asp Asn Arg Ile Ser Gly Ala Ala Glu
            420                 425                 430
Pro Ala Ala Ala Thr Gly Glu Val Glu Ala Asp Cys Gly Glu Arg Val
        435                 440                 445
Trp Pro Gln Ser Arg Asp Leu Ala Leu Gly Pro Leu Gly Thr Pro Gly
    450                 455                 460

Ser Glu Ala Phe Met Pro Ser Cys Arg Thr Leu Asn Phe Thr Leu Asp
465                 470                 475                 480

Leu Ser Arg Asn Asn Leu Val Thr Val Gln Pro Glu Met Phe Val Arg
                485                 490                 495

Leu Ala Arg Leu Gln Cys Leu Gly Leu Ser His Asn Ser Ile Ser Gln
            500                 505                 510

Ala Val Asn Gly Ser Gln Phe Val Pro Leu Ser Asn Leu Arg Val Leu
        515                 520                 525

Asp Leu Ser His Asn Lys Leu Asp Leu Tyr His Gly Arg Ser Phe Thr
    530                 535                 540

Glu Leu Pro Arg Leu Glu Ala Leu Asp Leu Ser Tyr Asn Ser Gln Pro
545                 550                 555                 560

Phe Ser Met Arg Gly Val Gly His Asn Leu Ser Phe Val Ala Gln Leu
                565                 570                 575

Pro Ala Leu Arg Tyr Leu Ser Leu Ala His Asn Gly Ile His Ser Arg
            580                 585                 590

Val Ser Gln Gln Leu Arg Ser Ala Ser Leu Arg Ala Leu Asp Phe Ser
        595                 600                 605

Gly Asn Thr Leu Ser Gln Met Trp Ala Glu Gly Asp Leu Tyr Leu Arg
    610                 615                 620

Phe Phe Gln Gly Leu Arg Ser Leu Val Gln Leu Asp Leu Ser Gln Asn
625                 630                 635                 640

Arg Leu His Thr Leu Leu Pro Arg Asn Leu Asp Asn Leu Pro Lys Ser
                645                 650                 655

Leu Arg Leu Leu Arg Leu Arg Asp Asn Tyr Leu Ala Phe Phe Asn Trp
            660                 665                 670

Ser Ser Leu Ala Leu Leu Pro Lys Leu Glu Ala Leu Asp Leu Ala Gly
        675                 680                 685

Asn Gln Leu Lys Ala Leu Ser Asn Gly Ser Leu Pro Asn Gly Thr Gln
    690                 695                 700

Leu Gln Arg Leu Asp Leu Ser Gly Asn Ser Ile Gly Phe Val Val Pro
705                 710                 715                 720

Ser Phe Phe Ala Leu Ala Val Arg Leu Arg Glu Leu Asn Leu Ser Ala
                725                 730                 735

Asn Ala Leu Lys Thr Val Glu Pro Ser Trp Phe Gly Ser Leu Ala Gly

            740                 745                 750

Ala Leu Lys Val Leu Asp Val Thr Ala Asn Pro Leu His Cys Ala Cys
        755                 760                 765

Gly Ala Thr Phe Val Asp Phe Leu Leu Glu Val Gln Ala Ala Val Pro
    770                 775                 780

Gly Leu Pro Ser Arg Val Lys Cys Gly Ser Pro Gly Gln Leu Gln Gly
785                 790                 795                 800

Arg Ser Ile Phe Ala Gln Asp Leu Arg Leu Cys Leu Asp Glu Ala Leu
                805                 810                 815

Ser Trp Val Cys Phe Ser Leu Ser Leu Leu Ala Val Ala Leu Ser Leu
            820                 825                 830

Ala Val Pro Met Leu His Gln Leu Cys Gly Trp Asp Leu Trp Tyr Cys
        835                 840                 845

Phe His Leu Cys Leu Ala Trp Leu Pro Arg Arg Gly Arg Arg Arg Gly
    850                 855                 860

Val Asp Ala Leu Ala Tyr Asp Ala Phe Val Val Phe Asp Lys Ala Gln
865                 870                 875                 880

Ser Ser Val Ala Asp Trp Val Tyr Asn Glu Leu Arg Val Gln Leu Glu
                885                 890                 895

Glu Arg Arg Gly Arg Arg Ala Leu Arg Leu Cys Leu Glu Glu Arg Asp
            900                 905                 910

Trp Val Pro Gly Lys Thr Leu Phe Glu Asn Leu Trp Ala Ser Val Tyr
        915                 920                 925

Ser Ser Arg Lys Thr Leu Phe Val Leu Ala Arg Thr Asp Arg Val Ser
    930                 935                 940

Gly Leu Leu Arg Ala Ser Phe Leu Leu Ala Gln Gln Arg Leu Leu Glu
945                 950                 955                 960

Asp Arg Lys Asp Val Val Val Leu Val Ile Leu Cys Pro Asp Ala His
                965                 970                 975

Arg Ser Arg Tyr Val Arg Leu Arg Gln Arg Leu Cys Arg Gln Ser Val
            980                 985                 990

Leu Leu Trp Pro His Gln Pro Ser Gly Gln Arg Ser Phe Trp Ala Gln
        995                 1000                1005

Leu Gly Thr Ala Leu Thr Arg Asp Asn Arg His Phe Tyr Asn Gln
    1010                1015                1020

Asn Phe Cys Arg Gly Pro Thr Thr Ala
    1025                    1030

<210> 11
<211> 15
<212> DNA
<213> Canis lupus

<400> 11
ctccttcgag aaagc                                                        15

<210> 12
<211> 18
<212> DNA
<213> Canis lupus

<400> 12
ttatcgtgaa gatcaagc                                                     18

<210> 13
<211> 18
<212> DNA
<213> Canis lupus

<400> 13
catcatctgg gtaacgaa                                                     18

<210> 14
<211> 20
<212> DNA
<213> Canis lupus

<400> 14
catttgaaat tgagaaatac                                                   20

<210> 15
<211> 19
<212> DNA
<213> Canis lupus

<400> 15
acaccaagtc gttagtaat                                                    19

<210> 16
<211> 14
<212> DNA
<213> Canis lupus

<400> 16
cccgccgtga gcag                                                         14

<210> 17
<211> 16
<212> DNA
<213> Canis lupus

<400> 17
tctccaagag cttctg                                                       16

<210> 18
<211> 18
<212> DNA
<213> Canis lupus

<400> 18
caaaactgca ggtgctgg 18

<210> 19
<211> 14
<212> DNA
<213> Canis lupus

<400> 19
cccatgcggg aaat 14

<210> 20
<211> 20
<212> DNA
<213> Canis lupus

<400> 20
aagttattcc gagtaagatt 20

<210> 21
<211> 15
<212> DNA
<213> Canis lupus

<400> 21
ttccccgaag ccctg 15

<210> 22
<211> 17
<212> DNA
<213> Canis lupus

<400> 22
tgtttgccgt cactcaa 17

<210> 23
<211> 14
<212> DNA
<213> Canis lupus

<400> 23
tccgggccgt cacc 14

<210> 24
<211> 13
<212> DNA
<213> Canis lupus

<400> 24
aggcgcaaca aag 13

<210> 25
<211> 15
<212> DNA
<213> Canis lupus

<400> 25

tccttccggg agctg                                                          15


<210>  26
<211>  14
<212>  DNA
<213>  Canis lupus

<400>  26
ctcaaccgga tccc                                                           14


<210>  27
<211>  13
<212>  DNA
<213>  Canis lupus

<400>  27
tcgcacggct tcg                                                            13


<210>  28
<211>  14
<212>  DNA
<213>  Canis lupus

<400>  28
agcccccgga acac                                                           14


<210>  29
<211>  14
<212>  DNA
<213>  Canis lupus

<400>  29
tcaggtcgag gccc                                                           14


<210>  30
<211>  13
<212>  DNA
<213>  Canis lupus

<400>  30
cgcagcgcac gtc                                                            13


<210>  31
<211>  15
<212>  DNA
<213>  Canis lupus

<400>  31
ctcgtggccg ccaag                                                          15


<210>  32
<211>  14
<212>  DNA
<213>  Canis lupus

<400>  32
ccaggtggga cgcg                                                           14


<210>  33
<211>  15
<212>  DNA

<213>  Canis lupus

<400>  33
cctggacggc gccct                                                        15


<210>  34
<211>  14
<212>  DNA
<213>  Canis lupus

<400>  34
cagcgccggt actt                                                         14


<210>  35
<211>  12
<212>  DNA
<213>  Canis lupus

<400>  35
cgcgcgcccc tc                                                           12


<210>  36
<211>  18
<212>  DNA
<213>  Canis lupus

<400>  36
ttcttttgca taataacc                                                     18


<210>  37
<211>  15
<212>  DNA
<213>  Canis lupus

<400>  37
agggagcctg cttct                                                        15


<210>  38
<211>  14
<212>  DNA
<213>  Canis lupus

<400>  38
tgcatcggaa atcg                                                         14


<210>  39
<211>  18
<212>  DNA
<213>  Canis lupus

<400>  39
cttgagaagc cccttcag                                                     18


<210>  40
<211>  14
<212>  DNA
<213>  Canis lupus

<400>  40
ccctccggaa gatt                                                         14

<210>  41
<211>  18
<212>  DNA
<213>  Canis lupus

<400>  41
ttgtccactt cgtccacc                                                    18


<210>  42
<211>  15
<212>  DNA
<213>  Canis lupus

<400>  42
cgctcagcaa gacca                                                       15


<210>  43
<211>  16
<212>  DNA
<213>  Canis lupus

<400>  43
cccagtgggc ccagag                                                      16


<210>  44
<211>  15
<212>  DNA
<213>  Canis lupus

<400>  44
acggctaggc aggcc                                                       15


<210>  45
<211>  12
<212>  DNA
<213>  Canis lupus

<400>  45
cccgccgccg cc                                                          12


<210>  46
<211>  16
<212>  DNA
<213>  Canis lupus

<400>  46
ccacagccat ccccag                                                      16


<210>  47
<211>  16
<212>  DNA
<213>  Canis lupus

<400>  47
ccaggaggaa cagcgt                                                      16


<210>  48
<211>  10
<212>  PRT
<213>  Canis lupus

<400>  48

Thr Ile Asn Ala Phe Leu Lys Glu Gln Leu
1               5                   10

<210>  49
<211>  130
<212>  PRT
<213>  Canis lupus

<220>
<221>  VARIANT
<222>  (7)..(7)
<223>  X is an amino acid selected from I and F

<220>
<221>  VARIANT
<222>  (129)..(129)
<223>  X is an amino acid selected from P and L

<400>  49

Lys Val Lys Val Leu Asp Xaa His Asp Asn Arg Ile Arg Ser Ile Pro
1               5                   10                  15

Lys Pro Ile Met Lys Leu Glu Asp Leu Gln Glu Leu Asn Val Ala Ser
            20                  25                  30

Asn Ser Leu Ala His Phe Pro Asp Cys Gly Thr Phe Asn Arg Leu Ser
        35                  40                  45

Val Leu Ile Ile Asp Ser Asn Ser Ile Ser Asn Pro Ser Ala Asp Phe
    50                  55                  60

Leu Gln Ser Cys His Asn Ile Arg Ser Met Ser Ala Gly Asn Asn Pro
65                  70                  75                  80

Phe Gln Cys Thr Cys Glu Leu Arg Glu Phe Val Gln Ser Leu Gly Gln
                85                  90                  95

Val Ala Ser Lys Val Val Glu Gly Trp Pro Asp Ser Tyr Lys Cys Asp
            100                 105                 110

Ser Pro Glu Asn Tyr Lys Gly Thr Leu Leu Lys Asp Phe His Val Ser
        115                 120                 125

Xaa Leu
    130

<210>  50
<211>  10
<212>  PRT
<213>  Canis lupus

<400>  50

Gln Asn Asn Ile Ile Trp Ala Thr Lys Lys
1               5                   10

<210>  51
<211>  10
<212>  PRT
<213>  Canis lupus

<400>  51

Gly Val Thr Leu Phe Leu Leu Ala Val Leu
1               5                   10


<210>  52
<211>  14
<212>  DNA
<213>  Canis lupus

<400>  52
cccagcttgc ttgc                                                         14


<210>  53
<211>  25
<212>  DNA
<213>  Artificial

<220>
<223>  forward primer

<400>  53
agttggtttg gcatacaagc ataga                                             25


<210>  54
<211>  25
<212>  DNA
<213>  Artificial

<220>
<223>  reverse primer

<400>  54
tctgaagtca gggtaacctt gtagt                                             25


<210>  55
<211>  24
<212>  DNA
<213>  Artificial

<220>
<223>  forward primer

<400>  55
gacacttcac tgaaggcctt atct                                              24


<210>  56
<211>  37
<212>  DNA
<213>  Artificial

<220>
<223>  reverse primer

<400>  56
agattttata gatataactt tgtggcaaat tgaatgc                                37

<210>  57
<211>  30
<212>  DNA
<213>  Artificial

<220>
<223>  forward primer

<400>  57
ccaccttaca aattatgaga atcagcagaa                                        30


<210>  58
<211>  23
<212>  DNA
<213>  Artificial

<220>
<223>  reverse primer

<400>  58
agcctgtgaa aggaatccag ttg                                               23


<210>  59
<211>  18
<212>  DNA
<213>  Artificial

<220>
<223>  forward primer

<400>  59
gccgtgtgct gtgtcttg                                                     18


<210>  60
<211>  18
<212>  DNA
<213>  Artificial

<220>
<223>  reverse primer

<400>  60
acagcccgtg gaaatggt                                                     18


<210>  61
<211>  24
<212>  DNA
<213>  Artificial

<220>
<223>  forward primer

<400>  61
aagttcttca cgcctcagta catt                                              24


<210>  62
<211>  27
<212>  DNA
<213>  Artificial

<220>
<223>  reverse primer

<400>  62

attaggaagt cagctccaat tggaaaa 27

<210> 63
<211> 22
<212> DNA
<213> Artificial

<220>
<223> forward primer

<400> 63
cagccattgc ttctccaact tc 22

<210> 64
<211> 24
<212> DNA
<213> Artificial

<220>
<223> reverse primer

<400> 64
cttgggcaat gtgtaaaagc tcat 24

<210> 65
<211> 25
<212> DNA
<213> Artificial

<220>
<223> forward primer

<400> 65
agatggcaac ggttggaaat agtta 25

<210> 66
<211> 25
<212> DNA
<213> Artificial

<220>
<223> reverse primer

<400> 66
ggaagtgaga acaaactcct tgaga 25

<210> 67
<211> 25
<212> DNA
<213> Artificial

<220>
<223> forward primer

<400> 67
gaacaaggac aacagcattt tccaa 25

<210> 68
<211> 23
<212> DNA
<213> Artificial

<220>

<223>  reverse primer

<400>  68
tctgatgttc acagtcacaa gca                                               23


<210>  69
<211>  24
<212>  DNA
<213>  Artificial

<220>
<223>  forward primer

<400>  69
cacgttctcc ctcttcatct tctc                                              24


<210>  70
<211>  17
<212>  DNA
<213>  Artificial

<220>
<223>  reverse primer

<400>  70
caaaggcccc ggagctt                                                      17


<210>  71
<211>  19
<212>  DNA
<213>  Artificial

<220>
<223>  forward primer

<400>  71
gtatcctccg cctgccatt                                                    19


<210>  72
<211>  24
<212>  DNA
<213>  Artificial

<220>
<223>  reverse primer

<400>  72
ttaccttgag gagagtctgt ttgc                                              24


<210>  73
<211>  18
<212>  DNA
<213>  Artificial

<220>
<223>  forward primer

<400>  73
   ctccgcctgc cattttcc                                                      18


<210>  74
<211>  25
<212>  DNA

<213>  Artificial

<220>
<223>  reverse primer

<400>  74
ggtccttgag aaacacttac cttga                                          25


<210>  75
<211>  22
<212>  DNA
<213>  Artificial

<220>
<223>  forward primer

<400>  75
ctcctgctga gcttcaacta ca                                             22


<210>  76
<211>  15
<212>  DNA
<213>  Artificial

<220>
<223>  reverse primer

<400>  76
gcagccgctc cagga                                                     15


<210>  77
<211>  25
<212>  DNA
<213>  Artificial

<220>
<223>  forward primer

<400>  77
cgtgttgaca gacggttatt tcaga                                          25


<210>  78
<211>  23
<212>  DNA
<213>  Artificial

<220>
<223>  reverse primer

<400>  78
ctcgaggctc ccaatctgat ttt                                            23


<210>  79
<211>  18
<212>  DNA
<213>  Artificial

<220>
<223>  forward primer

<400>  79
gggagcctcg agcttcac                                                  18

<210>  80
<211>  16
<212>  DNA
<213>  Artificial

<220>
<223>  reverse primer

<400>  80
acacgcagcc gggatc                                                        16


<210>  81
<211>  18
<212>  DNA
<213>  Artificial

<220>
<223>  forward primer

<400>  81
gctgggttcc ctgaggtc                                                      18


<210>  82
<211>  16
<212>  DNA
<213>  Artificial

<220>
<223>  reverse primer

<400>  82
agggagagcg ccttgc                                                        16


<210>  83
<211>  17
<212>  DNA
<213>  Artificial

<220>
<223>  forward primer

<400>  83
tgctgcggct ggatctg                                                       17


<210>  84
<211>  18
<212>  DNA
<213>  Artificial

<220>
<223>  reverse primer

<400>  84
gggcgttcag ggagaaga                                                      18


<210>  85
<211>  14
<212>  DNA
<213>  Artificial

<220>
<223>  forward primer

<400>  85

gggcggtgct ggga                                                        14


<210>  86
<211>  18
<212>  DNA
<213>  Artificial

<220>
<223>  reverse primer

<400>  86
tgtggttcag gtgcagca                                                    18


<210>  87
<211>  16
<212>  DNA
<213>  Artificial

<220>
<223>  forward primer

<400>  87
gacctcacgg cgctga                                                      16


<210>  88
<211>  17
<212>  DNA
<213>  Artificial

<220>
<223>  reverse primer

<400>  88
gcgtgctcag cctgttg                                                     17


<210>  89
<211>  18
<212>  DNA
<213>  Artificial

<220>
<223>  forward primer

<400>  89
gtggctcaac cggaccaa                                                    18


<210>  90
<211>  19
<212>  DNA
<213>  Artificial

<220>
<223>  reverse primer

<400>  90
agccctccat ggagacaga                                                   19


<210>  91
<211>  21
<212>  DNA
<213>  Artificial

<220>

<223>  forward primer

<400>  91
cctcttcatc ttctccaccg t                                                21


<210>  92
<211>  23
<212>  DNA
<213>  Artificial

<220>
<223>  reverse primer

<400>  92
gccttgtaac agaggaagca aag                                              23


<210>  93
<211>  16
<212>  DNA
<213>  Artificial

<220>
<223>  forward primer

<400>  93
tgcctggagg ccttcg                                                      16


<210>  94
<211>  18
<212>  DNA
<213>  Artificial

<220>
<223>  reverse primer

<400>  94
cacgaccacc aggacgag                                                    18


<210>  95
<211>  16
<212>  DNA
<213>  Artificial

<220>
<223>  forward primer

<400>  95
tgcctggagg ccttcg                                                      16


<210>  96
<211>  17
<212>  DNA
<213>  Artificial

<220>
<223>  reverse primer

<400>  96
cccacgacca ccaggac                                                     17


<210>  97
<211>  15
<212>  DNA

<213>  Artificial

<220>
<223>  forward primer

<400>  97
aggcacccgg ccatc                                                        15


<210>  98
<211>  18
<212>  DNA
<213>  Artificial

<220>
<223>  reverse primer

<400>  98
ccacgtcctg cagatcct                                                     18


<210>  99
<211>  18
<212>  DNA
<213>  Artificial

<220>
<223>  forward primer

<400>  99
acgctctccc gacacatc                                                     18


<210>  100
<211>  14
<212>  DNA
<213>  Artificial

<220>
<223>  reverse primer

<400>  100
cgcagcggga tgcc                                                         14


<210>  101
<211>  26
<212>  DNA
<213>  Artificial

<220>
<223>  forward primer

<400>  101
gttttcagat gtttacctcc caaggt                                            26


<210>  102
<211>  26
<212>  DNA
<213>  Artificial

<220>
<223>  reverse primer

<400>  102
agcttcatga ttggtttagg aatgct                                            26

<210>  103
<211>  22
<212>  DNA
<213>  Artificial

<220>
<223>  forward primer

<400>  103
atctgctacc aattgctcat tt                                                22


<210>  104
<211>  33
<212>  DNA
<213>  Artificial

<220>
<223>  reverse primer

<400>  104
agtgtttttg tatctagaat ttgaagactt cct                                     33


<210>  105
<211>  15
<212>  DNA
<213>  Artificial

<220>
<223>  forward primer

<400>  105
tcccgcgcag acgtg                                                        15


<210>  106
<211>  19
<212>  DNA
<213>  Artificial

<220>
<223>  reverse primer

<400>  106
agccctccat ggagacaga                                                    19


<210>  107
<211>  19
<212>  DNA
<213>  Artificial

<220>
<223>  forward primer

<400>  107
agagagaggc agggacaca                                                    19


<210>  108
<211>  19
<212>  DNA
<213>  Artificial

<220>
<223>  reverse primer

<400>  108

ggatcgagtc ccacatcgg 19

<210> 109
<211> 24
<212> DNA
<213> Artificial

<220>
<223> forward primer

<400> 109
ccagctggac tatctggtag agat 24

<210> 110
<211> 22
<212> DNA
<213> Artificial

<220>
<223> reverse primer

<400> 110
ccccagtcga acaggtatac ag 22

<210> 111
<211> 25
<212> DNA
<213> Artificial

<220>
<223> forward primer

<400> 111
tcatggatga aaaagtggac gtcat 25

<210> 112
<211> 22
<212> DNA
<213> Artificial

<220>
<223> reverse primer

<400> 112
ggagctggag gaacttggat tt 22

<210> 113
<211> 25
<212> DNA
<213> artificial

<220>
<223> forward primer

<400> 113
ggtctttgaa ggaacttagc ctgat 25

<210> 114
<211> 22
<212> DNA
<213> artificial

<220>

<223>  reverse primer

<400>  114
cgtgagccca gacgtattct tt                                                22


<210>  115
<211>  24
<212>  DNA
<213>  artificial

<220>
<223>  forward primer

<400>  115
gcttcgctac ctaaacctct ctag                                              24


<210>  116
<211>  22
<212>  DNA
<213>  artificial

<220>
<223>  reverse primer

<400>  116
catgttgtca aaccacgttg ca                                                22


<210>  117
<211>  23
<212>  DNA
<213>  artificial

<220>
<223>  forward primer

<400>  117
ccaccacctc catgactatg act                                               23


<210>  118
<211>  23
<212>  DNA
<213>  artificial

<220>
<223>  reverse primer

<400>  118
ggcagttcca cttgagattg aga                                               23


<210>  119
<211>  24
<212>  DNA
<213>  artificial

<220>
<223>  forward primer

<400>  119
gctggacata catggcatct tctt                                              24


<210>  120
<211>  15
<212>  DNA

<213>  artificial

<220>
<223>  reverse primer

<400>  120
ggtgggccag cgact                                                        15


<210>  121
<211>  20
<212>  DNA
<213>  artificial

<220>
<223>  forward primer

<400>  121
ggagagagtc tggccacagt                                                   20


<210>  122
<211>  18
<212>  DNA
<213>  artificial

<220>
<223>  reverse primer

<400>  122
cggcatgaag gcctctga                                                     18


<210>  123
<211>  16
<212>  DNA
<213>  artificial

<220>
<223>  forward primer

<400>  123
gctggaggtg caggct                                                       16


<210>  124
<211>  16
<212>  DNA
<213>  artificial

<220>
<223>  reverse primer

<400>  124
ggctgccgca cttgac                                                       16


<210>  125
<211>  15
<212>  DNA
<213>  artificial

<220>
<223>  forward primer

<400>  125
gtgcctggcc tggct                                                        15

<210>  126
<211>  20
<212>  DNA
<213>  artificial

<220>
<223>  reverse primer

<400>  126
gccttgtcga agaccacgaa                                                    20


<210>  127
<211>  25
<212>  DNA
<213>  artificial

<220>
<223>  forward primer

<400>  127
tctgttgatt gtcaccattg tggtt                                              25


<210>  128
<211>  25
<212>  DNA
<213>  artificial

<220>
<223>  reverse primer

<400>  128
cagatcaaag tagaagcagc agaag                                              25